# EUROPEAN PATENT SPECIFICATION

(11) **EP 3 978 480 B1**
(45) Date of publication and mention of the grant of the patent: **15.07.2026**
(21) Application number: 20814110.1
(22) Date of filing: 29.05.2020
(51) Int. Cl.: C07D 237/32, A61K 31/502, A61P 35/00, A61P 1/16

(54) **PHTHALAZINONE COMPOUNDS AND USE THEREOF**
PHTHALAZINONVERBINDUNGEN UND VERWENDUNG DAVON
COMPOSÉS DE PHTALAZINONE ET LEUR UTILISATION

(30) Priority: 29.05.2019 KR 20190063436
(43) Date of publication of application: 06.04.2022
(73) Proprietor: ST Pharm Co., Ltd., Siheung-si, Gyeonggi-do 15086 (KR); Korea Research Institute of Chemical Technology, Daejeon 34114 (KR)
(72) Inventor: KIM, Kyungjin, Seoul 06194 (KR); KIM, Kwangrok, Daejeon 34114 (KR); KIM, Uk-Il, Ansan-si, Gyeonggi-do 15610 (KR); BANG, Hyung Tae, Ansan-si, Gyeonggi-do 15610 (KR); LEE, Seul Ki, Ansan-si, Gyeonggi-do 15610 (KR); JEONG, Kwan-Young, Daejeon 34114 (KR); KANG, Seung Kyu, Daejeon 34114 (KR); JUNG, Heejung, Daejeon 34114 (KR); RHEE, Sang Dal, Daejeon 34114 (KR); JUNG, Won Hoon, Daejeon 34114 (KR); LEE, Jun Mi, Daejeon 34114 (KR)
(74) Representative: Kraus & Lederer PartGmbB
(86) International application number: PCT/KR2020/006995
(87) International publication number: WO 2020/242245

(56) References cited:
- EP-A1- 0 634 404
- WO-A1-2010/077947
- WO-A1-2016/139361
- US-A- 5 624 922
- DATABASE Registry CAS; 3 January 2003 (2003-01-03), "- 1(2H)-Phthalazinone, 2-(4-bromophenyl)-7-fluoro-4-(4-fluorophenyl)-", XP055764759, retrieved from STN Database accession no. RN 478066-05-6
- DATABASE Registry CAS; 30 May 2001 (2001-05-30), "- 1(2H)-Phthalazinone, 7-fluoro-4-(4-fluorophenyl)-2-[4- (trifluoromethyl)phenyl]-", XP055764768, retrieved from STN Database accession no. RN 339009-72-2
- DATABASE Registry CAS; 30 May 2001 (2001-05-30), "- 1(2H)-Phthalazinone, 7-fluoro-2,4-bis(4-fluorophenyl)-", XP055764773, retrieved from STN Database accession no. RN 339009-71-1
- DATABASE Registry CAS; 4 March 2018 (2018-03-04), "1(2H)-Phthalazinone, 8-chloro-2,4-diphenyl-", XP055764779, retrieved from STN Database accession no. RN2184101-07-1
- DATABASE Registry CAS; 4 March 2018 (2018-03-04), "- 1(2H)-Phthalazinone, 7-chloro-2,4-diphenyl-", XP055764784, retrieved from STN Database accession no. RN2183741-74-2
- DATABASE Registry CAS; 1 March 2018 (2018-03-01), "- 1(2H)-Phthalazinone, 7-chloro-4-phenyl-2-propyl-", XP055764787, retrieved from STN Database accession no. RN 2182402-34-0
- HAMEED, A. DH. ET AL.: "Synthesis and Biological Evaluation of New Phthalazinone Derivatives as Anti-Inflammatory and Anti-Proliferative Agents", ARCH. PHARM. CHEM. LIFE SCI., vol. 349, 2016, pages 150 - 159, XP055861161

## Description

### TECHNICAL FILED

The present invention relates to specific phthalazinone compounds, or racemates, stereoisomers or pharmaceutically acceptable salts thereof; and a pharmaceutical composition comprising the same as an active ingredient for the prevention or treatment of Sirtuin 6 protein (hereinafter, referred to as "Sirt6")-associated diseases.

### BACKGROUND ART

To date, a total of seven types of sirtuin proteins, Sirt1 to Sirt7, have been identified, and most of them have been reported as being involved in aging, metabolic diseases, and gene stability. It has been found that Sirt6 whole-body knockout mice are normal during fetal development but smaller than the wild type after birth and have lymphopenia, osteopenia, abnormal metabolism, genomic instability, excessive inflammation, or myocardial hypertrophy, and cellselective Sirt6 knockout mice display phenotypes such as growth retardation, fatty liver, cancer development, hepatocirrhosis/inflammation, and decreased insulin secretion (Proc. Natl. Acad. Sci. U S A. 2010, 107, 21790-21794; Cell Metab. 2010, 12, 224-236; Cell 2012, 151, 1185-1199; J. Biol. Chem. 2012, 287, 41903-41913). Based on the results of various animal model studies described above, it was reported that Sirt6 has the functions of DNA damage repair, metabolic homeostatis regulation (glucose metabolism and fat metabolism), inflammatory response regulation, and lifespan-associated mechanism regulation (Nature 2009, 460, 587-591). Sirt6 may be present in cytoplasmic stress granules and endoplasmic reticulum, but is mostly concentrated in the nucleus, particularly chromatin, and is activated by NAD⁺ cofactors (Sci. Rep. 2013, 3, 3085).

H2K9, H3K56, H3K18, and acetyltransferase GCN5 have been known as major target proteins of Sirt6 deacetylase enzyme activity (Curr. Top. Med. Chem. 2013, 13, 2991-3000; Nature 2008, 452,492-496; Cell Cycle 2009, 8, 2664-2666; and Nat. Struct. Mol. Biol. 2016, 23, 434-440). It has been reported that H3K9 and H3K56 associated with the Sirt6 protein regulate the transcriptional expression of specific genes through acetylation/deacetylation and control the stability of specific heterochromatins such as telomeres (Cell 2009, 136, 62-74; Cell, 2010, 140, 280-293), thereby maintaining genome stability, regulating the expression of genes associated with glucose metabolism and fat metabolism, and contributing to telomere stability. Because of the above-described chromatin regulatory functions, Sirt6 deficiency has been reported to induce cancer, fatty liver, myocardial hypertrophy, and premature aging (Curr. Top. Med. Chem. 2013, 13, 2991-3000).

Accordingly, it has been expected that a compound having Sirt6 activity or a pharmaceutically acceptable salt thereof can be used for the treatment or prevention of diseases associated with Sirt6 activity.

WO 2010/077947 A1 describes sirtuin-modulating compounds and methods of use thereof. The sirtuin-modulating compounds may be used for increasing the lifespan of a cell, and treating and/or preventing a wide variety of diseases and disorders including, for example, diseases or disorders related to aging or stress, diabetes, obesity, neurodegenerative diseases, cardiovascular disease, blood clotting disorders, inflammation, cancer, and/or flushing as well as diseases or disorders that would benefit from increased mitochondrial activity. Also described are compositions comprising a sirtuin- modulating compound in combination with another therapeutic agent.

EP 0 634 404 A1 describes phtalazin derivatives and their use as pesticides.

### DETAILED DESCRIPTION OF THE INVENTION

### TECHNICAL PROBLEM TO BE SOLVED

An object of the present invention is to provide a phthalazinone compound exhibiting Sirt6 activity, or a racemate, a stereoisomer or a pharmaceutically acceptable salt thereof.

Another object of the present invention is to provide a pharmaceutical composition for the prevention or treatment of Sirt6-related diseases, comprising a phthalazinone compound exhibiting Sirt6 activity, or a racemate, a stereoisomer or a pharmaceutically acceptable salt thereof.

### TECHNICAL SOLUTION

Technical solutions to achieve the objects of the present invention as descrived above are as follows:
1. A compound or a racemate, a stereoisomer or a pharmaceutically acceptable salt thereof as defined in claim 1.
   For reference and background, it is indicated that the conmpounds recited in claim 1 are compounds represented by the following Formula 1, but only the compounds recited in claim 1 and the racemates, stereoisomers and pharmaceutically acceptable salts thereof are according to the invention. 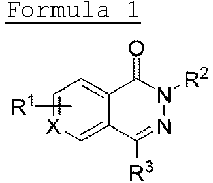 wherein,
   X is CH or N;
   R¹ is H, C₁₋₆ alkyl, or halo selected from the group consisting of fluoro, chloro and bromo;
   R² is selected from the group consisting of C₁₋₆ alkyl, aryl-substituted C₁₋₆ alkyl, aryl, heteroaryl, C₃₋₇ cycloalkyl and heterocyclyl;
   R³ is selected from the group consisting of aryl, heteroaryl and heterocyclyl,
   wherein each of the aryl, heteroaryl, cycloalkyl and heterocyclyl may be substituted with one to three substituents, and when substituted, the substituent thereof is each independently selected from the group consisting of halo selected from the group consisting of fluoro, chloro and bromo, nitro, cyano, hydroxy, oxo, C₁₋₆ alkyl unsubstituted or substituted with one to three halogen atoms, C₁₋₆ alkyloxy, aryl, heteroaryl, -NR⁴R⁵, -CH=N⁺(O⁻)R⁶, -C(O)R⁷, -SO₂R⁸, -C(=NOH)NR⁹R¹⁰, and -CR⁹R¹⁰R¹¹;
   R⁴ and R⁵ are each independently H, C₁₋₆ alkyl, C₁₋₆ alkyl substituted with one to three halogen atoms or hydroxy, -SO₂R¹², or -C(O)R¹⁵;
   R⁶ is C₁₋₆ alkyl or aryl-substituted C₁₋₆ alkyl;
   R⁷ is C₁₋₆ alkyloxy or -NR¹⁶R¹⁷;
   R⁸ is C₁₋₆ alkyl or -NR¹⁸R¹⁹;
   R⁹ and R¹⁰ are each independently H or C₁₋₆ alkyl, or R⁹ and R¹⁰ are joined with each other to form cycloalkyl together with carbon atom to which they are connected,
   R¹¹ is cyano, amino, unsubstituted or amino-substituted C₁₋₆ alkyl, unsubstituted or C₁₋₆ alkyl-substituted heteroaryl, heterocyclyl, -CO₂R²⁰, or -C(O)NR²⁰R²¹;
   R¹² is C₁₋₆ alkyl, C₁₋₆ alkyl substituted with one to three halogen atoms, a C₃₋₆ cycloalkyl, or -NR¹³R¹⁴;
   R¹³ and R¹⁴ are each independently H, C₁₋₆ alkyl or -CO₂R¹⁵;
   R¹⁵ is C₁₋₆ alkyl;
   R¹⁶ and R¹⁷ are each independently H, heterocyclyloxy, or hydroxy;
   R¹⁸ and R¹⁹ are each independently H or unsubstituted or hydroxy-substituted C₁₋₆ alkyl, or R¹⁸ and R¹⁹ are joined with each other to form heterocyclyl together with N atom to which they are connected;
   R²⁰ is H or C₁₋₆ alkyl, and R²¹ is H, unsubstituted or hydroxy-substituted C₁₋₆ alkyl, cycloalkyl, aryl, or unsubstituted or C₁₋₆ alkyl-substituted heteroaryl, or R²⁰ and R²¹ are joined with each other to form heterocyclyl together with N atom to which they are connected;
   the aryl is C₆₋₁₀ aromatic cyclic group;
   the cycloalkyl is C₃₋₇ aliphatic cyclic group;
   the heteroaryl is 5- or 6-membered heteroaromatic cyclic group containing one to four heteroatoms selected from N, O, and S in the cycle; and
   the heterocyclyl is 4- to 7-membered heterocyclic group including one or two heteroatoms selected from N and O in the cycle.
2. The pharmaceutical composition defined in claim 2.

### ADVANTAGEOUS EFFECTS

According to the present invention, a novel phthalazinone compound having excellent Sirt6 activity, or a racemate, a stereoisomer or a pharmaceutically acceptable salt thereof is provided. The compound of the present invention, or a racemate, a stereoisomer or a pharmaceutically acceptable salt thereof, has been found to have excellent Sirt6 activity and thus, it is expected to be useful for the prevention or treatment of Sirt6-related diseases.

### DETAILED DESCRIPTION OF THE EMBODIMENTS

The compound of the present invention may be provided in the form of a pharmaceutically acceptable salt.

Examples of the pharmaceutically acceptable salt may include an acid addition salt formed with a pharmaceutically acceptable free acid. The term "pharmaceutically acceptable salt" refers to an acid addition salt of the compound of the invention in which any side effects do not reduce the efficacy of the compound at a concentration having an effective action that is relatively non-toxic and harmless to a patient. Herein, the acid may be an organic acid or an inorganic acid. Examples of the inorganic acid include, but are not limited thereto, hydrochloric acid, phosphoric acid, sulfuric acid, nitric acid and the like, and examples of the organic acid include, but are not limited thereto, methanesulfonic acid, p-toluenesulfonic acid, acetic acid, trifluoroacetic acid, maleic acid, succinic acid, oxalic acid, benzoic acid, tartaric acid, fumaric acid, manderic acid, propanoic acid, citric acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid and the like.

Other examples of the pharmaceutically acceptable salt may include a metal salt prepared with a base. Examples of the metal salt include sodium salt, potassium salt, calcium salt and the like, of which calcium salt is particularly pharmaceutically appropriate, but are not limited thereto. In addition, the metal salt may also be provided in the form of a silver salt obtained by reacting an alkali metal salt or alkaline earth metal salt with an appropriate silver salt (e.g., silver nitrate).

Since the above-described salts are only examples, any pharmaceutically acceptable salt may be used as the salt of the compound of the invention without limitation as long as it has about the same level of Sirt6 activity as the compound itself.

Another aspect of the present invention relates to a pharmaceutical composition for the prevention or treatment of Sirt6-related diseases as defined in claim 2, comprising the compound of the invention, or a racemate, a stereoisomer or a pharmaceutically acceptable salt thereof.

Since the compound of the present invention, or a racemate, a stereoisomer or a pharmaceutically acceptable salt thereof, exhibits Sirt6 activity, it can be useful for the prevention or treatment of Sirt6-related diseases. In the present invention, the above-described Sirt6-related diseases are cancer, fatty liver, hepatocirrhosis/inflammation, decreased insulin secretion and diseases caused by aging.

In the present invention, the term "prevention" refers to any action that inhibits or delays the occurrence, spread, and recurrence of a Sirt6-related disease by administering a composition of the present invention, and the term "treatment" refers to any action that improves or beneficially changes symptoms of the disease by administering a composition of the present invention.

A pharmaceutical composition of the present invention may further comprise, in addition to the compound of the invention, or a racemate, a stereoisomer or a pharmaceutically acceptable salt thereof as an active ingredient, known drugs used for the prevention or treatment of each type of disease to be prevented or treated. For example, when used for the prevention or treatment of a Sirt6-related disease, the pharmaceutical composition may further comprise a known additive commonly used in the field of the present invention, for example, any one of a pharmaceutically acceptable carrier, an excipient and a diluent, in addition to the compound of the invention, or a racemate, a stereoisomer or a pharmaceutically acceptable salt thereof as an active ingredient, and the pharmaceutical composition may be used in combination with other treatments known to treat the disease. Examples of other treatments include chemotherapy, radiation therapy, hormone therapy, bone marrow transplantation, stem-cell replacement therapy, other biological therapies, and immunotherapy, but the present invention is not limited thereto.

### EXAMPLES

Hereinafter, the present invention will be described in more detail through Preparation Examples and Examples. However, these examples are merely illustrative of the present invention, and the scope of the present invention is not limited by these examples.

### Preparation Example 1: Preparation of Compound I-1 to Compound I-8

### Preparation Example 1-1. Preparation of 2-([1,1'-biphenyl]-4-yl)-4-chlorophthalazin-1(2H)-one (I-1)

Dichloromethane (DCM; 60 mL), phenylboronic acid (1.10 g, 9.00 mmol), copper acetate (3.24 g, 17.94 mmol) and pyridine (2.40 mL, 29.88 mmol) were added to 4-chlorophthalazin-1(2H)-one (1.08 g, 6.00 mmol) and then stirred at room temperature for 24 hours. After confirming The reaction mixture by thinlayer chromatography (TLC), the reaction solution was diluted with DCM and washed with purified water. Organic layer was dehydrated over anhydrous Na₂SO₄, concentrated under reduced pressure, and then reaction product was separated by column chromatography, to obtain 363 mg of the title compound.
C-MS (ESI, m/z) = 257.1 (M+H⁺)

### Preparation Example 1-2. Preparation of 4-chloro-2-(4-fluorophenyl)phthalazin-1(2H)-one (I-2)

4-Chlorophthalazin-1(2H)-one (1.08 g, 6.00 mmol) and 4-fluorophenylboronic acid (0.92 g, 6.58 mmol) were reacted in the same manner as in Preparation Example 1-1, to obtain 401 mg of the title compound.
LC-MS (ESI, m/z) = 274.9 (M+H⁺)

### Preparation Example 1-3. Preparation of 4-chloro-2-(4-trifluoromethyl)phenyl)phthalazin-1(2H)-one (I-3)

4-Chlorophthalazin-1(2H)-one (1.08 g, 6.00 mmol) and 4-(trifluoromethyl)phenylboronic acid (1.25 g, 6.58 mmol) were reacted in the same manner as in Preparation Example 1-1, to obtain 217 mg of the title compound.
LC-MS (ESI, m/z) = 325.0 (M+H⁺)

### Preparation Example 1-4. Preparation of 4-chloro-2-(4-methoxyphenyl)phthalazin-1(2H)-one (I-4)

4-Chlorophthalazin-1(2H)-one (1.08 g, 6.00 mmol) and 4-methoxyphenylboronic acid (1.00 g, 6.58 mmol) were reacted in the same manner as in Preparation Example 1-1, to obtain 432 mg of the title compound.
LC-MS (ESI, m/z) = 287.1 (M+H⁺)

### Preparation Example 1-5. Preparation of 4-chloro-2-(2-fluorophenyl)phthalazin-1(2H)-one (I-5)

4-Chlorophthalazin-1(2H)-one (1.08 g, 6.00 mmol) and 2-fluorophenylboronic acid (0.92 g, 6.58 mmol) were reacted in the same manner as in Preparation Example 1-1, to obtain 345 mg of the title compound.
LC-MS (ESI, m/z) = 274.9 (M+H⁺)

### Preparation Example 1-6. Preparation of 4-chloro-2-(3-fluorophenyl)phthalazin-1(2H)-one (I-6)

4-Chlorophthalazin-1(2H)-one (1.08 g, 6.00 mmol) and 3-fluorophenylboronic acid (0.92 g, 6.58 mmol) were reacted in the same manner as in Preparation Example 1-1, to obtain 381 mg of the title compound.
LC-MS (ESI, m/z) = 274.9 (M+H⁺)

### Preparation Example 1-7. Preparation of 4-chloro-2-(3,5-difluorophenyl)phthalazin-1(2H)-one (I-7)

4-Chlorophthalazin-1(2H)-one (1.08 g, 6.00 mmol) and 3,5-difluorophenylboronic acid (1.04 g, 6.58 mmol) were reacted in the same manner as in Preparation Example 1-1, to obtain 410 mg of the title compound.
LC-MS (ESI, m/z) = 293.2 (M+H⁺)

### Preparation Example 1-8. Preparation of 4-chloro-2-(3-chloro-4-fluorophenyl)phthalazin-1(2H)-one (I-8)

4-Chlorophthalazin-1(2H)-one (1.08 g, 6.00 mmol) and (3-chloro-4-fluorophenyl)boronic acid (1.15 g, 6.58 mmol) were reacted in the same manner as in Preparation Example 1-1, to obtain 388 mg of the title compound.
LC-MS (ESI, m/z) = 308.8 (M+H⁺)

### Preparation Example 2: Preparation of Compound I-9 and Compound I-10

### Preparation Example 2-1. Preparation of 4-oxo-3-(2-(trifluoromethyl)phenyl)-3,4-dihydrophthalazin-1-yl trifluoromethanesulfonate (I-9)

Step 1: Phthalic anhydride (5 g, 33.76 mmol) and 2,4-difluorophenylhydrazine hydrochloride (6.70 g, 37.13 mmol) were added to acetic acid (50 mL) and then stirred at 120 °C for 24 hours. The reaction solution was allowed to cool to room temperature, and after adding 100 mL of purified water and stirring for one hour, filtering was performed to separate precipitated solid. The obtained solid and NaOH (2.02 g, 50.63 mmol) were diluted in EtOH and then stirred at 40 °C for one or more hours. Subsequently, the reaction solution was concentrated under reduced pressure and diluted in purified water, pH of which was adjusted to 5-6 using 1N HCl. After stirring precipitated crystals for 10 or more minutes, filtering was performed, to obtain 5.1 g of 2-(2,4-difluorophenyl)-2,3-dihydrophthalazine-1,4-dione.
LC-MS (ESI, m/z) = 275.3 (M+H⁺)

Step 2: DCM (250 mL) and trimethylamine (3.05 mL, 21.88 mmol) were added to the compound (2.50 g, 9.12 mmol) obtained in Step 1. Subsequently, the resultant was cooled in an icesalt bath and, after adding trifluoromethanesulfonic acid anhydride (2.30 mL, 13.68 mmol) dropwise, resulting mixture was stirred at room temperature for two hours. The reaction product was diluted with DCM and washed with 2 N HCl, and then organic layer was extracted. The separated organic layer was dehydrated over anhydrous Na₂SO₄, concentrated under reduced pressure, and then reaction product was separated by column chromatography, to obtain 1.91 g of the title compound.
LC-MS (ESI, m/z) = 407.1 (M+H⁺)

### Preparation Example 2-2. Preparation of 3-(2,6-difluorophenyl)-4-oxo-3,4-dihydrophthalazin-1-yl trifluoromethanesulfonate (I-10)

Phthalic anhydride (5.00 g, 33.76 mmol) and 2,6-difluorophenylhydrazine hydrochloride (6.70 g, 37.13 mmol) were reacted in the same manner as in Preparation Example 2-1, to obtain 1.70 g of the title compound.
LC-MS (ESI, m/z) = 407.0 (M+H⁺)

### Preparation Example 3: Preparation of Compound I-11 to Compound I-14

### Preparation Example 3-1. Preparation of 4-chloro-6-fluoro-2-phenylphthalazin-1(2H)-one (I-11) and 4-chloro-7-fluoro-2-phenylphthalazin-1(2H)-one (I-12)

Step 1: Phenylhydrazine hydrochloride (13 g, 90.36 mmol) and 10% HCl (400 mL) were added to 4-fluorophthalic anhydride (10 g, 60.24 mmol) and then stirred at 110 °C for 24 hours. After confirming The reaction mixture by TLC, the reaction solution was diluted with EtOAc and washed with purified water. Organic layer was dehydrated over anhydrous Na₂SO₄, concentrated under reduced pressure, and then reaction product was separated by column chromatography, to obtain intermediate compounds 6-fluoro-2-phenyl-2,3-dihydrophthalazine-1,4-dione (4.70 g, 30.4% yield) and 7-fluoro-2-phenyl-2,3-dihydrophthalazine-1,4-dione (5.1 g, 33.0% yield) were synthesized.

Step 2: Phosphoryl chloride (25 mL) was added to the intermediate compounds 6-fluoro-2-phenyl-2,3-dihydrophthalazine-1,4-dione and 7-fluoro-2-phenyl-2,3-dihydrophthalazine-1,4-dione (1.07 g, 4.17 mmol) obtained in Step 1 and then stirred at 120 °C for two hours. The reaction mixture was allowed to cool to room temperature and, after distilling off an excess solvent, put in ice water to recover a solid product. The solid obtained by filtration was dissolved in DCM, neutralized by adding saturated aqueous NaHCO₃, and extracted with DCM. Organic layer was dehydrated over anhydrous Na₂SO₄, concentrated under reduced pressure, and then reaction product was separated by column chromatography, to obtain 550 mg of Compound I-11 and 428 mg of Compound I-12 were obtained.
LC-MS (ESI, m/z) = 275.0 (M+H⁺)

### Preparation Example 3-2. Preparation of 3-(2,4-difluorophenyl)-7-fluoro-4-oxo-3,4-dihydrophthalazin-1-yl trifluoromethanesulfonate (I-13) and 3-(2,4-difluorophenyl)-6-fluoro-4-oxo-3,4-dihydrophthalazin-1-yl trifluoromethanesulfonate (I-14)

Phthalic anhydride (5.00 g, 33.76 mmol) and 2,4-difluorophenylhydrazine hydrochloride (6.70 g, 37.13 mmol) were reacted in the same manner as in Preparation Example 3-1, to obtain 648 mg of Compound I-13 and 530 mg of Compound I-14 were obtained.
LC-MS (ESI, m/z) = 407.1 (M+H⁺)

### Preparation Example 4: Preparation of 2-(4-fluorophenyl)-1-oxo-1,2-dihydropyrido[3,4-d]pyridazin-4-yl trifluoromethanesulfonate (I-15)

Step 1: Furo[3,4-c]pyridine-1,3-dione (200 mg, 1.341 mmol) and (4-fluorophenyl)hydrazine hydrochloride (262 mg, 1.61 mmol) were diluted in acetic acid (2 mL) and then stirred at 120 °C for 15 hours. The temperature of the reaction solution was lowered to 0 °C to 5 °C, and after adding purified water, resulting mixture was stirred for 30 minutes, and filtered to separate precipitated solid. The obtained solid and NaOH (80 mg, 2.011 mmol) were diluted in EtOH and then stirred at 40 °C for one or more hours. The reaction solution was then concentrated under reduced pressure and diluted in purified water, and pH of which was adjusted to 6 using 1N HCl. After stirring precipitated crystals for 10 or more minutes, filtering was performed, to obtain 260 mg of 2-(4-fluorophenyl)-2,3-dihydropyrido[3,4-d]pyridazine-1,4-dione.
LC-MS (ESI, m/z) = 258.1(M+H⁺)

Step 2: The compound (200 mg, 0.778 mmol) obtained in Step 1 and tetraethylammonium (TEA; 217 µL, 1.555 mmol) were diluted in 5 mL of DCM, of which temperature was then lowered to -10 °C to -5 °C. Trifluoromethanesulfonic anhydride (158 µL, 0.933 mmol) diluted in 1 mL of DCM was added dropwise to the reaction solution for 10 minutes and then stirred for one hour while gradually increasing the temperature to room temperature. Subsequently, after diluting the reaction solution in an aqueous ammonium chloride solution and DCM, organic layer was dehydrated over Na₂SO₄, concentrated under reduced pressure, and then reaction product was separated by column chromatography, to obtain 64 mg of the title compound.
LC-MS (ESI, m/z) = 390.1(M+H⁺)

### Preparation Example 5: Preparation of methyl 2-methyl-2-(piperazin-2-yl)propionate (I-16)

Step 1: Dibenzylethylenediamine (1.61 g, 6.70 mmol), methyl (E)-4-bromobut-2-enoate (1.00 g, 6.00 mmol) and Et₃N (2.8 ml, 16.75 mmol) were dissolved in anhydrous toluene (20 ml) and then stirred at room temperature for 48 hours. The reaction product was diluted with EtOAc and washed with saturated aqueous NaHCO₃, and then organic layer was extracted. The separated organic layer was dehydrated over anhydrous Na₂SO₄, concentrated under reduced pressure, and then reaction product was separated by column chromatography, to obtain 1.1 g of methyl 2-(1,4-dibenzylpiperazin-2-yl)acetate.
LC-MS (ESI, m/z) = 339.2 (M+H⁺)

Step 2: The compound (1.00 g, 2.96 mmol) obtained in Step 1 was dissolved in anhydrous tetrahydrofuran (THF; 20 ml), and temperature was lowered to -78 °C. 2M Lithium diisopropylamide (LDA)-THF (5.9 ml, 11.80 mmol) was slowly added and stirred at -30 °C for one hour. After again lowering the temperature of the reaction solution to -78 °C, iodomethane (0.55 ml, 8.86 mmol) and hexamethylphosphoramide (HMPA; 10 µl, 0.06 mmol) were added. The temperature of the reaction mixture was slowly increased to 0 °C, and then stirring was performed at room temperature for five hours. Subsequently, the reaction product was diluted with EtOAc and washed with saturated aqueous NaHCO₃, and then organic layer was extracted. The separated organic layer was dehydrated over anhydrous Na₂SO₄, concentrated under reduced pressure, and then reaction product was separated by column chromatography, to obtain 360 mg of methyl 2-(1,4-dibenzylpiperazin-2-yl)-2-methylpropionate.
LC-MS (ESI, m/z) = 367.2 (M+H⁺)

Step 3: After dissolving the compound (260 mg, 0.71 mmol) obtained in Step 2 in anhydrous MeOH (10 ml), Pd/C (38 mg) and Pd(OH)₂ were added and stirred at room temperature for four hours. The reaction solution was filtered using a Celite Pad, washed with MeOH, concentrated under reduced pressure to remove an organic solvent, and then reaction product was separated by column chromatography, to obtain 125 mg of the title compound.
LC-MS (ESI, m/z) = 339.2 (M+H⁺)

### Example 1: N-(3-(4-Oxo-3-phenyl-3,4-dihydrophthalazin-1-yl)phenyl)ethanesulfonamide

Step 1: Compound I-1 (500 mg, 1.95 mmol), 3-aminophenylboronic acid (293 mg, 2.14 mmol), Pd(OAc)₂ (13 mg, 0.06 mmol) and sSPhos (60 mg, 0.12 mmol) were added to 1,4-dioxane/distilled water (14 mL, 1:1 ratio) and then stirred at 100 °C for five hours. The reaction product was diluted with EtOAc and washed with purified water. Organic layer was separated, dehydrated over anhydrous Na₂SO₄, concentrated under reduced pressure, and then reaction product was separated by column chromatography, to obtain 412 mg of 4-(3-aminophenyl)-2-phenylphthalazin-1(2H)-one.
LC-MS (ESI, m/z) = 314.4 (M+H⁺)

Step 2: Pyridine (10 mL) was added to the compound (200 mg, 0.63 mmol) obtained in Step 1 and, after adding ethanesulfonyl chloride (73 µL, 0.76 mmol) dropwise, resulting mixture was stirred at room temperature for five hours. The reaction product was diluted with EtOAc and washed with purified water. Organic layer was separated, dehydrated over anhydrous Na₂SO₄, and concentrated under reduced pressure, and then reaction product was separated by column chromatography, to obtain 122 mg of the title compound.
LC-MS (ESI, m/z) = 406.5 (M+H⁺)

### Example 2: 4-(3-Aminophenyl)-2-(4-(trifluoromethyl)phenyl)phthalazin-1(2H)-one

Compound I-3 (500 mg, 1.54 mmol) was used instead of Compound I-1 and reacted in the same manner as in Step 1 of Example 1, to obtain 408 mg of the title compound in white solid.
LC-MS (ESI, m/z) = 382.2 (M+H⁺)

### Example 3: N-(3-(4-Oxo-3-(4-(trifluoromethyl)phenyl)-3,4-dihydrophthalazin-1-yl)phenyl)ethanesulfonamide

The compound (100 mg, 0.26 mmol) of Example 2 was reacted in the same manner as in Step 2 of Example 1, to obtain 78 mg of the title compound.
LC-MS (ESI, m/z) = 473.9 (M+H⁺)

### Example 4: 4-(3-(Ethylamino)phenyl)-2-(4-(trifluoromethyl)phenyl)phthalazin-1(2H)-one

The compound (100 mg, 0.26 mmol) of Example 2, acetaldehyde (24 µL, 0.40 mmol) and an 8M borane-pyridine complex (50 µL, 0.40 mmol) were dissolved in MeOH (7 mL) and then reacted at room temperature for 24 hours in the presence of a 4Å molecular sieve. The reaction solution was filtered using a Celite Pad, washed with MeOH, concentrated under reduced pressure to remove an organic solvent, and then reaction product was separated by column chromatography, to obtain 45 mg of the title compound in white solid.
LC-MS (ESI, m/z) = 410.5 (M+H⁺)

### Example 5: N-Methyl-N-(3-(4-oxo-3-(4-(trifluoromethyl)phenyl)-3,4-dihydrophthalazin-1-yl)phenyl)ethanesulfonamide

The compound (50 mg, 0.11 mmol) of Example 3 was dissolved in dimethylformamide (DMF; 2 mL) and, after adding K₂CO₃ (30 mg, 0.22 mmol) and iodomethane (8 µL, 0.13 mmol), reacted at 50 °C for 24 hours. After diluting The reaction mixture with EtOAc and washing with purified water, organic layer was dehydrated over anhydrous Na₂SO₄, concentrated under reduced pressure, and then reaction product was separated by column chromatography, to obtain 45 mg of the title compound.
LC-MS (ESI, m/z) = 474.1 (M+H⁺)

### Example 6: N-(3-(4-Oxo-3-phenyl-3,4-dihydrophthalazin-1-yl)phenyl)methanesulfonamide

The compound (50 mg, 0.16 mmol) obtained in Step 1 of Example 1 and methanesulfonyl chloride (14.8 µL, 0.19 mmol) were reacted in the same manner as in Step 2 of Example 1, to obtain 48 mg of the title compound.
LC-MS (ESI, m/z) = 392.0 (M+H⁺)

### Example 7: N-Methyl-N-(3-(4-oxo-3-phenyl-3,4-dihydro-phthalazin-1-yl)phenyl)methanesulfonamide

The compound (20 mg, 0.05 mmol) of Example 6 was reacted in the same manner as in Example 5, to obtain 21 mg of the title compound.
LC-MS (ESI, m/z) = 406.1 (M+H⁺)

### Example 8: N-(4-(4-Oxo-3-phenyl-3,4-dihydrophthalazin-1-yl)phenyl)ethanesulfonamide

Compound I-1 (500 mg, 1.95 mmol) and 4-aminophenylboronic acid (293 mg, 2.14 mmol) were reacted in the same manner as in Example 1, to obtain 187 mg of the title compound.
LC-MS (ESI, m/z) = 406.5 (M+H⁺)

### Example 9: N-(3-(3-Phenyl)-4-oxo-3,4-dihydro-phthalazin-1-yl)phenyl)-dimethylsulfamoylamide

The compound (50 mg, 0.16 mmol) obtained in Step 1 of Example 1 and N,N-dimethylsulfamoyl chloride (20 µL, 0.19 mmol) were reacted in the same manner as in Step 2 of Example 1, to obtain 41 mg of the title compound.
LC-MS (ESI, m/z) = 421.1 (M+H⁺)

### Example 10: N-(3-(3-(4-Fluorophenyl)-4-oxo-3,4-dihydrophthalazin-1-yl)phenyl)methanesulfonamide

Step 1: Compound I-2 (500 mg, 1.83 mmol) and 3-aminophenylboronic acid (375 mg, 2.74 mmol) were reacted in the same manner as in Step 1 of Example 1, to obtain 440 mg of 4-(3-aminophenyl)-2-(4-fluorophenyl)phthalazin-1(2H)-one.
LC-MS (ESI, m/z) = 332.1 (M+H⁺)

Step 2: The compound (100 mg, 0.30 mmol) obtained in Step 1 and methanesulfonic anhydride (68 µL, 0.36 mmol) were reacted in the same manner as in Step 2 of Example 1, to obtain 78 mg of the title compound.
LC-MS (ESI, m/z) = 410.0 (M+H⁺)

### Example 11: N-(3-(3-(4-Fluorophenyl)-4-oxo-3,4-dihydrophthalazin -1-yl)phenyl)ethanesulfonamide

The compound (50 mg, 0.15 mmol) obtained in Step 1 of Example 10 and ethanesulfonyl chloride (17 µL, 0.18 mmol) were reacted in the same manner as in Step 2 of Example 1, to obtain 47 mg of the title compound in white solid.
LC-MS (ESI, m/z) = 424.2 (M+H⁺)

### Example 12: N-(3-(3-(4-Fluorophenyl)-4-oxo-3,4-dihydrophthalazin-1-yl)phenyl)dimethylsulfamoylamide

The compound (50 mg, 0.15 mmol) obtained in Step 1 of Example 10 and N,N-dimethylsulfamoyl chloride (19 µL, 0.18 mmol) were reacted in the same manner as in Step 2 of Example 1, to obtain 34 mg of the title compound.
LC-MS (ESI, m/z) = 439.2 (M+H⁺)

### Example 13: (Z)-N-Methyl-1-(3-(4-oxo-3-phenyl-3,4-dihydrophthalazin-1-yl)phenyl)methanimine oxide

Step 1: Compound I-1 (1 g, 3.02 mmol), 3-formylphenylboronic acid (543 mg, 3.62 mmol) and Pd(PPh₃)₄ (104 mg, 0.09 mmol) were added to 1,4-dioxane (50 mL) and, after also adding Na₂CO₃ (1.5 g, 14.48 mmol) dissolved in H₂O (15 mL), resulting mixture was stirred at 110 °C for two hours. The reaction product was diluted with EtOAc and washed with purified water, and then organic layer was separated, dehydrated over anhydrous Na₂SO₄, concentrated under reduced pressure, and then reaction product was separated by column chromatography, to obtain 1.2 g of 3-(4-oxo-3-phenyl-3,4-dihydrophthalazin-1-yl)benzaldehyde.
LC-MS (ESI, m/z) = 327.1 (M+H⁺)

Step 2: The compound (100 mg, 0.31 mmol) obtained in Step 1 was dissolved in anhydrous EtOH (5 mL) and, after adding N-methylhydroxylamine hydrochloride (128 mg, 1.53 mmol) and potassium acetate (150 mg, 1.53 mmol), resulting mixture was stirred at room temperature for 48 hours. After concentred to remove the EtOH solvent, dilution with EtOAc and washing with purified water were performed. Organic layer was separated, dehydrated over anhydrous Na₂SO₄, concentrated under reduced pressure, and then reaction product was separated by column chromatography, to obtain 45 mg of the title compound.
LC-MS (ESI, m/z) = 356.1 (M+H⁺)

### Example 14: (Z)-N-Isopropyl-1-(3-(4-oxo-3-phenyl-3,4-dihydrophthalazin-1-yl)phenyl)methanimine oxide

The compound (100 mg, 0.31 mmol) obtained in Step 1 of Example 13 and N-isopropylhydroxylamine hydrochloride (170 mg, 1.53 mmol) were reacted in the same manner as in Step 2 of Example 13, to obtain 51 mg of the title compound.
LC-MS (ESI, m/z) = 384.2 (M+H⁺)

### Example 15: (Z)-1-(3-(3-(4-Fluorophenyl)-4-oxo-3,4-dihydrophthalazin-1-yl)phenyl)-N-methylmethanimine oxide

Compound I-2 (100 mg, 0.36 mmol) was used as a starting material and reacted in the same manner as in Example 13, to obtain 42 mg of the title compound.
LC-MS (ESI, m/z) = 374.1 (M+H⁺)

### Example 16: (Z)-N-Ethyl-1-(3-(3-(4-fluorophenyl)-4-oxo-3,4-dihydrophthalazin-1-yl)phenyl)methanimine oxide

Compound I-2 (100 mg, 0.36 mmol) and N-ethylhydroxylamine hydrochloride (174 mg, 1.8 mmol) were reacted in the same manner as in Example 13, to obtain 54 mg of the title compound.
LC-MS (ESI, m/z) = 388.1 (M+H⁺)

### Example 17: (Z)-1-(3-(3-(4-Fluorophenyl)-4-oxo-3,4-dihydrophthalazin-1-yl)phenyl)-N-isopropylmethanimine oxide

Compound I-2 (100 mg, 0.36 mmol) and N-isopropylhydroxylamine hydrochloride (199 mg, 1.8 mmol) were reacted in the same manner as in Example 13, to obtain 44 mg of the title compound.
LC-MS (ESI, m/z) = 402.2 (M+H⁺)

### Example 18: N-(2-Chloro-5-(4-oxo-3-phenyl-3,4-dihydrophthalazin -1-yl)phenyl)-N-(methylsulfonyl)methane-sulfonamide

Compound I-1 (100 mg, 0.39 mmol), (3-amino-4-chlorophenyl)boronic acid (80 mg, 0.47 mmol) and methanesulfonic anhydride (162 µL, 0.85 mmol) were reacted in the same manner as in Example 10, to obtain 37 mg of the title compound.
LC-MS (ESI, m/z) = 504.04 (M+H⁺)

### Example 19: N-(3-(3-(Phenyl)-4-oxo-3,4-dihydrophthalazin-1-yl)-6-chlorophenyl)dimethyl sulfamoylamide

Compound I-1 (100 mg, 0.39 mmol), (3-amino-4-chlorophenyl)boronic acid (80 mg, 0.47 mmol) and N,N-dimethylsulfamoyl chloride (50 µL, 0.47 mmol) were reacted in the same manner as in Example 10, to obtain 54 mg of the title compound.
LC-MS (ESI, m/z) = 455.09 (M+H⁺)

### Example 20: Methyl (N-(3-(3-(4-fluorophenyl)-4-oxo-3,4-dihydrophthalazin-1-yl)phenyl)sulfamoyl) carbamate

The compound (100 mg, 0.30 mmol) obtained in Step 1 of Example 11 and methyl (chlorosulfonyl)carbamate (62 mg, 0.36 mmol) were reacted in the same manner as in Step 2 of Example 1, to obtain 77 mg of the title compound in white solid.
LC-MS (ESI, m/z) = 469.0 (M+H⁺)

### Example 21: (Z)-1-(3-(3-(2,4-Difluorophenyl)-4-oxo-3,4-dihydrophthalazin-1-yl)phenyl)-N-methyl methanimine oxide

Compound I-9 (100 mg, 0.34 mmol) was used instead of Compound I-1 and reacted in the same manner as in Example 13, to obtain 50 mg of the title compound.
LC-MS (ESI, m/z) = 392.1 (M+H⁺)

### Example 22: (Z)-1-(3-(3-(2,4-Difluorophenyl)-4-oxo-3,4-dihydrophthalazin-1-yl)phenyl)-N-isopropyl methanimine oxide

Compound I-9 (100 mg, 0.34 mmol) and N-isopropylhydroxylamine hydrochloride (190 mg, 1.70 mmol) were reacted in the same manner as in Example 13, to obtain 58 mg of the title compound.
LC-MS (ESI, m/z) = 420.0 (M+H⁺)

### Example 23: (Z)-N-tert-Butyl-1-(3-(3-(2,4-difluoro phenyl)-4-oxo-3,4-dihydrophthalazin-1-yl)phenyl)methanimine oxide

Compound I-9 (100 mg, 0.34 mmol) and N-tert-butylhydroxylamine hydrochloride (212 mg, 1.70 mmol) were reacted in the same manner as in Example 13, to obtain 36 mg of the title compound.
LC-MS (ESI, m/z) = 434.2 (M+H⁺)

### Example 24: 4-(3-Aminophenyl)-2-(2,4-difluorophenyl)phthalazin-1(2H)-one

Compound I-9 (1.00 g, 3.41 mmol) and 3-aminophenylboronic acid (513 mg, 3.75 mmol) were reacted in the same manner as in Step 1 of Example 1, to obtain 801 mg of the title compound.
LC-MS (ESI, m/z) = 349.9 (M+H⁺)

### Example 25: N-(3-(3-(2,4-Difluorophenyl)-4-oxo-3,4-dihydrophthalazin-1-yl)phenyl)ethanesulfonamide

The compound (50 mg, 0.14 mmol) obtained in Example 24 and ethanesulfonyl chloride (16 µL, 0.17 mmol) were reacted in the same manner as in Step 2 of Example 1, to obtain 33 mg of the title compound in white solid.
LC-MS (ESI, m/z) = 442.1 (M+H⁺)

### Example 26: Methyl (N-(3-(3-(2,4-difluorophenyl)-4-oxo-3,4-dinydrophthalazin-1-yl)phenyl)sulfamoyl)carbamate

The compound (100 mg, 0.28 mmol) of Example 24 and methyl (chlorosulfonyl)carbamate (57 mg, 0.33 mmol) were reacted in the same manner as in Step 2 of Example 1, to obtain 71 mg of the title compound in white solid.
LC-MS (ESI, m/z) = 487.0 (M+H⁺)

### Example 27: (Z)-1-(3-(3-(3-Fluorophenyl)-4-oxo-3,4-dihydrophthalazin-1-yl)phenyl)-N-methylmethan imine oxide

Compound I-6 (100 mg, 0.36 mmol), 3-formylphenylboronic acid (64 mg, 0.43 mmol) and N-methylhydroxylamine hydrochloride (151 mg, 1.80 mmol) were reacted in the same manner as in Example 13, to obtain 42 mg of the title compound.
LC-MS (ESI, m/z) = 374.0 (M+H⁺)

### Example 28: (Z)-1-(3-(3-(3-Fluorophenyl)-4-oxo-3,4-dihydrophthalazin-1-yl)phenyl)-N-isopropylmethan imine oxide

Compound I-6 (100 mg, 0.36 mmol), 3-formylphenylboronic acid (64 mg, 0.43 mmol) and N-isopropylhydroxylamine hydrochloride (201 mg, 1.80 mmol) were reacted in the same manner as in Example 13, to obtain 55 mg of the title compound.
LC-MS (ESI, m/z) = 402.2 (M+H⁺)

### Example 29: (Z)-N-tert-Butyl-1-(3-(3-(3-fluorophenyl)-4-oxo-3,4-dihydrophthalazin-1-yl)phenyl)methanimine oxide

Compound I-6 (100 mg, 0.36 mmol), 3-formylphenylboronic acid (64 mg, 0.43 mmol) and N-tert-butylhydroxylamine hydrochloride (226 mg, 1.80 mmol) were reacted in the same manner as in Example 13, to obtain 30 mg of the title compound.
LC-MS (ESI, m/z) = 416.1 (M+H⁺)

### Example 30: N-(3-(3-(3-Fluorophenyl)-4-oxo-3,4-dihydro phthalazin-1-yl)phenyl)ethanesulfonamide

Compound I-6 (100 mg, 0.36 mmol), 3-aminophenylboronic acid (59 mg, 0.43 mmol) and ethenesulfonyl chloride (41 µL, 0.43 mmol) were reacted in the same manner as in Example 1, to obtain 66 mg of the title compound.
LC-MS (ESI, m/z) = 424.1 (M+H⁺)

### Example 31: (Z)-1-(3-(3-(3,5-Difluorophenyl)-4-oxo-3,4-dihydrophthalazin-1-yl)phenyl)-N-isopropyl methanimine oxide

Compound I-7 (100 mg, 0.34 mmol), 3-formylphenylboronic acid (64 mg, 0.43 mmol) and N-isopropylhydroxylamine hydrochloride (201 mg, 1.80 mmol) were reacted in the same manner as in Example 13, to obtain 64 mg of the title compound.
LC-MS (ESI, m/z) = 420.1 (M+H⁺)

### Example 32: (Z)-N-Isopropyl-1-(3-(3-(4-methoxy phenyl)-4-oxo-3,4-dihydrophthalazin-1-yl)phenyl) methanimine oxide

Compound I-4 (100 mg, 0.35 mmol), 3-formylphenylboronic acid (62 mg, 0.42 mmol) and N-isopropylhydroxylamine hydrochloride (196 mg, 1.75 mmol) were reacted in the same manner as in Example 13, to obtain 55 mg of the title compound.
LC-MS (ESI, m/z) = 414.1 (M+H⁺)

### Example 33: (Z)-1-(3-(3-(3-Chloro-4-fluorophenyl)-4-oxo-3,4-dihydrophthalazin-1-yl)phenyl)-N-isopropyl-methanimine oxide

Compound I-8 (100 mg, 0.33 mmol), 3-formylphenylboronic acid (60 mg, 0.40 mmol) and N-isopropylhydroxylamine hydrochloride (184 mg, 1.65 mmol) were reacted in the same manner as in Example 13, to obtain 47 mg of the title compound.
LC-MS (ESI, m/z) = 436.1 (M+H⁺)

### Example 34: (Z)-N-tert-Butyl-1-(3-(3-(3-chloro-4-fluorophenyl)-4-oxo-3,4-dihydrophthalazin-1-yl)phenyl) methanimine oxide

Compound I-8 (100 mg, 0.33 mmol), 3-formylphenylboronic acid (60 mg, 0.40 mmol) and N-tert-butylhydroxylamine hydrochloride (208 mg, 1.65 mmol) were reacted in the same manner as in Example 13, to obtain 41 mg of the title compound.
LC-MS (ESI, m/z) = 450.3 (M+H⁺)

### Example 35: (Z)-N-Benzyl-1-(3-(4-oxo-3-phenyl-3,4-dihydrophthalazin-1-yl)phenyl)methanimine oxide

Compound I-1 (100 mg, 0.38 mmol), 3-formylphenylboronic acid (68 mg, 0.45 mmol) and N-benzylhydroxylamine hydrochloride (302 mg, 1.90 mmol) were reacted in the same manner as in Example 13, to obtain 60 mg of the title compound.
LC-MS (ESI, m/z) = 432.1 (M+H⁺)

### Example 36: (E)-1-(4-(3-(4-Fluorophenyl)-4-oxo-3,4-dihydrophthalazin-1-yl)phenyl)-N-methylmethanimine oxide

Compound I-2 (100 mg, 0.36 mmol), 4-formylphenylboronic acid (65 mg, 0.43 mmol) and N-methylhydroxylamine hydrochloride (149 mg, 1.80 mmol) were reacted in the same manner as in Example 13, to obtain 51 mg of the title compound.
LC-MS (ESI, m/z) = 374.0 (M+H⁺)

### Example 37: (Z)-1-(3-(3-(4-Fluorophenyl)-4-oxo-3,4-dihydrophthalazin-1-yl)phenyl)-N-isopropyl methanimine oxide

Compound I-2 (100 mg, 0.36 mmol), 3-formylphenylboronic acid (65 mg, 0.43 mmol) and N-isopropylhydroxylamine hydrochloride (200 mg, 1.80 mmol) were reacted in the same manner as in Example 13, to obtain 43 mg of the title compound.
LC-MS (ESI, m/z) = 402.2 (M+H⁺)

### Example 38: (E)-1-(3-Fluoro-4-(3-(4-fluorophenyl)-4-oxo-3,4-dihydrophthalazin-1-yl)phenyl)-N-methylmethanimine oxide

Compound I-2 (100 mg, 0.36 mmol), (2-fluoro-4-formylphenyl)boronic acid (72 mg, 0.43 mmol) and N-methylhydroxylamine hydrochloride (149 mg, 1.80 mmol) were reacted in the same manner as in Example 13, to obtain 37 mg of the title compound.
LC-MS (ESI, m/z) = 392.1 (M+H⁺)

### Example 39: (E)-1-(3-Fluoro-4-(3-(4-fluorophenyl)-4-oxo-3,4-dihydrophthalazin-1-yl)phenyl)-N-isopropylmethan imine oxide

Compound I-2 (100 mg, 0.36 mmol), (2-fluoro-4-formylphenyl)boronic acid (72 mg, 0.43 mmol) and N-isopropylhydroxylamine hydrochloride (200 mg, 1.80 mmol) were reacted in the same manner as in Example 13, to obtain 44 mg of the title compound.
LC-MS (ESI, m/z) = 420.0 (M+H⁺)

### Example 40: (Z)-1-(2-(3-(4-Fluorophenyl)-4-oxo-3,4-dihydrophthalazin-1-yl)phenyl)-N-methylmethanimine oxide

Compound I-2 (100 mg, 0.36 mmol), 2-formylphenylboronic acid (65 mg, 0.43 mmol) and N-methylhydroxylamine hydrochloride (149 mg, 1.80 mmol) were reacted in the same manner as in Example 13, to obtain 54 mg of the title compound.
LC-MS (ESI, m/z) = 374.3 (M+H⁺)

### Example 41: (Z)-1-(2-(3-(4-Fluorophenyl)-4-oxo-3,4-dihydrophthalazin-1-yl)phenyl)-N-isopropyl methanimine oxide

Compound I-2 (100 mg, 0.36 mmol), 2-formylphenylboronic acid (65 mg, 0.43 mmol) and N-isopropylhydroxylamine hydrochloride (200 mg, 1.80 mmol) were reacted in the same manner as in Example 13, to obtain 30 mg of the title compound.
LC-MS (ESI, m/z) = 402.1 (M+H⁺)

### Example 42: (Z)-N-tert-Butyl-1-(2-(3-(4-fluorophenyl)-4-oxo-3,4-dihydrophthalazin-1-yl)phenyl)methanimine oxide

Compound I-2 (100 mg, 0.36 mmol), 2-formylphenylboronic acid (65 mg, 0.43 mmol) and N-tert-butylhydroxylamine hydrochloride (225 mg, 1.80 mmol) were reacted in the same manner as in Example 13, to obtain 39 mg of the title compound.
LC-MS (ESI, m/z) = 416.1 (M+H⁺)

### Example 43: (Z)-N-tert-Butyl-1-(3-(3-(4-fluorophenyl)-4-oxo-3,4-dihydrophthalazin-1-yl)phenyl)methanimine oxide

Compound I-2 (100 mg, 0.36 mmol), 3-formylphenylboronic acid (65 mg, 0.43 mmol) and N-tert-butylhydroxylamine hydrochloride (225 mg, 1.80 mmol) were reacted in the same manner as in Example 13, to obtain 48 mg of the title compound.
LC-MS (ESI, m/z) = 416.2 (M+H⁺)

### Example 44: (Z)-N-Benzyl-1-(3-(3-(4-fluorophenyl)-4-oxo-3,4-dihydrophthalazin-1-yl)phenyl)methanimine oxide

Compound I-2 (100 mg, 0.36 mmol), 3-formylphenylboronic acid (65 mg, 0.43 mmol) and N-benzylhydroxylamine hydrochloride (286 mg, 1.80 mmol) were reacted in the same manner as in Example 13, to obtain 62 mg of the title compound.
LC-MS (ESI, m/z) = 450.0 (M+H⁺)

### Example 45: (Z)-1-(5-(3-(4-Fluorophenyl)-4-oxo-3,4-dihydrophthalazin-1-yl)thiophen-2-yl)-N-methylmethanimine oxide

Compound I-2 (100 mg, 0.36 mmol), 5-formyl-2-thienylboronic acid (67 mg, 0.43 mmol) and N-methylhydroxylamine hydrochloride (149 mg, 1.80 mmol) were reacted in the same manner as in Example 13, to obtain 55 mg of the title compound.
LC-MS (ESI, m/z) = 380.3 (M+H⁺)

### Example 46: (Z)-1-(5-(3-(4-Fluorophenyl)-4-oxo-3,4-dihydrophthalazin-1-yl)thiophen-2-yl)-N-isopropylmethan imine oxide

Compound I-2 (100 mg, 0.36 mmol), 5-formyl-2-thienylboronic acid (67 mg, 0.43 mmol) and N-isopropylhydroxylamine hydrochloride (200 mg, 1.80 mmol) were reacted in the same manner as in Example 13, to obtain 47 mg of the title compound.
LC-MS (ESI, m/z) = 407.9 (M+H⁺)

### Example 47: (Z)-N-tert-Butyl-1-(5-(3-(4-fluorophenyl)-4-oxo-3,4-dihydrophthalazin-1-yl)thiophen-2-yl)methanimine oxide

Compound I-2 (100 mg, 0.36 mmol), 5-formyl-2-thienylboronic acid (67 mg, 0.43 mmol) and N-tert-butylhydroxylamine hydrochloride (225 mg, 1.80 mmol) were reacted in the same manner as in Example 13, to obtain 35 mg of the title compound.
LC-MS (ESI, m/z) = 422.2 (M+H⁺)

### Example 48: (Z)-1-(5-(3-(4-Fluorophenyl)-4-oxo-3,4-dihydrophthalazin-1-yl)furan-2-yl)-N-isopropylmethanimine oxide

Compound I-2 (100 mg, 0.36 mmol), 5-formyl-2-furanylboronic acid (60 mg, 0.43 mmol) and N-isopropylhydroxylamine hydrochloride (200 mg, 1.80 mmol) were reacted in the same manner as in Example 13, to obtain 40 mg of the title compound.
LC-MS (ESI, m/z) = 392.1 (M+H⁺)

### Example 49: (Z)-N-tert-Butyl-1-(5-(3-(4-fluorophenyl)-4-oxo-3,4-dihydrophthalazin-1-yl)-2-methoxyphenyl)methan imine oxide

Compound I-2 (100 mg, 0.36 mmol), 3-formyl-4-methoxyphenylboronic acid (77 mg, 0.43 mmol) and N-tert-butylhydroxylamine hydrochloride (225 mg, 1.80 mmol) were reacted in the same manner as in Example 13, to obtain 45 mg of the title compound.
LC-MS (ESI, m/z) = 446.2 (M+H⁺)

### Example 50: (Z)-N-Benzyl-1-(5-(3-(4-fluorophenyl)-4-oxo-3,4-dihydrophthalazin-1-yl)-2-methoxyphenyl)methanimine oxide

Compound I-2 (100 mg, 0.36 mmol), 3-formyl-4-methoxyphenylboronic acid (77 mg, 0.43 mmol) and N-benzylhydroxylamine hydrochloride (286 mg, 1.80 mmol) were reacted in the same manner as in Example 13, to obtain 50 mg of the title compound.
LC-MS (ESI, m/z) = 480.0 (M+H⁺)

### Example 51: (Z)-1-(2-Fluoro-5-(3-(4-fluorophenyl)-4-oxo-3,4-dihydrophthalazin-1-yl)phenyl)-N-methylmethanimine oxide

Compound I-2 (100 mg, 0.36 mmol), 4-fluoro-3-formylphenylboronic acid (72 mg, 0.43 mmol) and N-methylhydroxylamine hydrochloride (149 mg, 1.80 mmol) were reacted in the same manner as in Example 13, to obtain 43 mg of the title compound.
LC-MS (ESI, m/z) = 392.1 (M+H⁺)

### Example 52: (Z)-1-(2-Fluoro-5-(3-(4-fluorophenyl)-4-oxo-3,4-dihydrophthalazin-1-yl)phenyl)-N-isopropylmethan imine oxide

Compound I-2 (100 mg, 0.36 mmol), 4-fluoro-3-formylphenylboronic acid (72 mg, 0.43 mmol) and N-isopropylhydroxylamine hydrochloride (200 mg, 1.80 mmol) were reacted in the same manner as in Example 13, to obtain 47 mg of the title compound.
LC-MS (ESI, m/z) = 420.0 (M+H⁺)

### Example 53: (Z)-N-tert-Butyl-1-(2-fluoro-5-(3-(4-fluorophenyl)-4-oxo-3,4-dihydrophthalazin-1-yl)phenyl) methanimine oxide

Compound I-2 (100 mg, 0.36 mmol), 4-fluoro-3-formylphenylboronic acid (72 mg, 0.43 mmol) and N-tert-butylhydroxylamine hydrochloride (225 mg, 1.80 mmol) were reacted in the same manner as in Example 13, to obtain 30 mg of the title compound.
LC-MS (ESI, m/z) = 434.1 (M+H⁺)

### Example 54: (Z)-N-Benzyl-1-(2-fluoro-5-(3-(4-fluorophenyl)-4-oxo-3,4-dihydrophthalazin-1-yl)phenyl) methanimine oxide

Compound I-2 (100 mg, 0.36 mmol), 4-fluoro-3-formylphenylboronic acid (72 mg, 0.43 mmol) and N-benzylhydroxylamine hydrochloride (286 mg, 1.80 mmol) were reacted in the same manner as in Example 13, to obtain 57 mg of the title compound.
LC-MS (ESI, m/z) = 467.9 (M+H⁺)

### Example 55: (Z)-1-(4-Fluoro-3-(3-(4-fluorophenyl)-4-oxo-3,4-dihydrophthalazin-1-yl)phenyl)-N-methylmethanimine oxide

Compound I-2 (100 mg, 0.36 mmol), 2-fluoro-5-formylphenylboronic acid (72 mg, 0.43 mmol) and N-methylhydroxylamine hydrochloride (149 mg, 1.80 mmol) were reacted in the same manner as in Example 13, to obtain 46 mg of the title compound.
LC-MS (ESI, m/z) = 392.1 (M+H⁺)

### Example 56: (Z)-1-(4-Fluoro-3-(3-(4-fluorophenyl)-4-oxo-3,4-dihydrophthalazin-1-yl)phenyl)-N-isopropylmethan imine oxide

Compound I-2 (100 mg, 0.36 mmol), 2-fluoro-5-formylphenylboronic acid (72 mg, 0.43 mmol) and N-isopropylhydroxylamine hydrochloride (200 mg, 1.80 mmol) were reacted in the same manner as in Example 13, to obtain 42 mg of the title compound.
LC-MS (ESI, m/z) = 419.8 (M+H⁺)

### Example 57: (Z)-N-tert-Butyl-1-(4-fluoro-3-(3-(4-fluorophenyl)-4-oxo-3,4-dihydrophthalazin-1-yl)phenyl) methanimine oxide

Compound I-2 (100 mg, 0.36 mmol), 2-fluoro-5-formylphenylboronic acid (72 mg, 0.43 mmol) and N-tert-butylhydroxylamine hydrochloride (225 mg, 1.80 mmol) were reacted in the same manner as in Example 13, to obtain 31 mg of the title compound.
LC-MS (ESI, m/z) = 434.1 (M+H⁺)

### Example 58: (Z)-N-Benzyl-1-(4-fluoro-3-(3-(4-fluorophenyl)-4-oxo-3,4-dihydrophthalazin-1-yl)phenyl) methanimine oxide

Compound I-2 (100 mg, 0.36 mmol), 2-fluoro-5-formylphenylboronic acid (72 mg, 0.43 mmol) and N-benzylhydroxylamine hydrochloride (286 mg, 1.80 mmol) were reacted in the same manner as in Example 13, to obtain 43 mg of the title compound.
LC-MS (ESI, m/z) = 468.0 (M+H⁺)

### Example 59: 4-(4-Chloro-3-nitrophenyl)-2-(4-fluorophenyl)phthalazin-1(2H)-one

Compound I-2 (100 mg, 0.36 mmol) and 4-chloro-3-nitrophenylboronic acid (86 mg, 0.43 mmol) were reacted in the same manner as in Step 1 of Example 1, to obtain 125 mg of the title compound.
LC-MS (ESI, m/z) = 396.0 (M+H⁺)

### Example 60: 4-(3-Aminophenyl)-2-(4-fluorophenyl) phthalazin-1(2H)-one

Compound I-2 (1.00 g, 3.64 mmol) and 3-aminophenylboronic acid (548 mg, 4.00 mmol) were reacted in the same manner as in Step 1 of Example 1, to obtain 1.14 g of the title compound.
LC-MS (ESI, m/z) = 332.1 (M+H⁺)

### Example 61: N-(3-(3-Benzyl-4-oxo-3,4-dihydrophthalazin-1-yl)phenyl)ethanesulfonamide

Step 1: 4-Chlorophthalazin-1(2H)-one (200 mg, 1.10 mmol) was dissolved in DMF (10 mL), of which temperature was then lowered to 0 °C. After adding NaH (50 mg, 1.3 mmol) and stirring for 10 minutes at 0 °C, benzyl bromide (145 µL, 1.20 mmol) was slowly added. After reacting at room temperature for two hours, completion of the reaction was confirmed by TLC, and the reaction solution was diluted with EtOAc and washed with purified water. Organic layer was dehydrated over anhydrous Na₂SO₄, concentrated under reduced pressure, and then reaction product was separated by column chromatography, to obtain 170 mg of 2-benzyl-4-chlorophthalazin-1(2H)-one.
LC-MS (ESI, m/z) = 271.0 (M+H⁺)

Step 2: The compound (100 mg, 0.37 mmol) obtained in Step 1 was dissolved in dioxane (5 mL), and then Pd(dppf)Cl₂ (27 mg, 10 mol%), (3-aminophenyl)boronic acid (56 mg, 0.40 mmol), K₃PO₄ (235 mg, 1.10 mmol) and sSPhos (19 mg, 10 mol%) were added. The resulting mixture was stirred at 100 °C for eight hours, and then the reaction solution was diluted with EtOAc and washed with purified water. Organic layer was dehydrated over anhydrous Na₂SO₄, concentrated under reduced pressure, and then reaction product was separated by column chromatography, to obtain 83 mg of 4-(3-aminophenyl)-2-benzylphthalazin-1(2H)-one.
LC-MS (ESI, m/z) = 328.2 (M+H⁺)

Step 3: The compound (50 mg, 0.15 mmol) obtained in Step 2 was reacted in the same manner as in Step 2 of Example 1, to obtain 34 mg of the title compound.
LC-MS (ESI, m/z) = 420.1 (M+H⁺)

### Example 62: N-(3-(4-Oxo-3-phenethyl-3,4-dihydro phthalazin-1-yl)phenyl)ethanesulfonamide

(2-Bromoethyl)benzene (147 µL, 1.08 mmol) was used instead of benzyl bromide and reacted in the same manner as in Example 61, to obtain 61 mg of the title compound.
LC-MS (ESI, m/z) = 434.0 (M+H⁺)

### Example 63: N-(3-(3-(4-Fluorobenzyl)-4-oxo-3,4-dihydrophthalazin-1-yl)phenyl)ethanesulfonamide

1-(Bromomethyl)-4-fluorobenzene (135 µL, 1.08 mmol) was used instead of benzyl bromide and reacted in the same manner as in Example 61, to obtain 57 mg of the title compound.
LC-MS (ESI, m/z) = 438.1 (M+H⁺)

### Example 64: N-(3-(3-([1,1'-Biphenyl]-4-yl)-4-oxo-3,4-dihydrophthalazin-1-yl)phenyl)ethane sulfonamide

[1,1'-Biphenyl]-4-ylboronic acid (513.20 mg, 2.59 mmol) was used instead of 3-aminophenylboronic acid and reacted in the same manner as in Example 1, to obtain 130 mg of the title compound.
LC-MS (ESI, m/z) = 482.2 (M+H⁺)

### Example 65: N-(3-(3-([1,1'-Biphenyl]-4-yl)-4-oxo-3,4-dihydrophthalazin-1-yl)phenyl)-N-methylethanesulfonamide

The compound (60 mg, 0.10 mmol) of Example 64 was used as a starting material and reacted in the same manner as in Example 5, to obtain 59 mg of the title compound.
LC-MS (ESI, m/z) = 496.0 (M+H⁺)

### Example 66: Methyl 3-(3-(4-fluorophenyl)-4-oxo-3,4-dihydrophthalazin-1-yl)benzoate

Compound I-2 (1.00 g, 3.64 mmol) and (3-(methoxycarbonyl)phenyl)boronic acid (851.76 mg, 4.73 mmol) were reacted in the same manner as in Step 1 of Example 1, to obtain 785 mg of the title compound.
LC-MS (ESI, m/z) = 374.0 (M+H⁺)

### Example 67: N-(3-(3-(4-Fluorophenyl)-4-oxo-3,4-dihydro phthalazin-1-yl)phenyl)propan-2-sulfonamide

Compound I-2 (100 mg, 0.36 mmol), 3-aminophenylboronic acid (59 mg, 0.43 mmol) and 2-propanesulfonyl chloride (48 µL, 0.43 mmol) were reacted in the same manner as in Example 1, to obtain 67 mg of the title compound.
LC-MS (ESI, m/z) = 438.1 (M+H⁺)

### Example 68: N-(3-(3-(4-Fluorophenyl)-4-oxo-3,4-dihydrophthalazin-1-yl)phenyl)propan-1-sulfonamide

Compound I-2 (100 mg, 0.36 mmol), 3-aminophenylboronic acid (59 mg, 0.43 mmol) and 1-propanesulfonyl chloride (48 µL, 0.43 mmol) were reacted in the same manner as in Example 1, to obtain 67 mg of the title compound.
LC-MS (ESI, m/z) = 438.1 (M+H⁺)

### Example 69: N-(3-(3-(4-Fluorophenyl)-4-oxo-3,4-dihydrophthalazin-1-yl)phenyl)-N-methylmethane sulfonamide

The compound (60 mg, 0.15 mmol) of Example 10 was used as a starting material and reacted in the same manner as in Example 5, to obtain 56 mg of the title compound.
LC-MS (ESI, m/z) = 424.1 (M+H⁺)

### Example 70: N-(3-(3-(4-Fluorophenyl)-4-oxo-3,4-dihydrophthalazin-1-yl)phenyl)-N-methylethane sulfonamide

The compound (60 mg, 0.14 mmol) of Example 11 was used as a starting material and reacted in the same manner as in Example 5, to obtain 51 mg of the title compound.
LC-MS (ESI, m/z) = 438.0 (M+H⁺)

### Example 71: N-(3-(3-(4-Fluorophenyl)-4-oxo-3,4-dihydro phthalazin-1-yl)phenyl)cyclopropane sulfonamide

Compound I-2 (100 mg, 0.36 mmol), 3-aminophenylboronic acid (59 mg, 0.43 mmol) and cyclopropanesulfonyl chloride (43 µL, 0.43 mmol) were reacted in the same manner as in Example 1, to obtain 121 mg of the title compound.
LC-MS (ESI, m/z) = 436.1 (M+H⁺)

### Example 72: N-(3-(3-(4-Fluorophenyl)-4-oxo-3,4-dihydrophthalazin-1-yl)phenyl)-N-methylpropan-1-sulfonamide

The compound (30 mg, 0.07 mmol) of Example 68 was used as a starting material and reacted in the same manner as in Example 5, to obtain 26 mg of the title compound.
LC-MS (ESI, m/z) = 452.2 (M+H⁺)

### Example 73: N-(3-(3-(4-Fluorophenyl)-4-oxo-3,4-dihydrophthalazin-1-yl)phenyl)-N-methylpropan-2-sulfonamide

The compound (30 mg, 0.07 mmol) of Example 67 was used as a starting material and reacted in the same manner as in Example 5, to obtain 25 mg of the title compound.
LC-MS (ESI, m/z) = 452.1 (M+H⁺)

### Example 74: N-(3-(3-(4-Fluorophenyl)-4-oxo-3,4-dihydrophthalazin-1-yl)phenyl)-N-methylcyclopropane sulfonamide

The compound (30 mg, 0.07 mmol) of Example 71 was used as a starting material and reacted in the same manner as in Example 5, to obtain 20 mg of the title compound.
LC-MS (ESI, m/z) = 450.1 (M+H⁺)

### Example 75: N-(3-(3-(4-Fluorophenyl)-4-oxo-3,4-dihydro phthalazin-1-yl)phenyl)-N-methyl-dimethyl sulfamoylamide

The compound (30 mg, 0.07 mmol) of Example 12 was used as a starting material and reacted in the same manner as in Example 5, to obtain 23 mg of the title compound.
LC-MS (ESI, m/z) = 453.1 (M+H⁺)

### Example 76: Methyl (N-(3-(3-(4-fluorophenyl)-4-oxo-3,4-dihydrophthalazin-1-yl)phenyl)-N-methyl sulfamoyl) (methyl) carbamate

The compound (150 mg, 0.32 mmol) of Example 20 was used as a starting material and reacted in the same manner as in Example 5, to obtain 117 mg of the title compound.
LC-MS (ESI, m/z) = 497.0 (M+H⁺)

### Example 77: 1,1,1-Trifluoro-N-(3-(3-(4-fluorophenyl)-4-oxo-3,4-dihydrophthalazin-1-yl)phenyl)methanesulfonamide

Compound I-2 (200 mg, 0.72 mmol), 3-aminophenylboronic acid (118 mg, 0.86 mmol) and trifluoromethanesulfonic anhydride (144 µL, 0.86 mmol) were reacted in the same manner as in Example 1, to obtain 56 mg of the title compound.
LC-MS (ESI, m/z) = 463.9 (M+H⁺)

### Example 78: 1,1,1-Trifluoro-N-(3-(3-(4-fluorophenyl)-4-oxo-3,4-dihydrophthalazin-1-yl)phenyl)-N-methylmethane sulfonamide

The compound (30 mg, 0.07 mmol) of Example 77 was used as a starting material and reacted in the same manner as in Example 5, to obtain 17 mg of the title compound.
LC-MS (ESI, m/z) = 477.9 (M+H⁺)

### Example 79: 3-(3-(4-Fluorophenyl)-4-oxo-3,4-dihydrophthalazin-1-yl)-N-((tetrahydro-2H-pyran-2-yl)oxy)benzamide

Step 1: The compound (750 mg, 2.00 mmol) of Example 66 was dissolved in THF/MeOH/H₂O (100 mL, 3:1:1 ratio) and, after adding LiOH-H₂O (420 mg, 10.02 mmol), resulting mixture was stirred at room temperature for three hours. The reaction mixture was concentrated under reduced pressure, diluted with EtOAc, washed with a 1N aqueous HCl solution, and dehydrated over anhydrous Na₂SO₄, to obtain 667 mg of 3-(3-(4-fluorophenyl)-4-oxo-3,4-dihydrophthalazin-1-yl)benzoic acid.
LC-MS (ESI, m/z) = 361.0 (M+H⁺)

Step 2: The compound (150 mg, 0.42 mmol) obtained in Step 1 was dissolved in DCM (4 mL) and, after adding diisopropylethylamine (DIPEA; 0.7 mL, 4.16 mmol), 1-ethyl-3-(3-dimethylaminopropyl) carbodiimide (EDCI; 159 mg, 0.83 mmol) and hydroxybenzotriazole (HOBt; 84 mg, 0.62 mmol), resulting mixture was stirred at room temperature for 12 hours. The reaction mixture was concentrated under reduced pressure, diluted in EtOAc, and then washed with purified water. Organic layer was dehydrated over anhydrous Na₂SO₄, concentrated under reduced pressure, and then reaction product was separated by column chromatography, to obtain 144 mg of the title compound in white solid.
LC-MS (ESI, m/z) = 460.0 (M+H⁺)

### Example 80: N-(3-(3-(4-Fluorophenyl)-4-oxo-3,4-dihydrophthalazin-1-yl)phenyl)-N-methyl-methyl sulfamoylamide

The compound (63 mg, 0.13 mmol) of Example 76 was dissolved in THF/MeOH/H₂O (5 mL, 3:1:1 ratio) and, after adding LiOH-H₂O (16 mg, 0.38 mmol), resulting mixture was stirred at room temperature for three hours. The resulting solid compound was filtered and then dried, to obtain 40 mg of the title compound.
LC-MS (ESI, m/z) = 439.0 (M+H⁺)

### Example 81: 4-(3-(1H-Tetrazol-5-yl)phenyl)-2-(4-fluorophenyl)phthalazin-1(2H)-one

Step 1: Compound I-2 (200 mg, 0.72 mmol) and 3-cyanophenylboronic acid (127 mg, 0.86 mmol) were reacted in the same manner as in Step 1 of Example 1, to obtain 214 mg of 3-(3-(4-fluorophenyl)-4-oxo-3,4-dihydrophthalazin-1-yl)benzonitrile.
LC-MS (ESI, m/z) = 342.1 (M+H⁺)

Step 2: The compound (100 mg, 0.29 mmol) obtained in Step 1, NaN₃ (23 mg, 0.35 mmol) and NH₄Cl (19 mg, 0.35 mmol) were added to DMF (5 mL) and stirred at 120 °C for 12 hours. A 0.5 N aqueous HCl solution was added to adjust pH to 5-6, and the resulting crystals were filtered, to obtain 24 mg of the title compound.
LC-MS (ESI, m/z) = 385.0 (M+H⁺)

### Example 82: N-(5-(3-(4-Fluorophenyl)-4-oxo-3,4-dihydrophthalazin-1-yl)pyridin-3-yl)ethanesulfonamide

Compound I-2 (100 mg, 0.36 mmol), (5-aminopyridin-3-yl)boronic acid (59 mg, 0.43 mmol) and ethanesulfonyl chloride (40 µL, 0.43 mmol) were reacted in the same manner as in Example 1, to obtain 42 mg of the title compound.
LC-MS (ESI, m/z) = 425.0 (M+H⁺)

### Example 83: 3-(3-(4-Fluorophenyl)-4-oxo-3,4-dihydro phthalazin-1-yl)-N-hydroxybenzamide

MeOH (20 mL) and trifluoroacetic acid (TFA; 10 mL) were added to the compound (100 mg, 0.22 mmol) of Example 79 and then stirred at room temperature for three hours. The reaction mixture was concentrated under reduced pressure, dissolved in EtOAc, and then washed with a 1N aqueous HCl solution, and organic layer was extracted. After concentration under reduced pressure to remove the organic solvent, a small amount of diethyl ether was added and the resulting solid compound was filtered, to obtain 37 mg of the title compound.
LC-MS (ESI, m/z) = 376.0 (M+H⁺)

### Example 84: (Z)-1-(3-(3-([1,1'-Biphenyl]-4-yl)-4-oxo-3,4-dihydrophthalazin-1-yl)phenyl)-N-methylmethanimine oxide

[1,1'-Biphenyl]-4-ylboronic acid (100 mg, 0.50 mmol) was used instead of 3-formylphenylboronic acid and reacted in the same manner as in Example 13, to obtain 32 mg of the title compound.
LC-MS (ESI, m/z) = 432.1 (M+H⁺)

### Example 85: (Z)-1-(3-(3-([1,1'-Biphenyl]-4-yl)-4-oxo-3,4-dihydrophthalazin-1-yl)phenyl)-N-isopropylmethanimine oxide

N-isopropylhydroxylamine hydrochloride and [1,1'-biphenyl]-4-ylboronic acid (100 mg, 0.50 mmol) were used instead of N-ethylhydroxylamine hydrochloride and 3-formylphenylboronic acid, respectively, and reacted in the same manner as in Example 16, to obtain 24 mg of the title compound.
LC-MS (ESI, m/z) = 460.0 (M+H⁺)

### Example 86: 3-(3-(4-Fluorophenyl)-4-oxo-3,4-dihydrophthalazin-1-yl)-N-methylbenzenesulfonamide

Compound I-2 (80 mg, 0.29 mmol), methyl 3-boronobenzenesulfonamide (81 mg, 0.38 mmol), Pd[Ph₃P]₄ (33 mg, 0.03 mmol) and Na₂CO₃ (124 mg, 1.17 mmol) were added to 1,4-dioxane (10 mL) and then stirred at 110 °C for two hours. The reaction product was diluted with EtOAc and washed with purified water, and then organic layer was extracted. The separated organic layer was dehydrated over anhydrous Na₂SO₄, concentrated under reduced pressure, and then reaction product was separated by column chromatography, to obtain 95 mg of the title compound.
LC-MS (ESI, m/z) = 410.1 (M+H⁺)

### Example 87: N-Ethyl-3-(3-(4-fluorophenyl)-4-oxo-3,4-dihydrophthalazin-1-yl)benzenesulfonamide

Ethyl 3-boronobenzenesulfonamide (81 mg, 0.38 mmol) was reacted in the same manner as in Example 86, to obtain 84 mg of the title compound.
LC-MS (ESI, m/z) = 424.1 (M+H⁺)

### Example 88: 3-(3-(4-Fluorophenyl)-4-oxo-3,4-dihydrophthalazin-1-yl)-N,N-dimethylbenzene sulfonamide

N,N-Dimethyl 3-boronobenzenesulfonamide (81 mg, 0.38 mmol) was used instead of methyl 3-boronobenzenesulfonamide and reacted in the same manner as in Example 86, to obtain 47 mg of the title compound.
LC-MS (ESI, m/z) = 424.0 (M+H⁺)

### Example 89: 2-(2,4-Difluorophenyl)-4-(3-(methyl sulfonyl)phenyl)phthalazin-1(2H)-one

Compound I-9 (77 mg, 0.19 mmol) and (3-(methylsulfonyl)phenyl)boronic acid (49 mg, 0.25 mmol) were reacted in the same manner as in Example 86, to obtain 34 mg of the title compound.
LC-MS (ESI, m/z) = 413.2 (M+H⁺)

### Example 90: 2-(2,4-Difluorophenyl)-4-(3-(ethylsulfonyl)phenyl)phthalazin-1(2H)-one

Compound I-9 (77 mg, 0.19 mmol) and (3-(ethylsulfonyl)phenyl)boronic acid (52 mg, 0.25 mmol) were reacted in the same manner as in Example 86, to obtain 30 mg of the title compound.
LC-MS (ESI, m/z) = 427.1 (M+H⁺)

### Example 91: 3-(3-(2,4-Difluorophenyl)-4-oxo-3,4-dihydrophthalazin-1-yl)-N-methylbenzene sulfonamide

Compound I-9 (77 mg, 0.19 mmol) and (3-(N-methylsulfamoyl)phenyl)boronic acid (52 mg, 0.25 mmol) were reacted in the same manner as in Example 86, to obtain 37 mg of the title compound.
LC-MS (ESI, m/z) = 427.9 (M+H⁺)

### Example 92: 3-(3-(2,4-Difluorophenyl)-4-oxo-3,4-dihydrophthalazin-1-yl)-N-ethylbenzene sulfonamide

Compound I-9 (77 mg, 0.19 mmol) and (3-(N-ethylsulfamoyl)phenyl)boronic acid (56 mg, 0.25 mmol) were reacted in the same manner as in Example 86, to obtain 44 mg of the title compound.
LC-MS (ESI, m/z) = 442.1 (M+H⁺)

### Example 93: 3-(3-(2,4-Difluorophenyl)-4-oxo-3,4-dihydrophthalazin-1-yl)-N,N-dimethylbenzene sulfonamide

Compound I-9 (77 mg, 0.19 mmol) and (3-(N,N-dimethylsulfamoyl)phenyl)boronic acid (56 mg, 0.25 mmol) were reacted in the same manner as in Example 86, to obtain 44 mg of the title compound.
LC-MS (ESI, m/z) = 442.0 (M+H⁺)

### Example 94: (Z)-1-(3-(3-(2-Fluorophenyl)-4-oxo-3,4-dihydrophthalazin-1-yl)phenyl)-N-methyl methanimine oxide

Compound I-5 (100 mg, 0.36 mmol), 3-formylphenylboronic acid (63 mg, 0.42 mmol) and N-methylhydroxylamine hydrochloride (145 mg, 1.75 mmol) were reacted in the same manner as in Example 13, to obtain 51 mg of the title compound.
LC-MS (ESI, m/z) = 374.1 (M+H⁺)

### Example 95: (Z)-1-(3-(3-(2-Fluorophenyl)-4-oxo-3,4-dihydrophthalazin-1-yl)phenyl)-N-isopropyl methanimine oxide

Compound I-5 (100 mg, 0.36 mmol), 3-formylphenylboronic acid (63 mg, 0.42 mmol) and N-isopropylhydroxylamine hydrochloride (194 mg, 1.75 mmol) were reacted in the same manner as in Example 13, to obtain 32 mg of the title compound.
LC-MS (ESI, m/z) = 402.0 (M+H⁺)

### Example 96: (Z)-N-tert-Butyl-1-(3-(3-(2-fluorophenyl)-4-oxo-3,4-dihydrophthalazin-1-yl)phenyl)methanimine oxide

Compound I-5 (100 mg, 0.36 mmol), 3-formylphenylboronic acid (63 mg, 0.42 mmol) and N-tert-butylhydroxylamine hydrochloride (218 mg, 1.75 mmol) were reacted in the same manner as in Example 13, to obtain 30 mg of the title compound.
LC-MS (ESI, m/z) = 416.1 (M+H⁺)

### Example 97: 2-(2-Fluorophenyl)-4-(3-(methylsulfonyl)phenyl)phthalazin-1(2H)-one

Compound I-5 (52 mg, 0.19 mmol) and (3-(methylsulfonyl)phenyl)boronic acid (49 mg, 0.25 mmol) were reacted in the same manner as in Example 86, to obtain 39 mg of the title compound.
LC-MS (ESI, m/z) = 395.0 (M+H⁺)

### Example 98: 4-(3-(Ethylsulfonyl)phenyl)-2-(2-fluoro phenyl)phthalazin-1(2H)-one

Compound I-5 (52 mg, 0.19 mmol) and (3-(ethylsulfonyl)phenyl)boronic acid (53 mg, 0.25 mmol) were reacted in the same manner as in Example 86, to obtain 41 mg of the title compound.
LC-MS (ESI, m/z) = 408.8 (M+H⁺)

### Example 99: 3-(3-(2-Fluorophenyl)-4-oxo-3,4-dihydrophthalazin-1-yl)-N-methylbenzenesulfonamide

Compound I-5 (52 mg, 0.19 mmol) and (3-(N-methylsulfamoyl)phenyl)boronic acid (52 mg, 0.25 mmol) were reacted in the same manner as in Example 86, to obtain 28 mg of the title compound.
LC-MS (ESI, m/z) = 410.0 (M+H⁺)

### Example 100: N-Ethyl-3-(3-(2-fluorophenyl)-4-oxo-3,4-dihydrophthalazin-1-yl)benzenesulfonamide

Compound I-5 (52 mg, 0.19 mmol) and (3-(N-ethylsulfamoyl)phenyl)boronic acid (56 mg, 0.25 mmol) were reacted in the same manner as in Example 86, to obtain 36 mg of the title compound.
LC-MS (ESI, m/z) = 423.8 (M+H⁺)

### Example 101: 3-(3-(2-Fluorophenyl)-4-oxo-3,4-dihydrophthalazin-1-yl)-N,N-dimethylbenzene sulfonamide

Compound I-5 (52 mg, 0.19 mmol) and (3-(N,N-dimethylsulfamoyl)phenyl)boronic acid (56 mg, 0.25 mmol) were reacted in the same manner as in Example 86, to obtain 30 mg of the title compound.
LC-MS (ESI, m/z) = 424.1 (M+H⁺)

### Example 102: (Z)-1-(3-(3-(2,6-Difluorophenyl)-4-oxo-3,4-dihydrophthalazin-1-yl)phenyl)-N-methylmethanimine oxide

Compound I-10 (140 mg, 0.34 mmol), 3-formylphenylboronic acid (64 mg, 0.43 mmol) and N-methylhydroxylamine hydrochloride (150 mg, 1.80 mmol) were reacted in the same manner as in Example 13, to obtain 45 mg of the title compound.
LC-MS (ESI, m/z) = 392.1 (M+H⁺)

### Example 103: (Z)-1-(3-(3-(2,6-Difluorophenyl)-4-oxo-3,4-dihydrophthalazin-1-yl)phenyl)-N-isopropylmethanimine oxide

Compound I-10 (140 mg, 0.34 mmol), 3-formylphenylboronic acid (64 mg, 0.43 mmol) and N-isopropylhydroxylamine hydrochloride (200 mg, 1.80 mmol) were reacted in the same manner as in Example 13, to obtain 39 mg of the title compound.
LC-MS (ESI, m/z) = 420.1 (M+H⁺)

### Example 104: (Z)-N-tert-Butyl-1-(3-(3-(2,6-difluoro phenyl)-4-oxo-3,4-dihydrophthalazin-1-yl)phenyl)methanimine oxide

Compound I-10 (140 mg, 0.34 mmol), 3-formylphenylboronic acid (64 mg, 0.43 mmol) and N-tert-butylhydroxylamine hydrochloride (225 mg, 1.80 mmol) were reacted in the same manner as in Example 13, to obtain 36 mg of the title compound.
LC-MS (ESI, m/z) = 434.0 (M+H⁺)

### Example 105: 2-(2,6-Difluorophenyl)-4-(3-(methyl sulfonyl)phenyl)phthalazin-1(2H)-one

Compound I-10 (70 mg, 0.17 mmol) and (3-(methylsulfonyl)phenyl)boronic acid (49 mg, 0.25 mmol) were reacted in the same manner as in Example 86, to obtain 30 mg of the title compound.
LC-MS (ESI, m/z) = 413.0 (M+H⁺)

### Example 106: 2-(2,6-Difluorophenyl)-4-(3-(ethyl sulfonyl)phenyl)phthalazin-1(2H)-one

Compound I-10 (70 mg, 0.17 mmol) and (3-(ethylsulfonyl)phenyl)boronic acid (53 mg, 0.25 mmol) were reacted in the same manner as in Example 86, to obtain 48 mg of the title compound.
LC-MS (ESI, m/z) = 427.0 (M+H⁺)

### Example 107: 3-(3-(2,6-Difluorophenyl)-4-oxo-3,4-dihydrophthalazin-1-yl)-N-methylbenzenesulfonamide

Compound I-10 (70 mg, 0.17 mmol) and (3-(N-methylsulfamoyl)phenyl)boronic acid (53 mg, 0.25 mmol) were reacted in the same manner as in Example 86, to obtain 39 mg of the title compound.
LC-MS (ESI, m/z) = 428.2 (M+H⁺)

### Example 108: 3-(3-(2,6-Difluorophenyl)-4-oxo-3,4-dihydrophthalazin-1-yl)-N-ethylbenzenesulfonamide

Compound I-10 (70 mg, 0.17 mmol) and (3-(N-ethylsulfamoyl)phenyl)boronic acid (57 mg, 0.25 mmol) were reacted in the same manner as in Example 86, to obtain 46 mg of the title compound.
LC-MS (ESI, m/z) = 442.0 (M+H⁺)

### Example 109: 3-(3-(2,6-Difluorophenyl)-4-oxo-3,4-dihydrophthalazin-1-yl)-N,N-dimethylbenzenesulfonamide

Compound I-10 (70 mg, 0.17 mmol) and (3-(N,N-dimethylsulfamoyl)phenyl)boronic acid (57 mg, 0.25 mmol) were reacted in the same manner as in Example 86, to obtain 30 mg of the title compound.
LC-MS (ESI, m/z) = 442.1 (M+H⁺)

### Example 110: 6-Fluoro-4-(3-(methylsulfonyl)phenyl)-2-phenylphthalazin-1(2H)-one

Compound I-11 (70 mg, 0.26 mmol) and (3-(methylsulfonyl)phenyl)boronic acid (66 mg, 0.33 mmol) were reacted in the same manner as in Example 86, to obtain 91 mg of the title compound.
LC-MS (ESI, m/z) = 395.0 (M+H⁺)

### Example 111: 3-(7-Fluoro-4-oxo-3-phenyl-3,4-dihydrophthalazin-1-yl)-N-methylbenzenesulfonamide

Compound I-11 (70 mg, 0.26 mmol) and (3-(N-methylsulfamoyl)phenyl)boronic acid (71 mg, 0.33 mmol) were reacted in the same manner as in Example 86, to obtain 44 mg of the title compound.
LC-MS (ESI, m/z) = 410.0 (M+H⁺)

### Example 112: 7-Fluoro-4-(3-(methylsulfonyl)phenyl)-2-phenylphthalazin-1(2H)-one

Compound I-12 (70 mg, 0.26 mmol) and (3-(methylsulfonyl)phenyl)boronic acid (66 mg, 0.33 mmol) were reacted in the same manner as in Example 86, to obtain 84 mg of the title compound.
LC-MS (ESI, m/z) = 395.0 (M+H⁺)

### Example 113: 3-(6-Fluoro-4-oxo-3-phenyl-3,4-dihydro phthalazin-1-yl)-N-methylbenzenesulfonamide

Compound I-12 (70 mg, 0.26 mmol) and (3-(N-methylsulfamoyl)phenyl)boronic acid (71 mg, 0.33 mmol) were reacted in the same manner as in Example 86, to obtain 51 mg of the title compound.
LC-MS (ESI, m/z) = 410.0 (M+H⁺)

### Example 114: 3-(3-(4-Fluorophenyl)-4-oxo-3,4-dihydrophthalazin-1-yl)pyridine 1-oxide

Step 1: Compound I-2 (173 mg, 0.63 mmol) and 3-pyridinylboronic acid (141 mg, 0.94 mmol) were reacted in the same manner as in Step 1 of Example 1, to obtain 94 mg of 2-(4-fluorophenyl)-4-(pyridin-3-yl)phthalazin-1(2H)-one.
LC-MS (ESI, m/z) = 318.0 (M+H⁺)

Step 2: The compound (74 mg, 0.26 mmol) obtained in Step 1 was dissolved in DCM (40 mL) and, after adding metachloroperoxybenzoic acid (mCPBA; 136 mg, 0.79 mmol), resulting mixture was stirred at room temperature for four hours. The reaction solution was diluted with EtOAc and washed with purified water, and organic layer was dehydrated over anhydrous Na₂SO₄ and then reaction product was separated by column chromatography, to obtain 46 mg of the title compound.
LC-MS (ESI, m/z) = 334.0 (M+H⁺)

### Example 115: 2-(2,4-Difluorophenyl)-4-(pyridin-3-yl)phthalazin-1(2H)-one

Compound I-9 (180 mg, 0.44 mmol) and 3-pyridinylboronic acid (99 mg, 0.66 mmol) were reacted in the same manner as in Step 1 of Example 1, to obtain 85 mg of the title compound.
LC-MS (ESI, m/z) = 336.0 (M+H⁺)

### Example 116: 2-(2,6-Difluorophenyl)-4-(pyridin-3-yl)phthalazin-1(2H)-one

Compound I-10 (180 mg, 0.44 mmol) and 3-pyridinylboronic acid (99 mg, 0.66 mmol) were reacted in the same manner as in Step 1 of Example 1, to obtain 96 mg of the title compound.
LC-MS (ESI, m/z) = 336.2 (M+H⁺)

### Example 117: 3-(3-(2,4-Difluorophenyl)-4-oxo-3,4-dihydrophthalazin-1-yl)pyridine 1-oxide

The compound (70 mg, 0.21 mmol) of Example 115 was reacted in the same manner as in Step 2 of Example 114, to obtain 60 mg of the title compound.
LC-MS (ESI, m/z) = 352.0 (M+H⁺)

### Example 118: 3-(3-(2,6-Difluorophenyl)-4-oxo-3,4-dihydrophthalazin-1-yl)pyridine 1-oxide

The compound (70 mg, 0.21 mmol) of Example 116 was reacted in the same manner as in Step 2 of Example 114, to obtain 54 mg of the title compound.
LC-MS (ESI, m/z) = 352.0 (M+H⁺)

### Example 119: 2-(2,4-Difluorophenyl)-6-fluoro-4-(3-(methylsulfonyl)phenyl)phthalazin-1(2H)-one

Compound I-13 (70 mg, 0.16 mmol) and (3-(methylsulfonyl)phenyl)boronic acid (42 mg, 0.21 mmol) were reacted in the same manner as in Example 86, to obtain 36 mg of the title compound.
LC-MS (ESI, m/z) = 431.0 (M+H⁺)

### Example 120: 2-(2,4-Difluorophenyl)-7-fluoro-4-(3-(methylsulfonyl)phenyl)phthalazin-1(2H)-one

Compound I-14 (70 mg, 0.16 mmol) and (3-(methylsulfonyl)phenyl)boronic acid (42 mg, 0.21 mmol) were reacted in the same manner as in Example 86, to obtain 30 mg of the title compound.
LC-MS (ESI, m/z) = 431.0 (M+H⁺)

### Example 121: 3-(3-(2,4-Difluorophenyl)-7-fluoro-4-oxo-3,4-dihydrophthalazin-1-yl)-N-methylbenzenesulfonamide

Compound I-13 (70 mg, 0.16 mmol) and (3-(N-methylsulfamoyl)phenyl)boronic acid (45 mg, 0.21 mmol) were reacted in the same manner as in Example 86, to obtain 9 mg of the title compound.
LC-MS (ESI, m/z) = 446.0 (M+H⁺)

### Example 122: 3-(3-(2,4-Difluorophenyl)-6-fluoro-4-oxo-3,4-dihydrophthalazin-1-yl)-N-methylbenzenesulfonamide

Compound I-14 (70 mg, 0.16 mmol) and (3-(N-methylsulfamoyl)phenyl)boronic acid (45 mg, 0.21 mmol) were reacted in the same manner as in Example 86, to obtain 39 mg of the title compound.
LC-MS (ESI, m/z) = 446.1 (M+H⁺)

### Example 123: 2-(2,4-Difluorophenyl)-4-(3-(ethylsulfonyl)phenyl)-6-fluorophthalazin-1(2H)-one

Compound I-13 (70 mg, 0.16 mmol) and (3-(ethylsulfonyl)phenyl)boronic acid (45 mg, 0.21 mmol) were reacted in the same manner as in Example 86, to obtain 26 mg of the title compound.
LC-MS (ESI, m/z) = 445.0 (M+H⁺)

### Example 124: 3-(3-(2,4-Difluorophenyl)-7-fluoro-4-oxo-3,4-dihydrophthalazin-1-yl)-N-ethylbenzenesulfonamide

Compound I-13 (70 mg, 0.16 mmol) and (3-(N-ethylsulfonyl)phenyl)boronic acid (49 mg, 0.21 mmol) were reacted in the same manner as in Example 86, to obtain 18 mg of the title compound.
LC-MS (ESI, m/z) = 460.1 (M+H⁺)

### Example 125: 3-(3-(2,4-Difluorophenyl)-7-fluoro-4-oxo-3,4-dihydrophthalazin-1-yl)-N,N-dimethylbenzenesulfonamide

Compound I-13 (70 mg, 0.16 mmol) and (3-(N,N-dimethylsulfonyl)phenyl)boronic acid (49 mg, 0.21 mmol) were reacted in the same manner as in Example 86, to obtain 28 mg of the title compound.
LC-MS (ESI, m/z) = 460.0 (M+H⁺)

### Example 126: 3-(3-(2,4-Difluorophenyl)-4-oxo-3,4-dihydrophthalazin-1-yl)benzenesulfonamide

Compound I-9 (70 mg, 0.17 mmol) and (3-sulfamoylphenyl)boronic acid (52 mg, 0.26 mmol) were reacted in the same manner as in Example 86, to obtain 44 mg of the title compound.
LC-MS (ESI, m/z) = 414.1 (M+H⁺)

### Example 127: 3-(3-(2,4-Difluorophenyl)-4-oxo-3,4-dihydrophthalazin-1-yl)-N-(2-hydroxyethyl)benzene sulfonamide

Compound I-9 (70 mg, 0.17 mmol) and (3-(N-(2-hydroxyethyl)sulfamoyl)phenyl)boronic acid (63 mg, 0.26 mmol) were reacted in the same manner as in Example 86, to obtain 51 mg of the title compound.
LC-MS (ESI, m/z) = 458.0 (M+H⁺)

### Example 128: N-(3-(3-Cyclohexyl-4-oxo-3,4-dihydro phthalazin-1-yl)phenyl)ethanesulfonamide

Step 1: 4-Bromophthalazin-1(2H)-one (300 mg, 1.333 mmol) and K₂CO₃ (368 mg, 2.67 mmol) were diluted in DMF (4.4 mL) and, after adding bromocyclohexane (326 mg, 2.00 mmol), resulting mixture was stirred at 100 °C for 24 hours. The reaction solution was allowed to cool to room temperature, diluted with ethyl acetate (EA), and then washed with purified water. Organic layer was dehydrated over Na₂SO₄, concentrated under reduced pressure, and then reaction product was separated by column chromatography, to obtain 108.7 mg of 4-bromo-2-cyclohexylphthalazin-1(2H)-one in white solid.
LC-MS (ESI, m/z) = 107.0 (M+H⁺)

Step 2: The compound (105 mg, 0.342 mmol) obtained in Step 1 and (3-aminophenyl)boronic acid (51.5 mg, 0.376 mmol) were reacted in the same manner as in Step 1 of Example 1, to obtain 60.1 mg of 4-(3-aminophenyl)-2-cyclohexylphthalazin-1(2H)-one in white solid.
LC-MS (ESI, m/z) = 320.1(M+H⁺)

Step 3: The compound (32 mg, 0.100 mmol) obtained in Step 2 was dissolved in pyridine, of which temperature was then lowered to 0 to 5 °C, and after adding ethanesulfonyl chloride (11.39 µL, 0.120 mmol) dropwise for five minutes, resulting mixture was stirred at room temperature for two hours. The reaction solution was diluted with EA and then washed four or five times with saturated aqueous CuSO₄ solution. Organic layer was dehydrated over Na₂SO₄, concentrated under reduced pressure, and then reaction product was separated by column chromatography, to obtain 31.3 mg of the title compound in white solid.
LC-MS (ESI, m/z) = 412.1(M+H⁺)

### Example 129: N-(3-(3-(1-Methylpiperidin-4-yl)-4-oxo-3,4-dihydrophthalazin-1-yl)phenyl)ethane sulfonamide

4-Bromo-1-methylpiperidine (356 mg, 2.00 mmol) was used instead of bromocyclohexane and reacted in the same manner as in Example 128, to obtain 34 mg of the title compound in white solid.
LC-MS (ESI, m/z) = 427.1(M+H⁺)

### Example 130: 3-(3-(4-Fluorophenyl)-4-oxo-3,4-dihydrophthalazin-1-yl)benzonitrile

Compound I-2 (147 mg, 0.461 mmol) and (3-cyanophenyl)boronic acid (74.5, mg, 0.507 mmol) were reacted in the same manner as in Step 1 of Example 1, to obtain 71 mg of the title compound in white solid.
LC-MS (ESI, m/z) = 342.0(M+H⁺)

### Example 131: (E)-3-(3-(4-Fluorophenyl)-4-oxo-3,4-dihydrophthalazin-1-yl)-N'-hydroxybenzimidamide

The compound (20 mg, 0.059 mmol) of Example 130, hydroxylamine hydrochloride (4.89 mg, 0.070 mmol) and NaHCO₃ (7.38 mg, 0.088 mmol) were dissolved in EtOH (585 µL) and stirred at 80 °C for three hours. After allowing the reaction solution to cool to room temperature, water was added, and precipitated solid was filtered and washed with distilled water. The filtered solid was crystallized and dried using DCM and MeOH, to obtain 6.4 mg of the title compound in white solid.
LC-MS (ESI, m/z) = 375.2 (M+H⁺)

### Example 132: N-(3-(3-Isopropyl-4-oxo-3,4-dihydro phthalazin-1-yl)phenyl)ethanesulfonamide

2-Iodopropane (340 mg, 2.00 mmol) was used instead of bromocyclohexane and reacted in the same manner as in Example 128, to obtain 45 mg of the title compound in white solid.
LC-MS (ESI, m/z) = 372.0 (M+H⁺)

### Example 133: N-(3-(3-(1-Methyl-1H-pyrazol-4-yl)-4-oxo-3,4-dihydrophthalazin-1-yl)phenyl)ethanesulfonamide

(1-Methyl-1H-pyrazol-4-yl)boronic acid (252 mg, 2.00 mmol) was used instead of bromocyclohexane and reacted in the same manner as in Example 128, to obtain 45 mg of the title compound in white solid.
LC-MS (ESI, m/z) = 410.1 (M+H⁺)

### Example 134: 4-(3-Aminopiperidin-1-yl)-2-(4-fluorophenyl)phthalazin-1(2H)-one·hydrochloride

Step 1: Compound I-2 (70 mg, 0.219 mmol), tert-butyl piperidin-3-ylcarbamate (52.7 mg, 0.263 mmol), XPhos (90 mg, 0.940 mmol) and NaOtBu (90 mg, 0.940 mmol) were diluted in toluene (1.04 mL) and, after adding Pd₂(dba)₃, reacted at 120 °C for one hour in a microwave reactor. The reaction solution was allowed to cool to room temperature, diluted with EA, and washed with purified water. Organic layer was dehydrated over Na₂SO₄, concentrated under reduced pressure, and then reaction product was separated by column chromatography, to obtain 47 mg of tert-butyl (1-(3-(4-fluorophenyl)-4-oxo-3,4-dihydrophthalazin-1-yl)piperidin-3-yl)carbamate in white solid.
LC-MS (ESI, m/z) = 439.1 (M+H⁺)

Step 2: The compound (50 mg, 0.114 mmol) obtained in Step 1 was dissolved in DCM (380 µL). After slowly adding 4M HCl in dioxane (855 µL) at 0 °C, reaction solution was stirred at room temperature for two hours. After concentrating, precipitated solid was filtered, washed with ether and then dried, to obtain 43 mg of the title compound in pale yellow solid.
LC-MS (ESI, m/z) = 339.1 (M+H⁺)

### Example 135: N-(1-(3-(4-Fluorophenyl)-4-oxo-3,4-dihydrophthalazin-1-yl)piperidin-3-yl)ethanesulfonamide

The compound (20 mg, 0.053 mmol) of Example 134 was reacted in the same manner as in Step 3 of Example 128, to obtain 5.5 mg of the title compound in white solid.
LC-MS (ESI, m/z) = 431.1 (M+H⁺)

### Example 136: N-(3-(3-(Oxetan-3-yl)-4-oxo-3,4-dihydrophthalazin-1-yl)phenyl)ethanesulfonamide

3-Tosyl oxetane (71 mg, 0.334 mmol) was used instead of bromocyclohexane and reacted in the same manner as in Example 128, to obtain 57 mg of the title compound in pale yellow solid.
LC-MS (ESI, m/z) = 386.1 (M+H⁺)

### Example 137: N-(1-(3-(4-Fluorophenyl)-4-oxo-3,4-dihydrophthalazin-1-yl)pyrrolidin-3-yl)ethanesulfonamide

tert-Butyl pyrrolidin-3-ylcarbamate (140 mg, 0.752 mmol) and Compound I-2 (200 mg, 0.627 mmol) were reacted in the same manner as in Example 134 and then Step 3 of Example 128, to obtain 55 mg of pale yellow title compound.
LC-MS (ESI, m/z) = 417.0 (M+H⁺)

### Example 138: (S)-N-(1-(3-(4-Fluorophenyl)-4-oxo-3,4-dihydrophthalazin-1-yl)piperidin-3-yl)ethanesulfonamide

*tert*-Butyl (S)-(1-(3-(4-fluorophenyl)-4-oxo-3,4-dihydrophthalazin-1-yl)carbamate (102 mg, 0.233 mmol) and Compound I-2 (150 mg, 0.470 mmol) were reacted in the same manner as in Example 137, to obtain 25 mg of the title compound in white solid.
LC-MS (ESI, m/z) = 431.1 (M+H⁺)

### Example 139: (R)-N-(1-(3-(4-Fluorophenyl)-4-oxo-3,4-dihydrophthalazin-1-yl)piperidin-3-yl)ethanesulfonamide

tert-Butyl (R)-(1-(3-(4-fluorophenyl)-4-oxo-3,4-dihydrophthalazin-1-yl)carbamate (113 mg, 0.564 mmol) and Compound I-2 (150 mg, 0.470 mmol) were reacted in the same manner as in Example 137, to obtain 14 mg of the title compound in white solid.
LC-MS (ESI, m/z) = 431.1 (M+H⁺)

### Example 140: N-(3-(3-(4,4-Difluorocyclohexyl)-4-oxo-3,4-dihydrophthalazin-1-yl)phenyl)ethanesulfonamide

Step 1: 4-Bromophthalazin-1(2H)-one (50 mg, 0.222 mmol) and 4,4-difluorocyclohexan-1-ol (36.6 mg, 0.267 mmol) were dissolved in THF (741 µL) and, after adding PPh₃ (87 mg, 0.333 mmol) and diisopropyl azodicarboxylate (DIAD; 67.4 mg, 0.333 mmol) at 0 °C, resultant was stirred at room temperature for one hour. The reaction solution was diluted with EA and washed with purified water. Organic layer was dehydrated over Na₂SO₄, concentrated under reduced pressure, and then reaction product was separated by column chromatography, to obtain 49.5 mg of 4-bromo-2-(4,4-difluorocyclohexyl)phthalazin-1(2H)-one in white solid.
LC-MS (ESI, m/z) = 343.0(M+H⁺)

Step 2: The compound (150 mg, 0.437 mmol) obtained in Step 1 was reacted in the same manner as in Step 2 and Step 3 of Example 128, to obtain 18.6 mg of the title compound in pale ocher solid.
LC-MS (ESI, m/z) = 448.1 (M+H⁺)

### Example 141: N-(1-(3-(4-Fluorophenyl)-4-oxo-3,4-dihydrophthalazin-1-yl)azepan-3-yl)ethanesulfonamide

tert-Butyl azepan-3-ylcarbamate (121 mg, 0.564 mmol) and Compound I-2 (150 mg, 0.470 mmol) were reacted in the same manner as in Example 137, to obtain 15.6 mg of the title compound in white solid.
LC-MS (ESI, m/z) = 445.1 (M+H⁺)

### Example 142: 3-(3-Cyclohexyl-4-oxo-3,4-dihydro phthalazin-1-yl)benzenesulfonamide

The compound (156 mg, 0.508 mmol) obtained in Step 1 of Example 128 and (3-sulfamoylphenyl)boronic acid (122 mg, 0.610 mmol) were reacted in the same manner as in Step 2 of Example 128, to obtain 20 mg of the title compound in white solid.
LC-MS (ESI, m/z) = 384.0 (M+H⁺)

### Example 143: 4-(3-Aminoazepan-1-yl)-2-cyclohexyl phthalazin-1(2H)-one·hydrochloride

4-Bromo-2-cyclohexylphthalazin-1(2H)-one (132 mg, 0.430 mmol) and tert-butyl azepan-3-ylcarbamate (111 mg, 0.516 mmol) were reacted in the same manner as in Example 134, to obtain 55.8 mg of the title compound inn ivory solid.
LC-MS (ESI, m/z) = 341.0 (M+H⁺)

### Example 144: N-(1-(3-Cyclohexyl-4-oxo-3,4-dihydrophthalazin-1-yl)azepan-3-yl)ethanesulfonamide

The compound (30 mg, 0.080 mmol) of Example 143 was reacted in the same manner as in Step 3 of Example 128, to obtain 18 mg of the title compound in white solid.
LC-MS (ESI, m/z) = 433.2 (M+H⁺)

### Example 145: N-(3-(3-(4-Methylcyclohexyl)-4-oxo-3,4-dihydrophthalazin-1-yl)phenyl)ethanesulfonamide

1-Bromo-4-methylcyclohexane (590 mg, 3.33 mmol) and 4-bromophthalazin-1(2H)-one (500 mg, 2.22 mmol) were reacted in the same manner as in Example 128, to obtain 15.8 mg of the title compound in pale yellow solid.
LC-MS (ESI, m/z) = 426.0 (M+H⁺)

### Example 146: (S)-4-(3-Aminopiperidin-1-yl)-2-cyclohexylphthalazin-1(2H)-one·hydrochloride

4-Bromo-2-cyclohexylphthalazin-1(2H)-one (140 mg, 0.456 mmol) and tert-butyl (S)-piperidin-3-ylcarbamate (110 mg, 0.547 mmol) were reacted in the same manner as in Example 134, to obtain 87 mg of the title compound in white solid.
LC-MS (ESI, m/z) = 327.2 (M+H⁺)

### Example 147: (S)-N-(1-(3-Cyclohexyl-4-oxo-3,4-dihydrophthalazin-1-yl)piperidin-3-yl)ethanesulfonamide

The compound (30 mg, 0.083 mmol) of Example 146 was reacted in the same manner as in Step 3 of Example 128, to obtain 6 mg of the title compound in pale yellow compound.
LC-MS (ESI, m/z) = 419.1 (M+H⁺)

### Example 148: 4-(3-Aminoazepan-1-yl)-2-(4-fluorophenyl)phthalazin-1(2H)-one·hydrochloride

Compound I-2 (150 mg, 0.470 mmol) and tert-butyl azepan-3-ylcarbamate (121 mg, 0.564 mmol) were reacted in the same manner as in Example 134, to obtain 9 mg of the title compound inn ochre solid.
LC-MS (ESI, m/z) = 353.1 (M+H⁺)

### Example 149: N-(3-(2-(4-Fluorophenyl)-1-oxo-1,2-dihydropyrido[3,4-d]pyridin-4-yl)phenyl)ethanesulfonamide

Step 1: 2-(4-Fluorophenyl)-1-oxo-1,2-dihydropyrido [3,4-d]pyridazin-4-yl trifluoromethanesulfonate (64 mg, 0.164 mmol), (3-aminophenyl)boronic acid (24.77 mg, 0.181 mmol), lithium chloride (20.91 mg, 0.493 mmol), Pd(PPh₃)₄ (19 mg, 0.016 mmol) and a 2M aqueous Na₂CO₃ solution (247 µL, 0.493 mmol) were diluted in 1.6 mL of toluene and stirred at a temperature of 100 to 110°C for three hours. The reaction solution was allowed to cool to room temperature, diluted with EA, and then washed with purified water. Organic layer was dehydrated over Na₂SO₄, concentrated under reduced pressure, and then reaction product was separated by column chromatography, to obtain 35 mg of 4-(3-aminophenyl)-2-(4-fluorophenyl)pyrido[3,4-d]pyradizin-1(2H)-one in pale yellow solid.
LC-MS (ESI, m/z) = 333.0 (M+H⁺)

Step 2: The compound (35 mg, 0.105 mmol) obtained in Step 1 was reacted in the same manner as in Step 3 of Example 128, to obtain 16 mg of the title compound in pale yellow solid.
LC-MS (ESI, m/z) = 425.0 (M+H⁺)

### Example 150: N-(3-(3-(4-Fluorophenyl)-8-methyl-4-oxo-3,4-dihydrophthalazin-1-yl)phenyl)ethanesulfonamide

Step 1: 4-Methyl-isobenzofuran-1,3-dione (500 mg, 3.08 mmol) and (3-aminophenyl)boronic acid (602 mg, 3.7 mmol) were reacted in a manner similar to Preparation Example 4, to obtain 281 mg of 3-(4-fluorophenyl)-8-methyl-4-oxo-3,4-dihydrophthalazin-1-yl trifluoromethanesulfonate in white solid.
LC-MS (ESI, m/z) = 403.0 (M+H⁺)

Step 2: The compound (76 mg, 0.189 mmol) obtained in Step 1 was reacted in the same manner as in Example 149, to obtain 29 mg of the title compound in white solid.
LC-MS (ESI, m/z) = 438.0 (M+H⁺)

### Example 151: N-(3-(4-Oxo-3-(tetrahydro-2H-pyran-4-yl)-3,4-dihydrophthalazin-1-yl)phenyl)ethanesulfonamide

4-Iodotetrahydro-2H-pyran (300 mg, 1.333 mmol) was used instead of bromocyclohexane and reacted in the same manner as in Example 128, to obtain 34 mg of the title compound in white solid.
LC-MS (ESI, m/z) = 427.1(M+H⁺)

### Example 152: 2-(3-(3-(4-Fluorophenyl)-4-oxo-3,4-dihydrophthalazin-1-yl)phenyl)-2-methylpropanoic acid

Step 1: Compound I-2 (110 mg, 0.345 mmol) and methyl 2-methyl-2-(3-(4,4,5,5-tetramethyl-1,3,2-dioxaborolan-2-yl)phenyl)propionate (115 mg, 0.0.379 mmol) were reacted in the same manner as in Step 1 of Example 1, to obtain 82 mg of methyl 2-(3-(3-(4-fluorophenyl)-4-oxo-3,4-dihydrophthalazin-1-yl)phenyl)-2-methyl propionate in white solid.
LC-MS (ESI, m/z) = 417.0(M+H⁺)

Step 2: The compound (82 mg, 0.197 mmol) obtained in Step 1 and a 2M aqueous NaOH solution (148 µL, 0.295 mmol) were diluted in MeOH and THF and then stirred at 40°C for two hours. After concentrating, purified water was added, pH of which was adjusted to 6-7 using 1N HCl, and then extraction was performed using DCM. Organic layer was dehydrated over Na₂SO₄, concentrated under reduced pressure, and then crystallized with diethyl ether, to obtain 50 mg of the title compound in white solid.
LC-MS (ESI, m/z) = 403.0(M+H⁺)

### Example 153: 2-(3-(3-(4-Fluorophenyl)-4-oxo-3,4-dihydrophthalazin-1-yl)phenyl)-N,2-dimethylpropanamide

The compound (44 mg, 0.109 mmol) of Example 152, methylamine HCl (8.86 mg, 0.131 mmol) and DIPEA (57.3 µL, 0.328 mmol) were diluted with DMF (0.4 mL), a temperature was lowered to 0 to 5°C, and after adding PyBOP (85 mg, 0.164 mmol), resulting mixture was stirred for 18 hours while gradually increasing a temperature to room temperature. The reaction solution was diluted with EA and washed with purified water, and organic layer was dehydrated over Na₂SO₄, concentrated under reduced pressure, and then reaction product was separated by column chromatography, to obtain 22 mg of the title compound in white solid.
LC-MS (ESI, m/z) = 416.1 (M+H⁺)

### Example 154: 2-Cyclohexyl-4-(3-(methylsulfonyl)phenyl)phthalazin-1(2H)-one

4-Bromo-2-cyclohexylphthalazin-1(2H)-one (19 mg, 0.062 mmol) and (3-(methylsulfonyl)phenyl)boronic acid (14.85 mg, 0.074 mmol) were reacted in the same manner as in Step 2 of Example 128, to obtain 14 mg of the title compound in white solid.
LC-MS (ESI, m/z) = 383.0 (M+H⁺)

### Example 155: (S)-4-(3-Aminoazepan-1-yl)-2-cyclohexyl phthalazin-1(2H)-one·hydrochloride

4-Bromo-2-cyclohexylphthalazin-1(2H)-one (250 mg, 0.814 mmol) and tert-butyl (S)-azepan-3-ylcarbamate (209 mg, 0.977 mmol) were reacted in the same manner as in Example 134, to obtain 108 mg of the title compound in white solid.
LC-MS (ESI, m/z) = 341.2 (M+H⁺)

### Example 156: (S)-2-Cyclohexyl-4-(3-(methylamino) azepan-1-yl)phthalazin-1(2H)-one·hydrochloride

Step 1: 4-Bromo-2-cyclohexylphthalazin-1(2H)-one (250 mg, 0.814 mmol) and tert-butyl (S)-azepan-3-ylcarbamate (209 mg, 0.977 mmol) were reacted in the same manner as in Step 1 of Example 134, to obtain 108 mg of tert-butyl (S)-(1-(3-cyclohexyl-4-oxo-3,4-dihydrophthalazin-1-yl)azepan-3-yl)carbamate in white solid.
LC-MS (ESI, m/z) = 441.1 (M+H⁺)

Step 2: 60% NaH washed with THF was diluted in THF (221 µL), a temperature was lowered to 0°C, and after adding the compound (30 mg, 0.066 mmol) obtained in Step 1, resulting mixture was stirred for 12 minutes at the same temperature. After adding iodomethane (37.6 mg, 0.265 mmol) at 0°C, the temperature was gradually increased to room temperature, and stirring was performed for four hours. The reaction solution was diluted with EA and washed with purified water. Organic layer was dehydrated over Na₂SO₄, concentrated under reduced pressure, and then reaction product was separated by column chromatography, to obtain 22.3 mg of tert-butyl (S)-(1-(3-cyclohexyl-4-oxo-3,4-dihydrophthalazin-1-yl)azepan-3-yl) (methyl)carbamate in yellow oil.
LC-MS (ESI, m/z) = 454.9 (M+H⁺)

Step 3: The compound (22 mg, 0.047 mmol) obtained in Step 2 was reacted in the same manner as in Step 2 of Example 7, to obtain 11.6 mg of the title compound in white solid.
LC-MS (ESI, m/z) = 355.3 (M+H⁺)

### Example 157: (S)-4-(3-Aminoazepan-1-yl)-2-(2,4-difluorophenyl)phthalazin-1(2H)-one·hydrochloride

Step 1: Compound I-9 (22 mg, 0.054 mmol) and tert-butyl (S)-azepan-3-ylcarbamate (29 mg, 0.135 mmol) were diluted in dimethyl sulfoxide (DMSO) and then stirred at 90°C for one hour. The reaction solution was allowed to cool to room temperature, diluted with EA, and then washed with purified water. An organic solvent was dehydrated over Na₂SO₄, concentrated under reduced pressure, and then reaction product was separated by column chromatography, to obtain 14 mg of tert-butyl (S)-(1-(3-(2,4-difluorophenyl)-4-oxo-3,4-dihydrophthalazin-1-yl)azepan-3-yl)carbamate in white solid.
LC-MS (ESI, m/z) = 471.1 (M+H⁺)

Step 2: The compound (14 mg, 0.030 mmol) obtained in Step 1 was reacted in the same manner as in Step 2 of Example 134, to obtain 2.2 mg of the title compound in light brown solid.
LC-MS (ESI, m/z) = 370.9 (M+H⁺)

### Example 158: (S)-4-(3-Aminoazepan-1-yl)-2-(4-fluoro_ phenyl)phthalazin-1(2H)-one·hydrochloride

tert-Butyl (S)-azepan-3-ylcarbamate (161 mg, 0.752 mmol) and Compound I-2 (200 mg, 0.627 mmol) were reacted in the same manner as in Example 134, to obtain 8.7 mg of the title compound in yellow solid.
LC-MS (ESI, m/z) = 353.1(M+H⁺)

### Example 159: (R)-4-(3-Aminoazepan-1-yl)-2-cyclohexyl phthalazin-1(2H)-one·hydrochloride

4-Bromo-2-cyclohexylphthalazin-1(2H)-one (250 mg, 0.814 mmol) and tert-butyl (R)-azepan-3-ylcarbamate (209 mg, 0.977 mmol) were reacted in the same manner as in Example 134, to obtain 108 mg of the title compound in white solid.
LC-MS (ESI, m/z) = 341.2 (M+H⁺)

### Example 160: 2-(3-(3-Cyclohexyl-4-oxo-3,4-dihydrophthalazin-1-yl)phenyl)-2-methylpropanoic acid

4-Bromo-2-cyclohexylphthalazin-1(2H)-one (250 mg, 0.814 mmol) and 2-methyl-2-(3-(4,4,5,5-tetramethyl-1,3,2-dioxaborolan-2-yl)phenyl)propionate (131 mg, 0.430 mmol) were reacted in the same manner as in Example 152, to obtain 62.1 mg of the title compound in white solid.
LC-MS (ESI, m/z) = 391.1(M+H⁺)

### Example 161: 3-(3-(3-(4-Fluorophenyl)-4-oxo-3,4-dihydrophthalazin-1-yl)phenyl)oxazolidin-2-one

Step 1: Compound I-2 (250 mg, 0.783 mmol) and (3-aminophenyl)boronic acid (118 mg, 0.862 mmol) were reacted in the same manner as in Step 1 of Example 1, to obtain 80 mg of 4-(3-aminophenyl)-2-(4-fluorophenyl)phthalazin-1(2H)-one inn ochre solid.
LC-MS (ESI, m/z) = 332.0(M+H⁺)

Step 2: The compound (30 mg, 0.091 mmol) obtained in Step 1 and ethylene carbonate (39.9 mg, 0.453 mmol) were diluted in 1,8-diazabicyclo[5.4.0]undec-7-ene (DBU; 13.65 µL, 0.091 mmol) and then stirred at 100°C for three hours. The reaction solution was allowed to cool to room temperature, diluted with DCM, and then washed with purified water. Organic layer was dehydrated over Na₂SO₄, concentrated under reduced pressure, and then reaction product was separated by column chromatography, to obtain 20.7 mg of the title compound in pale yellow solid.
LC-MS (ESI, m/z) = 402.0(M+H⁺)

### Example 162: (R)-4-(3-Aminoazepan-1-yl)-2-(4-fluoro_ phenyl)phthalazin-1(2H)-one·hydrochloride

Compound I-2 (150 mg, 0.470 mmol) and tert-butyl (R)-azepan-3-ylcarbamate (121 mg, 0.564 mmol) were reacted in the same manner as in Example 134, to obtain 8.5 mg of the title compound in white solid.
LC-MS (ESI, m/z) = 353.1(M+H⁺)

### Example 163: 2-(3-(3-Cyclohexyl-4-oxo-3,4-dihydro phthalazin-1-yl)phenyl)-2-methylpropanamide

The compound (14 mg, 0.036 mmol) of Example 160 and ammonium chloride (2.88 mg, 0.054 mmol) were reacted in the same manner as in Example 153, to obtain 8.6 mg of the title compound in white solid.
LC-MS (ESI, m/z) = 390.1(M+H⁺)

### Example 164: 2-(3-(3-Cyclohexyl-4-oxo-3,4-dihydrophthalazin-1-yl)phenyl)-N,2-dimethylpropanamide

The compound (14 mg, 0.036 mmol) of Example 160 and methylamine hydrochloride (3.63 mg, 0.054 mmol) were reacted in the same manner as in Example 153, to obtain 9.2 mg of the title compound in white solid.
LC-MS (ESI, m/z) = 404.1(M+H⁺)

### Example 165: (R)-4-(3-Aminoazepan-1-yl)-2-(2,4-difluorophenyl)phthalazin-1(2H)-one·hydrochloride

Compound I-9 (300 mg, 0.738 mmol) and tert-butyl (R)-azepan-3-ylcarbamate (396 mg, 1.846 mmol) were reacted in the same manner as in Example 157, to obtain 121.7 mg of the title compound in pale yellow solid.
LC-MS (ESI, m/z) = 371.0(M+H⁺)

### Example 166: 2-(3-(3-(2,4-Difluorophenyl)-4-oxo-3,4-dihydrophthalazin-1-yl)phenyl)-2-methylpropanoic acid

Compound I-9 (300 mg, 0.738 mmol) and 2-methyl-2-(3-(4,4,5,5-tetramethyl-1,3,2-dioxaborolan-2-yl)phenyl) propionate (247 mg, 0.812 mmol) were reacted in the same manner as in Example 152, to obtain 165 mg of the title compound in white solid.
LC-MS (ESI, m/z) = 421.0(M+H⁺)

### Example 167: 4-(3-Aminoazepan-1-yl)-2-(2,4-difluoro phenyl)phthalazin-1(2H)-one·hydrochloride

Compound I-9 (50 mg, 0.123 mmol) and tert-butyl azepan-3-ylcarbamate(65.9 mg, 0.308 mmol) were reacted in the same manner as in Example 157, to obtain 22.9 mg of the title compound in white solid.
LC-MS (ESI, m/z) = 371.1(M+H⁺)

### Example 168: 2-(3-(3-(2,4-Difluorophenyl)-4-oxo-3,4-dihydrophthalazin-1-yl)phenyl)-2-methylpropanenitrile

Compound I-9 (400 mg, 0.985 mmol) and 2-methyl-2-(3-(4,4,5,5-tetramethyl-1,3,2-dioxaborolan-2-yl)phenyl) propanenitrile (294 mg, 1.083 mmol) were reacted in the same manner as in Step 1 of Example 152, to obtain 311.5 mg of the title compound in white solid.
LC-MS (ESI, m/z) = 402.0(M+H⁺)

### Example 169: 4-(3-(2-(1H-Tetrazol-5-yl)propan-2-yl)phenyl)-2-(2,4-difluorophenyl)phthalazin-1(2H)-one

The compound (50 mg, 0.125 mmol) of Example 168, NaN₃ (10.53 mg, 0.162 mmol) and ammonium chloride (9.99 mg, 0.187 mmol) were diluted in DMF and then stirred at 100°C for 16 hours. The reaction solution was allowed to cool to room temperature, diluted with EA, and then washed with purified water. Organic layer was dehydrated over Na₂SO₄, concentrated under reduced pressure, and then reaction product was separated by column chromatography, to obtain 3.1 mg of the title compound in pale yellow solid.
LC-MS (ESI, m/z) = 445.0(M+H⁺)

### Example 170: (E)-2-(3-(3-(2,4-Difluorophenyl)-4-oxo-3,4-dihydrophthalazin-1-yl)phenyl)-N'-hydroxy-2-methyl propanimideamide

The compound (100 mg, 0.249 mmol) of Example 168, NaHCO₃ (41.9 mg, 0.498 mmol) and hydroxylamine hydrochloride (24.23 mg, 0.349 mmol) were diluted in EtOH and then stirred at 80°C for 27 hours. The reaction solution was allowed to cool to room temperature, diluted with EA, and then washed with purified water. Organic layer was dehydrated over Na₂SO₄, concentrated under reduced pressure, and then reaction product was separated by column chromatography, to obtain 11.4 mg of the title compound in white solid.
LC-MS (ESI, m/z) = 435.0(M+H⁺)

### Example 171: 2-(3-(3-(2-Fluorophenyl)-4-oxo-3,4-dihydrophthalazin-1-yl)phenyl)-2-methylpropanoic acid

The 3-(2-fluorophenyl)-4-oxo-3,4-dihydrophthalazin-1-yl trifluoromethanesulfonate (100 mg, 0.258 mmol) prepared by the method of Preparation Example 4 and 2-methyl-2-(3-(4,4,5,5-tetramethyl-1,3,2-dioxaborolan-2-yl)phenyl) propionate (86 mg, 0.283 mmol) were reacted in the same manner as in Example 152, to obtain 37.5 mg of the title compound in white solid.
LC-MS (ESI, m/z) = 403.0(M+H⁺)

### Example 172: 4-(3-Aminoazepan-1-yl)-2-cyclobutyl phthalazin-1(2H)-one·hydrochloride

4-Bromo-2-cyclobutylphthalazin-1(2H)-one (100 mg, 0.358 mmol) prepared by the method of Step 1 of Example 128 while using bromocyclobutane instead of bromocyclohexane was reacted with tert-butyl azepan-3-ylcarbamate(92 mg, 0.430 mmol) in the same manner as in Example 157, to obtain 16.6 mg of the title compound in pale yellow solid.
LC-MS (ESI, m/z) = 313.1(M+H⁺)

### Example 173: 4-(3-Aminoazepan-1-yl)-2-cyclopentyl phthalazin-1(2H)-one·hydrochloride

4-Bromo-2-cyclopentylphthalazin-1(2H)-one (100 mg, 0.341 mmol) prepared by the method of Step 1 of Example 128 while using bromocyclopentane instead of bromocyclohexane, was reacted with tert-butyl azepan-3-ylcarbamate (88 mg, 0.409 mmol) in the same manner as in Example 157, to obtain 23 mg of the title compound inn ochre solid.
LC-MS (ESI, m/z) = 327.2 (M+H⁺)

### Example 174: 2-(2,4-Difluorophenyl)-4-(3-(2-(5-methyl-1,2,4-oxadiazol-3-yl)propan-2-yl)phenyl)phthalazin-1(2H)-one

The compound (40 mg, 0.092 mmol) of Example 170, p-TsOH (5.25 mg, 0.028 mmol) and ZnCl₂ (3.76 mmol, 0.028 mmol) were diluted in CAN, and then stirred at 82°C for 17 hours. The reaction solution was allowed to cool to room temperature, diluted with EA, and washed with purified water and saturated aqueous NaHCO₃. Organic layer was dehydrated over Na₂SO₄, concentrated under reduced pressure, and then reaction product was separated by column chromatography, to obtain 12.5 mg of the title compound in white solid.
LC-MS (ESI, m/z) = 459.0(M+H⁺)

### Example 175: (R)-4-(3-Aminoazepan-1-yl)-2-(2-fluoro phenyl)phthalazin-1(2H)-one·hydrochloride

3-(2-fluorophenyl)-4-oxo-3,4-dihydrophthalazin-1-yl trifluoromethanesulfonate (100 mg, 0.258 mmol) prepared by the method of Preparation Example 4 and tert-butyl (R)-azepan-3-ylcarbamate (138 mg, 0.644 mmol) were reacted in the same manner as in Example 157, to obtain 26.9 mg of the title compound in white solid.
LC-MS (ESI, m/z) = 353.0(M+H⁺)

### Example 176: 2-(3-(3-(2,4-Difluorophenyl)-4-oxo-3,4-dihydrophthalazin-1-yl)phenyl)-N-isopropyl-2-methylpropanamide

The compound (50 mg, 0.119 mmol) of Example 166 and propan-2-amine hydrochloride (17.05 mg, 0,178 mmol) were reacted in the same manner as in Example 153, to obtain 16.4 mg of the title compound in pale yellow solid.
LC-MS (ESI, m/z) = 462.1 (M+H⁺)

### Example 177: 2-(2,4-Difluorophenyl)-4-(3-(2-methyl-1-oxo-1-(pyrrolidin-1-yl)propan-2-yl)phenyl)phthalazin-1(2H)-one

The compound (50 mg, 0.119 mmol) of Example 166 and pyrrolidine (12.69 mg, 0.178 mmol) were reacted in the same manner as in Example 153, to obtain 30.7 mg of the title compound in white solid.
LC-MS (ESI, m/z) = 474.1 (M+H⁺)

### Example 178: N-Cyclopropyl-2-(3-(3-(2,4-difluoro phenyl)-4-oxo-3,4-dihydrophthalazin-1-yl)phenyl)-2-methyl propanamide

The compound (50 mg, 0.119 mmol) of Example 166 and cyclopropanamine (10.19 mg, 0.178 mmol) were reacted in the same manner as in Example 153, to obtain 7.8 mg of the title compound in pale yellow solid.
LC-MS (ESI, m/z) = 460.1 (M+H⁺)

### Example 179: 2-(2,4-Difluorophenyl)-4-(3-(2-methyl-1-morpholino-1-oxopropan-2-yl)phenyl)phthalazin-1(2H)-one

The compound (50 mg, 0.119 mmol) of Example 166 and morpholine (15.54 mg, 0.178 mmol) were reacted in the same manner as in Example 153, to obtain 10.1 mg of the title compound in white solid.
LC-MS (ESI, m/z) = 490.1 (M+H⁺)

### Example 180: N-Cyclohexyl-2-(3-(3-(2,4-difluoro phenyl)-4-oxo-3,4-dihydrophthalazin-1-yl)phenyl)-2-methylpropanamide

The compound (50 mg, 0.119 mmol) of Example 166 and cyclohexanamine (17.69 mg, 0.178 mmol) were reacted in the same manner as in Example 153, to obtain 34.7 mg of the title compound in white solid.
LC-MS (ESI, m/z) = 502.1 (M+H⁺)

### Example 181: 2-(3-(3-(2,4-difluorophenyl)-4-oxo-3,4-dihydrophthalazin-1-yl)phenyl)-2-methyl-N-phenylpropanamide

The compound (50 mg, 0.119 mmol) of Example 166 and aniline (16.61 mg, 0.178 mmol) were reacted in the same manner as in Example 153, to obtain 10.3 mg of the title compound in white solid.
LC-MS (ESI, m/z) = 496.1 (M+H⁺)

### Example 182: 2-(3-(3-(2,4-Difluorophenyl)-4-oxo-3,4-dihydrophthalazin-1-yl)phenyl)-2-methyl-N-(1-methyl-1H-pyrazol-4-yl)propanamide

The compound (50 mg, 0.119 mmol) of Example 166 and 1-methyl-1H-pyrazol-4-amine (17.33 mg, 0.178 mmol) were reacted in the same manner as in Example 153, to obtain 22.8 mg of the title compound in pale orange solid.
LC-MS (ESI, m/z) = 500.1 (M+H⁺)

### Example 183: 1-(3-(3-(2,4-Difluorophenyl)-4-oxo-3,4-dihydrophthalazin-1-yl)phenyl)cyclopropan-1-carboxylic acid

Compound I-9 (122 mg, 0.301 mmol) and 1-(3-(4,4,5,5-tetramethyl-1,3,2-dioxaborolan-2-yl)phenyl)cyclopropan-1-carboxylic acid (100 mg, 0.331 mmol) were reacted in the same manner as in Example 152, to obtain 43.1 mg of the title compound in white solid.
LC-MS (ESI, m/z) = 419.0 (M+H⁺)

### Example 184: 4-(1,4-Diazepan-1-yl)-2-(2,4-difluoro phenyl)phthalazin-1(2H)-one·hydrochloride

Compound I-9 (100 mg, 0.246 mmol) and tert-butyl 1,4-diazepane-1-carboxylate (59.2 mg, 0.295 mmol) were reacted in the same manner as in Example 157, to obtain 5 mg of the title compound in white solid.
LC-MS (ESI, m/z) = 392.1 (M+H⁺)

### Example 185: (R)-4-(3-Aminoazepan-1-yl)-2-(4-chloro phenyl)phthalazin-1(2H)-one·hydrochloride

3-(4-chlorophenyl)-4-oxo-3,4-dihydrophthalazin-1-yl trifluoromethanesulfonate (100 mg, 0.247 mmol) prepared by the method of Preparation Example 2 and tert-butyl (R)-azepan-3-ylcarbamate (63.5 mg, 0.296 mmol) were reacted in the same manner as in Example 157, to obtain 72.5 mg of the title compound in white solid.
LC-MS (ESI, m/z) = 369.1 (M+H⁺)

### Example 186: 2-(3-(3-(2,4-Difluorophenyl)-4-oxo-3,4-dihydrophthalazin-1-yl)phenyl)-N-(2-hydroxyethyl)-2-methyl propanamide

Step 1: The compound (100 mg, 0.238 mmol) of Example 166 and 2-((tert-butyldimethylsilyl)oxy)ethan-1-amine (62.6 mg, 0.357 mmol) were reacted in the same manner as in Example 153, to obtain 50 mg of N-(2-((tert-butyldimethylsilyl)oxy)ethyl)-2-(3-(3-(2,4-difluorophthal)-4-oxo-3,4-dihydrophthalazin-1-yl)phenyl)-2-methylpropan amide.
LC-MS (ESI, m/z) = 577.7 (M+H⁺)

Step 2: The compound (50 mg, 0.087 mmol) obtained in Step 1 was dissolved in THF (433 µL) and, after adding a 1M tetra-n-butylammonium fluoride (TBAF) solution in THF (104 µL, 0.104 mmol) dropwise at -5°C, resulting mixture was stirred for 40 minutes. After adding water (10 mL), the reaction solution was diluted with EA and washed with purified water. Organic layer was dehydrated over Na₂SO₄, concentrated under reduced pressure, and then reaction product was separated by column chromatography, to obtain 17 mg of the title compound in white solid.
LC-MS (ESI, m/z) = 464.1 (M+H⁺)

### Example 187: (R)-N-(1-(3-(2,4-Difluorophenyl)-4-oxo-3,4-dihydrophthalazin-1-yl)azepan-3-yl)ethanesulfonamide

The compound (50 mg, 0.123 mmol) of Example 165 was dissolved in DCM (410 µL) and, after adding DIPEA (61.4 µL, 0.369 mmol) and ethanesulfonyl chloride (18.63 µL, 0.197 mmol) dropwise at 0°C, resulting mixture was stirred for three hours at room temperature. The reaction product was diluted with EA and washed with purified water, and subsequently, organic layer was separated, dehydrated over anhydrous Na₂SO₄, concentrated under reduced pressure, and then reaction product was separated by column chromatography, to obtain 18.5 mg of the title compound in white solid.
LC-MS (ESI, m/z) = 463.1 (M+H⁺)

### Example 188: 2-(3-(3-(2,4-Difluorophenyl)-4-oxo-3,4-dihydrophthalazin-1-yl)phenyl)-2-methylpropanamide

The compound (33 mg, 0.078 mmol) of Example 166 and ammonium chloride (6.3 mg, 0,178 mmol) were reacted in the same manner as in Example 153, to obtain 10.7 mg of the title compound in white solid.
LC-MS (ESI, m/z) = 420.1 (M+H⁺)

### Example 189: (R)-2-(2,4-Difluorophenyl)-4-(3-(methylamino)azepan-1-yl)phthalazin-1(2H)-one·hydrochloride

Step 1: The compound (50 mg, 0.123 mmol) of Example 165 was dissolved in DMF and, after adding calcium carbonate (51.0 mg, 0.369 mmol) and iodomethane (17.44 mmol, 0.123 mmol), resulting mixture was stirred at 50°C for 18 hours. The reaction product was diluted with EA and washed with purified water, and subsequently, organic layer was separated, dehydrated over anhydrous Na₂SO₄, and concentrated under reduced pressure, and then reaction product was separated by column chromatography, to obtain 20 mg of tert-butyl (R)-(1-(3-(2,4-difluorophenyl)-4-oxo-3,4-dihydrophthalazin-1-yl)azepan-3-yl) (methyl) carbamate in pale yellow solid.
LC-MS (ESI, m/z) = 384.1 (M+H⁺)

Step 2: The compound (20 mg, 0.052 mmol) obtained in Step 1 was dissolved in DCM and, after slowly adding a 4M HCl solution in dioxane (65.0 µL, 0.260 mmol) dropwise at 0°C, resulting mixture was stirred for one hour. The reaction solution was concentrated under reduced pressure, to obtain 7.8 mg of the title compound in pale yellow solid.
LC-MS (ESI, m/z) = 384.1 (M+H⁺)

### Example 190: 2-(2,4-Difluorophenyl)-4-(3-hydroxy azepan-1-yl)phthalazin-1(2H)-one

Step 1: Azepanone (200 mg, 1.767 mmol) was dissolved in methanol and, after slowly adding sodium bicarbonate (66.9 mg, 1.767 mmol) at 0°C, resulting mixture was stirred at room temperature for three hours. The reaction mixture was concentrated under reduced pressure and, after adding water and adjusting pH to 8-9 using 1N HCl, diluted with EA and washed with purified water. Organic layer was dehydrated over anhydrous Na₂SO₄, concentrated under reduced pressure, and then reaction product was separated by column chromatography, to obtain 98 mg of azepanol in pale yellow oil.
LC-MS (ESI, m/z) = 116.1 (M+H⁺)

Step 2: The compound (25 mg, 0.217 mmol) obtained in Step 1, 3-(4-chlorophenyl)-4-oxo-3,4-dihydrophthalazin-1-yl trifluoromethanesulfonate (76.5 mg, 0.181 mmol) and DIPEA (63.2 µL, 0.362 mmol) were reacted in the same manner as in Example 165, to obtain 3 mg of the title compound in white solid.
LC-MS (ESI, m/z) = 372.1 (M+H⁺)

### Example 191: (R)-2-(2,4-Difluorophenyl)-4-(3-((2-hydroxyethyl)amino)azepan-1-yl)phthalazin-1(2H)-one·hydro chloride

Step 1: The compound (50 mg, 0.123 mmol) of Example 165 was dissolved in DMF and, after adding (2-bromoethoxy) (tert-butyl)dimethylsilane (32.3 mg, 0.135 mmol) and cesium carbonate (80 mg, 0.246 mmol), resulting mixture was stirred at 50°C for 16 hours. The reaction product was diluted with EA and washed with purified water, and subsequently, organic layer was separated, dehydrated over anhydrous Na₂SO₄, concentrated under reduced pressure, and then reaction product was separated by column chromatography, to obtain 26 mg of (R)-4-(3-((2-((tert-butyldimethylsilyl)oxy)ethyl)amino)azepan-1-yl)phthalazin-1(2H)-one in pale yellow oil.
LC-MS (ESI, m/z) = 528.7 (M+H⁺)

Step 2: The compound (44.4 mg, 0.084 mmol) obtained in Step 1 was dissolved in DCM and, after slowly adding a 4M HCl solution in dioxane (840 µL, 3.36 mmol) dropwise at 0°C, resulting mixture was stirred at room temperature for one hour and thirty minutes. After adding water to the reaction mixture, pH of which was adjusted to 9 using saturated aqueous NaHCO₃, and then reaction product was extracted with EA and washed with purified water. Organic layer was dehydrated over anhydrous Na₂SO₄ and concentrated under reduced pressure, to obtain 22.8 mg of (R)-2-(2,4-difluorophenyl)-4-(3-((2-hydroxyethyl)amino)azepan-1-yl)phthalazin-1(2H)-one in pale yellow oil.
LC-MS (ESI, m/z) = 415.1 (M+H⁺)

Step 3: The compound (22.8 mg, 0.055 mmol) obtained in Step 2 was reacted in the same manner as in Step 2 of Example 189, to obtain 10.8 mg of the title compound in white solid.
LC-MS (ESI, m/z) = 415.1 (M+H⁺)

### Example 192: (R)-N-(1-(3-(2,4-Difluorophenyl)-4-oxo-3,4-dihydrophthalazin-1-yl)azepan-3-yl)acetamide

The compound (50 mg, 0.123 mmol) of Example 165 and acetyl chloride were reacted in the same manner as in Example 187, to obtain 12 mg of the title compound in white solid.
LC-MS (ESI, m/z) = 413.1 (M+H⁺)

### Example 193: 2-(2,4-Difluorophenyl)-4-(3-oxoazepan-1-yl)phthalazin-1(2H)-one

The compound (38.5 mg, 0.104 mmol) of Example 190 was dissolved in DCM and, after adding Dess-Martin periodinane (44 mg, 0.104 mmol) at 0°C, resulting mixture was stirred at room temperature for 23 hours. The reaction product was diluted with DCM and washed with purified water, and subsequently, organic layer was separated, dehydrated over anhydrous Na₂SO₄, concentrated under reduced pressure, and then reaction product was separated by column chromatography, to obtain 5.7 mg of the title compound in white solid.
LC-MS (ESI, m/z) = 370.1 (M+H⁺)

### Example 194: 2-(3-(3-(4-Chlorophenyl)-4-oxo-3,4-dihydrophthalazin-1-yl)phenyl)-2-methylpropanoic acid

The 3-(4-chlorophenyl)-4-oxo-3,4-dihydrophthalazin-1-yl trifluoromethanesulfonate (400 mg, 0.988 mmol) prepared by the method of Preparation Example 2 and methyl 2-methyl-2-(3-(4,4,5,5-tetramethyl-1,3,2-dioxaborolan-2-yl)phenyl)propionate (331 mg, 1.087 mmol) were reacted in the same manner as in Example 152, to obtain 219.7 mg of the title compound in white solid.
LC-MS (ESI, m/z) = 419.1 (M+H⁺)

### Example 195: 1-(3-(3-(4-Chlorophenyl)-4-oxo-3,4-dihydrophthalazin-1-yl)phenyl)cyclopropan-1-carboxylic acid

3-(4-Chlorophenyl)-4-oxo-3,4-dihydrophthalazin-1-yl trifluoromethanesulfonate (100 mg, 0.247 mmol) and methyl 1-(3-(4,4,5,5-tetramethyl-1,3,2-dioxaborolan-2-yl)phenyl)cyclopropan-1-carboxylate (82 mg, 0.272 mmol) were reacted in the same manner as in Example 152, to obtain 13.5 mg of the title compound in white solid.
LC-MS (ESI, m/z) = 417.0 (M+H⁺)

### Example 196: 2-(4-Chlorophenyl)-4-(3-hydroxyazepan-1-yl)phthalazin-1(2H)-one

3-(4-Chlorophenyl)-4-oxo-3,4-dihydrophthalazin-1-yl trifluoromethanesulfonate (179 mg, 0.442 mmol) and azepanone (102 mg, 0.884 mmol) were reacted in the same manner as in Example 190, to obtain 4 mg of the title compound in pale yellow solid.
LC-MS (ESI, m/z) = 370.1 (M+H⁺)

### Example 197: 2-(3-(3-(4-Chlorophenyl)-4-oxo-3,4-dihydrophthalazin-1-yl)phenyl)-2-methylpropanamide

The compound (50 mg, 0.119 mmol) of Example 194 was reacted in the same manner as in Example 163, to obtain 37.2 mg of the title compound in white solid.
LC-MS (ESI, m/z) = 418.1 (M+H⁺)

### Example 198: 1-(3-(3-(4-Chlorophenyl)-4-oxo-3,4-dihydrophthalazin-1-yl)phenyl)cyclopropan-1-carboxyamide

The compound (11 mg, 0.026 mmol) of Example 195 was reacted in the same manner as in Example 163, to obtain 6.7 mg of the title compound in white solid.
LC-MS (ESI, m/z) = 416.1 (M+H⁺)

### Example 199: 2-(3-(3-(2,4-Difluorophenyl)-4-oxo-3,4-dihydrophthalazin-1-yl)phenyl)acetic acid

Compound I-9 (100 mg, 0.246 mmol) and methyl 2-(3-(4,4,5,5-tetramethyl-1,3,2-dioxaborolan-2-yl)phenyl)acetate (82 mg, 0.295 mmol) were reacted in the same manner as in Example 152, to obtain 31.9 mg of the title compound in white solid.
LC-MS (ESI, m/z) = 393.0 (M+H⁺)

### Example 200: 4-(3-(2-Aminopropan-2-yl)phenyl)-2-(2,4-difluorophenyl)phthalazin-1(2H)-one·hydrochloride

Step 1: Compound I-9 (19.7 mg, 0.048 mmol) and 2-(3-(4,4,5,5-tetramethyl-1,3,2-dioxaborolan-2-yl)phenyl)carbamate (21 mg, 0.058 mmol) were reacted in the same manner as in Example 152, to obtain 14.5 mg of tert-butyl(2-(3-(4,4,5,5-tetramethyl-1,3,2-dioxaborolan-2-yl)phenyl)propan-2-yl)carbamate in white solid.
LC-MS (ESI, m/z) = 492.1 (M+H⁺)

Step 2: The compound (21 mg, 0.058 mmol) obtained in Step 1 was reacted in the same manner as in Step 2 of Example 157, to obtain 8.3 mg of the title compound in white solid.
LC-MS (ESI, m/z) = 393.0 (M+H⁺)

### Example 201: 2-(3-(3-(4-Chloro-2-fluorophenyl)-4-oxo-3,4-dihydrophthalazin-1-yl)phenyl)-2-methylpropanoic acid

2-(4-chloro-2-fluorophenyl)-4-((trifluoromethyl) sulfonyl)-3,4-dihydrophthalazin-1(2H)-one (100 mg, 0.237 mmol) prepared by the method of Preparation Example 2 was reacted in the same manner as in Example 152, to obtain 31.6 mg of the title compound in white solid.
LC-MS (ESI, m/z) = 437.0 (M+H⁺)

### Example 202: (R)-4-(3-Aminoazepan-1-yl)-2-(4-chloro-2-fluorophenyl)phthalazin-1-(2H)-one·hydrochloride

2-(4-Chloro-2-fluorophenyl)-4-((trifluoromethyl) sulfonyl)-3,4-dihydrophthalazin-1(2H)-one (100 mg, 0.237 mmol) was used as a starting material and reacted in the same manner as in Example 157, to obtain 38.7 mg of the title compound in white solid.
LC-MS (ESI, m/z) = 387.0 (M+H⁺)

### Example 203: 1-(3-(4-Chloro-2-fluorophenyl)-4-oxo-3,4-dihydrophthalazin-1-yl)azepane-3-carbonitrile

2-(4-Chloro-2-fluorophenyl)-4-((trifluoromethyl) sulfonyl)-3,4-dihydrophthalazin-1(2H)-one (127 mg, 0.301 mmol) and azepane-3-carbonitrile (74.7 mg, 0.602 mmol) were reacted in the same manner as in Example 157, to obtain 50.8 mg of the title compound in white solid.
LC-MS (ESI, m/z) = 397.0 (M+H⁺)

### Example 204: 4-(3-(1-Amino-2-methylpropan-2-yl)phenyl)-2-(2,4-difluorophenyl)phthalazin-1(2H)-one·hydro chloride

Step 1: The compound (30 mg, 0.075 mmol) of Example 168 was dissolved in methanol and, after adding 10% Pd/C (10 mg, 0.075 mmol) and 4M HCl (two drops) and then injecting hydrogen (g), resulting mixture was stirred at room temperature for 18 hours. The reaction product was diluted with EA and then washed with purified water. Organic layer was dehydrated over Na₂SO₄, concentrated under reduced pressure, and then reaction product was separated by column chromatography, to obtain 5 mg of 4-(3-(1-amino-2-methylpropan-2-yl)phenyl)-2-(2,4-difluorophenyl)phthalazin-1(2H)-one in light brown solid.
LC-MS (ESI, m/z) = 406.1 (M+H⁺)

Step 2: The compound (5 mg, 0.012 mmol) obtained in Step 1 was reacted in the same manner as in Step 2 of Example 157, to obtain 1.1 mg of the title compound in light brown solid.
LC-MS (ESI, m/z) = 406.1 (M+H⁺)

### Example 205: 2-(1-(3-(2,4-Difluorophenyl)-4-oxo-3,4-dihydrophthalazin-1-yl)piperidin-3-yl)-2-methylpropanoic acid

Step 1: Compound I-9 (100 mg, 0.246 mmol) and methyl 2-methyl-2-(piperidin-3-yl)propionate (91 mg, 0.492 mmol) were reacted in the same manner as in Example 157, to obtain 42.6 mg of methyl 2-(1-3-(2,4-difluorophenyl)-4-oxo-3,4-dihydrophthalazin-1-yl)-2-methylpropionate in pale yellow solid.
LC-MS (ESI, m/z) = 442.1 (M+H⁺)

Step 2: The compound (42.6 mg, 0.096 mmol) obtained in Step 1 was reacted in the same manner as in Step 2 of Example 152, to obtain 6.2 mg of the title compound in pale yellow solid.
LC-MS (ESI, m/z) = 428.1 (M+H⁺)

### Example 206: 2-(3-(3-(4-Cyclopropylphenyl)-4-oxo-3,4-dihydrophthalazin-1-yl)phenyl)-2-methylpropanoic acid

Step 1: 4-Bromophthalazin-1(2H)-one (300 mg, 1.333 mmol) and (4-cyclopropylphenyl)boronic acid (324 mg, 2.0 mmol) were reacted in the same manner as in Example 128, to obtain 110 mg of 4-bromo-2-(4-cyclopropylphenyl)phthalazin-1(2H)-one in white solid.
LC-MS (ESI, m/z) = 342.1 (M+H⁺)

Step 2: The compound (110 mg, 0.322 mmol) obtained in Step 1 was reacted in the same manner as in Example 152, to obtain 62.8 mg of the title compound in white solid.
LC-MS (ESI, m/z) = 425.1 (M+H⁺)

### Example 207: 1-(3-(3-(2,4-Difluorophenyl)-4-oxo-3,4-dihydrophthalazin-1-yl)phenyl)cyclobutane-1-carboxylic acid

Compound I-9 (100 mg, 0.246 mmol) and methyl 1-(3-bromophenyl)cyclobutane-1-carboxylate (93 mg, 0.295 mmol) were reacted in the same manner as in Example 152, to obtain 76.3 mg of pale yellow title compound.
LC-MS (ESI, m/z) = 433.0 (M+H⁺)

### Example 208: 1-(3-(3-(2,4-Difluorophenyl)-4-oxo-3,4-dihydrophthalazin-1-yl)phenyl)cyclopentane-1-carboxylic acid

Compound I-9 (100 mg, 0.246 mmol) and methyl 1-(3(4,4,5,5-tetramethyl-1,3,4-dioxaborolan-2-yl)phenyl)cyclopentane-1-carboxylate (98 mg, 0.295 mmol) were reacted in the same manner as in Example 152, to obtain 77.3 mg of the title compound in white solid.
LC-MS (ESI, m/z) = 447.0 (M+H⁺)

### Example 209: 2-(1-(3-(4-Chlorophenyl)-4-oxo-3,4-dihydrophthalazin-1-yl)piperidin-3-yl)-2-methylpropanoic acid

The compound (100 mg, 0.247 mmol) of Example 195 and methyl 2-methyl-2-(piperidin-3-yl)propionate were reacted in the same manner as in Example 205, to obtain 3.5 mg of the title compound in pale pink solid.
LC-MS (ESI, m/z) = 426.1 (M+H⁺)

### Examples 210-1 and 210-2: 2-(1-(3-(2,4-Difluoro phenyl)-4-oxo-3,4-dihydrophthalazin-1-yl)piperidin-3-yl)-2-methylpropanoic acid

Step 1: The compound (117 mg, 0.274 mmol) of Example 209 was dissolved in THF (0.55 mL) and, after adding hexafluorophosphate azabenzotriazole tetramethyl uronium (HATU; 114 mg, 0.301 mmol) and DIPEA (57.4 µL), resulting mixture was stirred at 35°C for one hour. In another reaction vessel, 60% NaH washed with THF was diluted in THF (0.55 mL), a temperature was lowered to 0°C, and after adding (R)-4-benzyloxazolidin-2-one (150 mg, 0.849 mmol), resulting mixture was stirred for 30 minutes. The solution was added, at 0°C, to a mixed solution in which 2-(1-(3-(4-chlorophenyl)-4-oxo-3,4-dihydrophthalazin-1-yl)piperidin-3-yl)-2-methylpropanoic acid is dissolved, and then stirred for one hour. The reaction product was extracted with EA and then washed three times with distilled water. Organic layer was dehydrated over Na₂SO₄, concentrated under reduced pressure, and then reaction product was separated by column chromatography, to obtain 56 mg of each of Compound a (up spot) and Compound b (down spot) ((5-benzyl-3-(2-(1-(3-(2,4-difluorophenyl)-4-oxo-3,4-dihydrophthalazin-1-yl)piperidin-3-yl)-2-methylpropanoyl)oxazolidin-2-one)) in pale yellow solid.
LC-MS (ESI, m/z) = 587.1 (M+H⁺)

Step 2: Compound a (up spot) (24.9 mg, 0.042 mmol) obtained in Step 1 was dissolved in THF/water (4:1, 0.29 mL:0.07 mL) and, after adding 30% hydrogen peroxide (15.69 µL, 0.514 mmol) at 0°C, resulting mixture was stirred for one hour. After concentrating the reaction solution, water was added, and then pH was adjusted to 2 with 1N HCl. The reaction product was extracted with EA and washed with distilled water. Organic layer was dehydrated over Na₂SO₄ and concentrated under reduced pressure, to obtain 7.9 mg of the compound of Example 210-1 in white solid.
LC-MS (ESI, m/z) = 428.1 (M+H⁺)

Compound b (down spot) (32 mg, 0.055 mmol) obtained in Step 1 was used and reacted in the same manner as Compound a, to obtain 12.7 mg of the compound of Example 210-2 in white solid.
LC-MS (ESI, m/z) = 428.1 (M+H⁺)

### Example 211: Methyl 1-(1-(3-(2,4-difluorophenyl)-4-oxo-3,4-dihydrophthalazin-1-yl)piperidin-3-yl)cyclopropan-1-carboxylate

Compound I-9 (420 mg, 1.03 mmol), methyl 1-(piperidin-3-yl)cyclopropan-1-carboxylate hydrochloride (303 mg, 1.65 mmol) and DIPEA (0.36 ml, 2.07 mmol) were dissolved in THF (15 ml) and then stirred at 110°C for 48 hours. The reaction product was diluted with EtOAc and washed with purified water. Organic layer was separated, dehydrated over anhydrous Na₂SO₄, concentrated under reduced pressure, and then reaction product was separated by column chromatography, to obtain 275 mg of the title compound.
LC-MS (ESI, m/z) = 440.0 (M+H⁺)

### Example 212: 1-(1-(3-(2,4-Difluorophenyl)-4-oxo-3,4-dihydrophthalazin-1-yl)piperidin-3-yl)cyclopropan-1-carboxylic acid

The compound (100 mg, 0.23 mmol) of Example 211 was reacted in the same manner as in Step 2 of Example 152, to obtain 87 mg of the title compound.
LC-MS (ESI, m/z) = 426.0 (M+H⁺)

### Examples 213-1 and 213-2: 1-(1-(3-(2,4-Difluoro phenyl)-4-oxo-3,4-dihydrophthalazin-1-yl)piperidin-3-yl)cyclopropan-1-carboxylic acid

The compound (163 mg, 0.383 mmol) of Example 212 was used and reacted in the same manner as in Example 210, to obtain 28.2 mg of a compound of Example 213-1 and 11.8 mg of a compound of Example 213-2 were obtained in white solids.
LC-MS (ESI, m/z) = 426.1 (M+H⁺)

### Example 214: 2-(2,4-Difluorophenyl)-4-(3-(pyrrolidin-1-ylsulfonyl)phenyl)phthalazin-1(2H)-one

Compound I-9 (100 mg, 0.25 mmol), (3-(pyrrolidin-1-ylsulfonyl)phenyl)boronic acid (71 mg, 0.28 mmol), Pd(PPh₃)₄ (30 mg, 0.02 mmol) and Na₂CO₃ (130 mg, 1.23 mmol) were added to 1,4-dioxane/distilled water (10 mL, 1:1 ratio), and then resulting mixture was stirred at 100°C for five hours. The reaction product was diluted with EtOAc and washed with purified water. Organic layer was separated, dehydrated over anhydrous Na₂SO₄, concentrated under reduced pressure, and then reaction product was separated by column chromatography, to obtain 55 mg of the title compound.
LC-MS (ESI, m/z) = 468.1 (M+H⁺)

### Example 215: 2-(2,4-Difluorophenyl)-4-(1,2,3,6-tetrahydropyridin-4-yl)phthalazin-1(2H)-one·hydrochloride

Step 1: Compound I-9 (100 mg, 0.25 mmol) and tert-butyl-4-(4,4,5,5-tetramethyl-1,3,2-dioxaborolan-2-yl)-3,6-dihydropyridin-1(2H)-carboxylate (92 mg, 0.30 mmol) were reacted in the same manner as in Example 214, to obtain 80 mg of tert-butyl-4-(3-(2,4-difluorophenyl)-4-oxo-3,4-dihydrophthalazin-1-yl)-3,6-dihydropyridin-1(2H)-carboxylate.
LC-MS (ESI, m/z) = 444.2 (M+H⁺)

Step 2: The compound (70 mg, 0.16 mmol) obtained in Step 1 was reacted in the same manner as in Step 2 of Example 157, to obtain 50 mg of the title compound.
LC-MS (ESI, m/z) = 340.2 (M+H⁺)

### Example 216: 3-(3-(2,4-Difluorophenyl)-4-oxo-3,4-dihydrophthalazin-1-yl)-N-ethyl-N-methylbenzenesulfonamide

The compound (70 mg, 0.16 mmol) of Example 91 was dissolved in anhydrous MeCN (5 ml) and, after adding K₂CO₃ (90 mg, 0.66 mmol) and bromoethane (20 µL, 0.33 mmol), resulting mixture was stirred at 90°C for two hours. The reaction product was diluted with EtOAc and washed with purified water. Organic layer was separated, dehydrated over anhydrous Na₂SO₄, concentrated under reduced pressure, and then reaction product was separated by column chromatography, to obtain 66 mg of the title compound.
LC-MS (ESI, m/z) = 456.5 (M+H⁺)

### Example 217: 3-(3-(2,4-Difluorophenyl)-4-oxo-3,4-dihydrophthalazin-1-yl)-N,N-diethylbenzenesulfonamide

The compound (70 mg, 0.16 mmol) of Example 92 was used and reacted in the same manner as in Example 216, to obtain 65 mg of the title compound.
LC-MS (ESI, m/z) = 470.5 (M+H⁺)

### Example 218: 2-(2,4-Difluorophenyl)-4-(1-(ethylsulfonyl)-1,2,3,6-tetrahydropyridin-4-yl)phthalazin-1(2H)-one

The compound (40 mg, 0.10 mmol) of Example 216 was dissolved in DCM (3 ml), and after adding ethane sulfonylchloride (20 mg, 0.16 mmol) and pyridine (18 µl, 0.21 mmol), resulting mixture was stirred at room temperature for four hours. The reaction product was diluted with EtOAc and washed with purified water. Organic layer was separated, dehydrated over anhydrous Na₂SO₄, concentrated under reduced pressure, and then reaction product was separated by column chromatography, to obtain 35 mg of the title compound.
LC-MS (ESI, m/z) = 432.1 (M+H⁺)

### Example 219: 2-(4-(3-(2,4-Difluorophenyl)-4-oxo-3,4-dihydrophthalazin-1-yl)piperazin-2-yl)-2-methylpropanoic acid

Step 1: Compound I-9 (200 mg, 0.50 mmol) and Compound I-16 (100 mg, 0.54 mmol) were reacted in a manner similar to Example 212, to obtain 130 mg of methyl 2-(4-(3-(2,4-difluorophenyl)-4-oxo-3,4-dihydrophthalazin-1-yl)piperazin-2-yl)-2-methylpropionate.
LC-MS (ESI, m/z) = 443.1 (M+H⁺)

Step 2: The compound (40 mg, 0.09 mmol) obtained in Step 1 was dissolved in THF/MeOH/H₂O (4 mL, 3:1:1 ratio) and, after adding LiOH·H₂O (11 mg, 0.27 mmol), resulting mixture was stirred at room temperature for five hours. The reaction product was diluted with EtOAc and washed with saturated aqueous NH₄Cl solution. Organic layer was separated, dehydrated over anhydrous Na₂SO₄, and concentrated under reduced pressure, to obtain 16 mg of the title compound.
LC-MS (ESI, m/z) = 429.2 (M+H⁺)

### Example 220: 2-(4-(3-(2,4-Difluorophenyl)-4-oxo-3,4-dihydrophthalazin-1-yl)-1-methylpiperazin-2-yl)-2-methylpropanoic acid

Step 1: The compound (80 mg, 0.18 mmol) obtained in Step 1 of Example 219 was dissolved in 1,4-dixoane (5 ml) and, after adding iodomethane (20 µl, 0.27 mmol) and K₂CO₃ (75 mg, 0.54 mmol), resulting mixture was stirred at room temperature for four hours. The reaction product was diluted with EtOAc and washed with purified water. Organic layer was separated, dehydrated over anhydrous Na₂SO₄ and concentrated under reduced pressure, and then reaction product was separated by column chromatography, to obtain 65 mg of methyl 2-(4-(3-(2,4-difluorophenyl)-4-oxo-3,4-dihydrophthalazin-1-yl)-1-methylpiperazin-2-yl)-2-methylpropionate.
LC-MS (ESI, m/z) = 457.1 (M+H⁺)

Step 2: The compound (65 mg, 0.14 mmol) obtained in Step 1 was reacted in the same manner as in Step 2 of Example 219, to obtain 46 mg of the title compound.
LC-MS (ESI, m/z) = 443.2 (M+H⁺)

### Example 221: 2-(4-(3-(2,4-Difluorophenyl)-4-oxo-3,4-dihydrophthalazin-1-yl)morpholin-2-yl)-2-methylpropanoic acid

Step 1: Compound I-9 (500 mg, 1.23 mmol) and methyl 2-(morpholin-2-yl)acetate (265 mg, 1.35 mmol) were reacted in the same manner as in Example 211, to obtain 360 mg of methyl 2-(4-(3-(2,4-difluorophenyl)-4-oxo-3,4-dihydro phthalazin-1-yl)morpholin-2-yl)acectate.
LC-MS (ESI, m/z) = 416.1 (M+H⁺)

Step 2: The compound (250 mg, 0.60 mmol) obtained in Step 1 was reacted in the same manner as in Step 2 of Preparation Example 5, to obtain 120 mg of methyl 2-(4-(3-(2,4-difluorophenyl)-4-oxo-3,4-dihydrophthalazin-1-yl)morpholin-2-yl)-2-methylpropionate.
LC-MS (ESI, m/z) = 444.1 (M+H⁺)

Step 3: The compound (70 mg, 0.16 mmol) obtained in Step 2 was reacted in the same manner as in Step 2 of Example 219, to obtain 16 mg of the title compound.
LC-MS (ESI, m/z) = 430.1 (M+H⁺)

### Experimental Example 1: Measurement of Sirt6 Activity

Compounds having Sirt6 activity were selected using a CycLex Sirt6 Deacetylase Fluorometric Assay Kit (cat. CY-1156V2) manufactured by CycLex.

When a fluorescent-attached acetylated lysine substrate is treated with Sirt6 enzyme, Sirt6 deacetylates the acetylated lysine of the substrate and lysylendopeptidase cleaves the C-terminal peptide bond connected to the lysine, by which fluorescence is emitted. The activity of a compound is calculated by observing the intensity of fluorescence when the compound is treated along with Sirt6 enzyme. When treating the compound, the compound aids in the activity of Sirt6 enzyme, so as to result in stronger fluorescence. Therefore, by calculating the ratio of the stronger fluorescence relative to the fluorescence emitted from the group without compound treatment (i.e., positive control group), relative activity can be calculated.

Activity evaluation was performed according to the following procedure.

The compound whose activity is to be evaluated was prepared by diluting in phosphate-buffered saline (PBS) to 10 times of the desired concentration (e.g., when measuring Sirt6 assay activity at 10 µM, the compound is prepared at a concentration of 100 µM in PBS). 1 µL of a 10x Sirt6 assay buffer, 0.8 µL of 50x fluoro-substrate peptide, 1 µL of nicotinamide adenine dinucleotide (NAD), 1 µL of a developer and 4.2 µL of H₂O were mixed to prepare mixtures equal to the number of samples, and 8 µL was dispensed into each Black 384 well. An additional 1 µL of PBS was added to each of a "no enzyme group" and a positive control group, and 1 µL of the compound diluted in PBS was added to an activity evaluation group. Recombinant Sirt6 was 1:8 diluted in a 1x Sirt6 assay buffer, and 1 µL thereof was added to each of the positive control group and the activity evaluation group. 1 µL of the 1x Sirt6 assay buffer was added to the "no enzyme group." After reacting at room temperature for one hour, results were measured with a fluorescence spectrometer (excitation: 490±10 nm, emission: 530±10 nm) to confirm the presence or absence of Sirt6 activity and measure the intensity of the activity.

Table 1 below summarizes the names and Sirt6 activity of compounds according to the present invention.

**Table 1**

| **Example No.** | **Compound Name** | **Sirt6 Activity (% activity, 10 µM)** |
|---|---|---|
| 1 | N-(3-(4-oxo-3-phenyl-3,4-dihydrophthalazin-1-yl)phenyl)ethanesulfonamide | 170 |
| 2 | 4-(3-aminophenyl)-2-(4-(trifluoromethyl)phenyl)phthalazin -1(2H)-one | 88 |
| 3 | N-(3-(4-oxo-3-(4-(trifluoromethyl)phenyl)-3,4-dihydrophthalazin-1-yl)phenyl)ethanesulfonamide | 104 |
| 4 | 4-(3-(ethylamino)phenyl)-2-(4-(trifluoromethyl)phenyl)phthalazin -1(2H)-one | 102 |
| 5 | N-methyl-N-(3-(4-oxo-3-(4-(trifluoromethyl)phenyl)-3,4-dihydrophthalazin-1-yl)phenyl)ethanesulfonamide | 105 |
| 6 | N-(3-(4-oxo-3-phenyl-3,4-dihydrophthalazin-1-yl)phenyl)methanesulfonamide | 102 |
| 7 | N-methyl-N-(3-(4-oxo-3-phenyl-3,4-dihydrophthalazin-1-yl)phenyl)methanesulfonamide | 173 |
| 8 | N-(4-(4-oxo-3-phenyl-3,4-dihydrophthalazin-1-yl)phenyl)ethanesulfonamide | 100 |
| 9 | N-(3-(3-phenyl)-4-oxo-3,4-dihydrophthalazin-1-yl)phenyl)-dimethylsulfamoylamide | 154 |
| 10 | N-(3-(3-(4-fluorophenyl)-4-oxo-3,4-dihydrophthalazin-1-yl)phenyl)methanesulfonamide | 198 |
| 11 | N-(3-(3-(4-fluorophenyl)-4-oxo-3,4-dihydrophthalazin-1-yl)phenyl)ethanesulfonamide | 162 |
| 12 | N-(3-(3-(4-fluorophenyl)-4-oxo-3,4-dihydrophthalazin-1-yl)phenyl)-dimethylsulfamoylamide | 189 |
| 13 | (Z)-N-methyl-1-(3-(4-oxo-3-phenyl-3,4-dihydrophthalazin-1-yl)phenyl)methanimine oxide | 181 |
| 14 | (Z)-N-isopropyl-1-(3-(4-oxo-3-phenyl-3,4-dihydrophthalazin-1-yl)phenyl)methanimine oxide | 121 |
| 15 | (Z)-1-(3-(3-(4-fluorophenyl)-4-oxo-3,4-dihydrophthalazin-1-yl)phenyl)-N-methylmethanimine oxide | 231 |
| 16 | (Z)-N-ethyl-1-(3-(3-(4-fluorophenyl)-4-oxo-3,4-dihydrophthalazin-1-yl)phenyl)methanimine oxide | 221 |
| 17 | (Z)-1-(3-(3-(4-fluorophenyl)-4-oxo-3,4-dihydrophthalazin-1-yl)phenyl)-N-isopropylmethanimine oxide | 207 |
| 18 | N-(2-chloro-5-(4-oxo-3-phenyl-3,4-dihydrophthalazin-1-yl)phenyl)-N-(methylsulfonyl)methanesulfonamide | 114 |
| 19 | N-(3-(3-(phenyl)-4-oxo-3,4-dihydrophthalazin-1-yl)-6-chlorophenyl)-dimethylsulfamoylamide | 120 |
| 20 | methyl (N-(3-(3-(4-fluorophenyl)-4-oxo-3,4-dihydrophthalazin-1-yl)phenyl)sulfamoyl)carbamate | 211 |
| 21 | (Z)-1-(3-(3-(2,4-difluorophenyl)-4-oxo-3,4-dihydrophthalazin-1-yl)phenyl)-N-methylmethanimine oxide | 167 |
| 22 | (Z)-1-(3-(3-(2,4-difluorophenyl)-4-oxo-3,4-dihydrophthalazin-1-yl)phenyl)-N-isopropylmethanimine oxide | 201 |
| 23 | (Z)-N-tert-butyl-1-(3-(3-(2,4-difluorophenyl)-4-oxo-3,4-dihydrophthalazin-1-yl)phenyl)methanimine oxide | 192 |
| 24 | 4-(3-aminophenyl)-2-(2,4-difluorophenyl)phthalazin-1(2H)-one | 147 |
| 25 | N-(3-(3-(2,4-difluorophenyl)-4-oxo-3,4-dihydrophthalazin-1-yl)phenyl)ethanesulfonamide | 147 |
| 26 | methyl (N-(3-(3-(2,4-difluorophenyl)-4-oxo-3,4-dihydrophthalazin-1-yl)phenyl)sulfamoyl)carbamate | 180 |
| 27 | (Z)-1-(3-(3-(3-fluorophenyl)-4-oxo-3,4-dihydrophthalazin-1-yl)phenyl)-N-methylmethanimine oxide | 148 |
| 28 | (Z)-1-(3-(3-(3-fluorophenyl)-4-oxo-3,4-dihydrophthalazin-1-yl)phenyl)-N-isopropylmethanimine oxide | 154 |
| 29 | (Z)-N-tert-butyl-1-(3-(3-(3-fluorophenyl)-4-oxo-3,4-dihydrophthalazin-1-yl)phenyl)methanimine oxide | 162 |
| 30 | N-(3-(3-(3-fluorophenyl)-4-oxo-3,4-dihydrophthalazin-1-yl)phenyl)ethanesulfonamide | 117 |
| 31 | (Z)-1-(3-(3-(3,5-difluorophenyl)-4-oxo-3,4-dihydrophthalazin-1-yl)phenyl)-N-isopropylmethanimine oxide | 171 |
| 32 | (Z)-N-isopropyl-1-(3-(3-(4-methoxyphenyl)-4-oxo-3,4-dihydrophthalazin-1-yl)phenyl)methanimine oxide | 124 |
| 33 | (Z)-1-(3-(3-(3-chloro-4-fluorophenyl)-4-oxo-3,4-dihydrophthalazin-1-yl)phenyl)-N-isopropylmethanimine oxide | 183 |
| 34 | (Z)-N-tert-butyl-1-(3-(3-(3-chloro-4-fluorophenyl)-4-oxo-3,4-dihydrophthalazin-1-yl)phenyl)methanimine oxide | 176 |
| 35 | (Z)-N-benzyll-1-(3-(4-oxo-3-phenyl-3,4-dihydrophthalazin-1-yl)phenyl)methanimine oxide | 113 |
| 36 | (E)-1-(4-(3-(4-fluorophenyl)-4-oxo-3,4-dihydrophthalazin-1-yl)phenyl)-N-methylmethanimine oxide | 142 |
| 37 | (Z)-1-(3-(3-(4-fluorophenyl)-4-oxo-3,4-dihydrophthalazin-1-yl)phenyl)-N-isopropylmethanimine oxide | 126 |
| 38 | (E)-1-(3-fluoro-4-(3-(4-fluorophenyl)-4-oxo-3,4-dihydrophthalazin-1-yl)phenyl)-N-methylmethanimine oxide | 143 |
| 39 | (E)-1-(3-fluoro-4-(3-(4-fluorophenyl)-4-oxo-3,4-dihydrophthalazin-1-yl)phenyl)-N-isopropylmethanimine oxide | 106 |
| 40 | (Z)-1-(2-(3-(4-fluorophenyl)-4-oxo-3,4-dihydrophthalazin-1-yl)phenyl)-N-methylmethanimine oxide | 160 |
| 41 | (Z)-1-(2-(3-(4-fluorophenyl)-4-oxo-3,4-dihydrophthalazin-1-yl)phenyl)-N-isopropylmethanimine oxide | 129 |
| 42 | (Z)-N-tert-butyl-1-(2-(3-(4-fluorophenyl)-4-oxo-3,4-dihydrophthalazin-1-yl)phenyl)methanimine oxide | 161 |
| 43 | (Z)-N-tert-butyl-1-(3-(3-(4-fluorophenyl)-4-oxo-3,4-dihydrophthalazin-1-yl)phenyl)methanimine oxide | 205 |
| 44 | (Z)-N-benzyl-1-(3-(3-(4-fluorophenyl)-4-oxo-3,4-dihydrophthalazin-1-yl)phenyl)methanimine oxide | 142 |
| 45 | (Z)-1-(5-(3-(4-fluorophenyl)-4-oxo-3,4-dihydrophthalazin-1-yl)thiophen-2-yl)-N-methylmethanimine oxide | 195 |
| 46 | (Z)-1-(5-(3-(4-fluorophenyl)-4-oxo-3,4-dihydrophthalazin-1-yl)thiophen-2-yl)-N-isopropylmethanimine oxide | 154 |
| 47 | (Z)-N-tert-butyl-1-(5-(3-(4-fluorophenyl)-4-oxo-3,4-dihydrophthalazin-1-yl)thiophen-2-yl)methanimine oxide | 132 |
| 48 | (Z)-1-(5-(3-(4-fluorophenyl)-4-oxo-3,4-dihydrophthalazin-1-yl)furan-2-yl)-N-isopropylmethanimine oxide | 107 |
| 49 | (Z)-N-tert-butyl-1-(5-(3-(4-fluorophenyl)-4-oxo-3,4-dihydrophthalazin-1-yl)-2-methoxyphenyl)methanimine oxide | 107 |
| 50 | (Z)-N-benzyl-1-(5-(3-(4-fluorophenyl)-4-oxo-3,4-dihydrophthalazin-1-yl)-2-methoxyphenyl)methanimine oxide | 105 |
| 51 | (Z)-1-(2-fluoro-5-(3-(4-fluorophenyl)-4-oxo-3,4-dihydrophthalazin-1-yl)phenyl)-N-methylmethanimine oxide | 139 |
| 52 | (Z)-1-(2-fluoro-5-(3-(4-fluorophenyl)-4-oxo-3,4-dihydrophthalazin-1-yl)phenyl)-N-isopropylmethanimine oxide | 172 |
| 53 | (Z)-N-tert-butyl-1-(2-fluoro-5-(3-(4-fluorophenyl)-4-oxo-3,4-dihydrophthalazin-1-yl)phenyl)methanimine oxide | 172 |
| 54 | (Z)-N-benzyl-1-(2-fluoro-5-(3-(4-fluorophenyl)-4-oxo-3,4-dihydrophthalazin-1-yl)phenyl)methanimine oxide | 161 |
| 55 | (Z)-1-(4-fluoro-3-(3-(4-fluorophenyl)-4-oxo-3,4-dihydrophthalazin-1-yl)phenyl)-N-methylmethanimine oxide | 151 |
| 56 | (Z)-1-(4-fluoro-3-(3-(4-fluorophenyl)-4-oxo-3,4-dihydrophthalazin-1-yl)phenyl)-N-isopropylmethanimine oxide | 115 |
| 57 | (Z)-N-tert-butyl-1-(4-fluoro-3-(3-(4-fluorophenyl)-4-oxo-3,4-dihydrophthalazin-1-yl)phenyl)methanimine oxide | 149 |
| 58 | (Z)-N-benzyl-1-(4-fluoro-3-(3-(4-fluorophenyl)-4-oxo-3,4-dihydrophthalazin-1-yl)phenyl)methanimine oxide | 131 |
| 59 | 4-(4-chloro-3-nitrophenyl)-2-(4-fluorophenyl)phthalazin-1(2H)-one | 109 |
| 60 | 4-(3-aminophenyl)-2-(4-fluorophenyl)phthalazin-1(2H)-one | 176 |
| 61 | N-(3-(3-benzyl-4-oxo-3,4-dihydrophthalazin-1-yl)phenyl)ethanesulfonamide | 104 |
| 62 | N-(3-(4-oxo-3-phenethyl-3,4-dihydrophthalazin-1-yl)phenyl)ethanesulfonamide | 111 |
| 63 | N-(3-(3-(4-fluorobenzyl)-4-oxo-3,4-dihydrophthalazin-1-yl)phenyl)ethanesulfonamide | 103 |
| 64 | N-(3-(3-([1,1'-biphenyl]-4-yl)-4-oxo-3,4-dihydrophthalazin-1-yl)phenyl)ethanesulfonamide | 106 |
| 65 | N-(3-(3-([1,1'-biphenyl]-4-yl)-4-oxo-3,4-dihydrophthalazin-1-yl)phenyl)-N-methylethanesulfonamide | 96 |
| 66 | methyl 3-(3-(4-fluorophenyl)-4-oxo-3,4-dihydrophthalazin-1-yl)benzoate | 103 |
| 67 | N-(3-(3-(4-fluorophenyl)-4-oxo-3,4-dihydrophthalazin-1-yl)phenyl)propan-2-sulfonamide | 166 |
| 68 | N-(3-(3-(4-fluorophenyl)-4-oxo-3,4-dihydrophthalazin-1-yl)phenyl)propan-1-sulfonamide | 160 |
| 69 | N-(3-(3-(4-fluorophenyl)-4-oxo-3,4-dihydrophthalazin-1-yl)phenyl)-N-methylmethanesulfonamide | 209 |
| 70 | N-(3-(3-(4-fluorophenyl)-4-oxo-3,4-dihydrophthalazin-1-yl)phenyl)-N-methylethanesulfonamide | 135 |
| 71 | N-(3-(3-(4-fluorophenyl)-4-oxo-3,4-dihydrophthalazin-1-yl)phenyl)cyclopropanesulfonamide | 150 |
| 72 | N-(3-(3-(4-fluorophenyl)-4-oxo-3,4-dihydrophthalazin-1-yl)phenyl)-N-methylpropan-1-sulfonamide | 115 |
| 73 | N-(3-(3-(4-fluorophenyl)-4-oxo-3,4-dihydrophthalazin-1-yl)phenyl)-N-methylpropan-2-sulfonamide | 124 |
| 74 | N-(3-(3-(4-fluorophenyl)-4-oxo-3,4-dihydrophthalazin-1-yl)phenyl)-N-methylcyclopropanesulfonamide | 128 |
| 75 | N-(3-(3-(4-fluorophenyl)-4-oxo-3,4-dihydrophthalazin-1-yl)phenyl)-N-methyl-dimethylsulfamoylamide | 139 |
| 76 | methyl (N-(3-(3-(4-fluorophenyl)-4-oxo-3,4-dihydrophthalazin-1-yl)phenyl)-N-methylsulfamoyl) (methyl) carbamate | 125 |
| 77 | 1,1,1-trifluoro-N-(3-(3-(4-fluorophenyl)-4-oxo-3,4-dihydrophthalazin-1-yl)phenyl)methanesulfonamide | 194 |
| 78 | 1,1,1-trifluoro-N-(3-(3-(4-fluorophenyl)-4-oxo-3,4-dihydrophthalazin-1-yl)phenyl)-N-methylmethanesulfonamide | 122 |
| 79 | 3-(3-(4-fluorophenyl)-4-oxo-3,4-dihydrophthalazin-1-yl)-N-((tetrahydro-2H-pyran-2-yl)oxy)benzamide | 167 |
| 80 | N-(3-(3-(4-fluorophenyl)-4-oxo-3,4-dihydrophthalazin-1-yl)phenyl)-N-methyl-methylsulfamoylamide | 216 |
| 81 | 4-(3-(1H-tetrazol-5-yl)phenyl)-2-(4-fluorophenyl)phthalazin-1(2H)-one | 115 |
| 82 | N-(5-(3-(4-fluorophenyl)-4-oxo-3,4-dihydrophthalazin-1-yl)pyridin-3-yl)ethanesulfonamide | 111 |
| 83 | 3-(3-(4-fluorophenyl)-4-oxo-3,4-dihydrophthalazin-1-yl)-N-hydroxybenzamide | 162 |
| 84 | (Z)-1-(3-(3-([1,1'-biphenyl]-4-yl)-4-oxo-3,4-dihydrophthalazin-1-yl)phenyl)-N-methylmethanimine oxide | 123 |
| 85 | (Z)-1-(3-(3-([1,1'-biphenyl]-4-yl)-4-oxo-3,4-dihydrophthalazin-1-yl)phenyl)-N-isopropylmethanimine oxide | 118 |
| 86 | 3-(3-(4-fluorophenyl)-4-oxo-3,4-dihydrophthalazin-1-yl)-N-methylbenzenesulfonamide | 196 |
| 87 | N-ethyl-3-(3-(4-fluorophenyl)-4-oxo-3,4-dihydrophthalazin-1-yl)benzenesulfonamide | 226 |
| 88 | 3-(3-(4-fluorophenyl)-4-oxo-3,4-dihydrophthalazin-1-yl)-N,N-dimethylbenzenesulfonamide | 180 |
| 89 | 2-(2,4-difluorophenyl)-4-(3-(methylsulfonyl)phenyl)phthalazin-1(2H)-one | 339 |
| 90 | 2-(2,4-difluorophenyl)-4-(3-(ethylsulfonyl)phenyl)phthalazin-1(2H)-one | 283 |
| 91 | 3-(3-(2,4-difluorophenyl)-4-oxo-3,4-dihydrophthalazin-1-yl)-N-methylbenzenesulfonamide | 272 |
| 92 | 3-(3-(2,4-difluorophenyl)-4-oxo-3,4-dihydrophthalazin-1-yl)-N-ethylbenzenesulfonamide | 208 |
| 93 | 3-(3-(2,4-difluorophenyl)-4-oxo-3,4-dihydrophthalazin-1-yl)-N,N-dimethylbenzenesulfonamide | 227 |
| 94 | (Z)-1-(3-(3-(2-fluorophenyl)-4-oxo-3,4-dihydrophthalazin-1-yl)phenyl)-N-methylmethanimine oxide | 272 |
| 95 | (Z)-1-(3-(3-(2-fluorophenyl)-4-oxo-3,4-dihydrophthalazin-1-yl)phenyl)-N-isopropylmethanimine oxide | 133 |
| 96 | (Z)-N-tert-butyl-1-(3-(3-(2-fluorophenyl)-4-oxo-3,4-dihydrophthalazin-1-yl)phenyl)methanimine oxide | 176 |
| 97 | 2-(2-fluorophenyl)-4-(3-(methylsulfonyl)phenyl)phthalazin-1(2H)-one | 286 |
| 98 | 4-(3-(ethylsulfonyl)phenyl)-2-(2-fluorophenyl)phthalazin-1(2H)-one | 264 |
| 99 | 3-(3-(2-fluorophenyl)-4-oxo-3,4-dihydrophthalazin-1-yl)-N-methylbenzenesulfonamide | 259 |
| 100 | N-ethyl-3-(3-(2-fluorophenyl)-4-oxo-3,4-dihydrophthalazin-1-yl)benzenesulfonamide | 196 |
| 101 | 3-(3-(2-fluorophenyl)-4-oxo-3,4-dihydrophthalazin-1-yl)-N,N-dimethylbenzenesulfonamide | 153 |
| 102 | (Z)-1-(3-(3-(2,6-difluorophenyl)-4-oxo-3,4-dihydrophthalazin-1-yl)phenyl)-N-methylmethanimine oxide | 206 |
| 103 | (Z)-1-(3-(3-(2,6-difluorophenyl)-4-oxo-3,4-dihydrophthalazin-1-yl)phenyl)-N-isopropylmethanimine oxide | 324 |
| 104 | (Z)-N-tert-butyl-1-(3-(3-(2,6-difluorophenyl)-4-oxo-3,4-dihydrophthalazin-1-yl)phenyl)methanimine oxide | 284 |
| 105 | 2-(2,6-difluorophenyl)-4-(3-(methylsulfonyl)phenyl)phthalazin-1(2H)-one | 226 |
| 106 | 2-(2,6-difluorophenyl)-4-(3-(ethylsulfonyl)phenyl)phthalazin-1(2H)-one | 141 |
| 107 | 3-(3-(2,6-difluorophenyl)-4-oxo-3,4-dihydrophthalazin-1-yl)-N-methylbenzenesulfonamide | 233 |
| 108 | 3-(3-(2,6-difluorophenyl)-4-oxo-3,4-dihydrophthalazin-1-yl)-N-ethylbenzenesulfonamide | 209 |
| 109 | 3-(3-(2,6-difluorophenyl)-4-oxo-3,4-dihydrophthalazin-1-yl)-N,N-dimethylbenzenesulfonamide | 188 |
| 110 | 6-fluoro-4-(3-(methylsulfonyl)phenyl)-2-phenylphthalazin-1(2H)-one | 129 |
| 111 | 3-(7-fluoro-4-oxo-3-phenyl-3,4-dihydrophthalazin-1-yl)-N-methylbenzenesulfonamide | 126 |
| 112 | 7-fluoro-4-(3-(methylsulfonyl)phenyl)-2-phenylphthalazin-1(2H)-one | 103 |
| 113 | 3-(6-fluoro-4-oxo-3-phenyl-3,4-dihydrophthalazin-1-yl)-N-methylbenzenesulfonamide | 116 |
| 114 | 3-(3-(4-fluorophenyl)-4-oxo-3,4-dihydrophthalazin-1-yl)pyridine 1-oxide | 226 |
| 115 | 2-(2,4-difluorophenyl)-4-(pyridin-3-yl)phthalazin-1(2H)-one | 405 |
| 116 | 2-(2,6-difluorophenyl)-4-(pyridin-3-yl)phthalazin-1(2H)-one | 227 |
| 117 | 3-(3-(2,4-difluorophenyl)-4-oxo-3,4-dihydrophthalazin-1-yl)pyridine 1-oxide | 318 |
| 118 | 3-(3-(2,6-difluorophenyl)-4-oxo-3,4-dihydrophthalazin-1-yl)pyridine 1-oxide | 231 |
| 119 | 2-(2,4-difluorophenyl)-6-fluoro-4-(3-(methylsulfonyl)phenyl)phthalazin-1(2H)-one | 166 |
| 120 | 2-(2,4-difluorophenyl)-7-fluoro-4-(3-(methylsulfonyl)phenyl)phthalazin-1(2H)-one | 178 |
| 121 | 3-(3-(2,4-difluorophenyl)-7-fluoro-4-oxo-3,4-dihydrophthalazin-1-yl)-N-methylbenzenesulfonamide | 157 |
| 122 | 3-(3-(2,4-difluorophenyl)-6-fluoro-4-oxo-3,4-dihydrophthalazin-1-yl)-N-methylbenzenesulfonamide | 177 |
| 123 | 2-(2,4-difluorophenyl)-4-(3-(ethylsulfonyl)phenyl)-6-fluorophthalazin-1(2H)-one | 253 |
| 124 | 3-(3-(2,4-difluorophenyl)-7-fluoro-4-oxo-3,4-dihydrophthalazin-1-yl)-N-ethylbenzenesulfonamide | 146 |
| 125 | 3-(3-(2,4-difluorophenyl)-7-fluoro-4-oxo-3,4-dihydrophthalazin-1-yl)-N,N-dimethylbenzenesulfonamide | 172 |
| 126 | 3-(3-(2,4-difluorophenyl)-4-oxo-3,4-dihydrophthalazin-1-yl)benzenesulfonamide | 185 |
| 127 | 3-(3-(2,4-difluorophenyl)-4-oxo-3,4-dihydrophthalazin-1-yl)-N-(2-hydroxyethyl)benzenesulfonamide | 321 |
| 128 | N-(3-(3-cyclohexyl-4-oxo-3,4-dihydrophthalazin-1-yl)phenyl)ethanesulfonamide | 177.53 |
| 129 | N-(3-(3-(1-methylpiperidin-4-yl)-4-oxo-3,4-dihydrophthalazin-1-yl)phenyl)ethanesulfonamide | 104.16 |
| 130 | 3-(3-(4-fluorophenyl)-4-oxo-3,4-dihydrophthalazin-1-yl)benzonitrile | 120.16 |
| 131 | (E)-3-(3-(4-fluorophenyl)-4-oxo-3,4-dihydrophthalazin-1-yl)-N'-hydroxybenzimideamide | 102.08 |
| 132 | N-(3-(3-isopropyl-4-oxo-3,4-dihydrophthalazin-1-yl)phenyl)ethanesulfonamide | 107.58 |
| 133 | N-(3-(3-(1-methyl-1H-pyrazol-4-yl)-4-oxo-3,4-dihydrophthalazin-1-yl)phenyl)ethanesulfonamide | 118.8 |
| 134 | 4-(3-aminopiperidin-1-yl)-2-(4-fluorophenyl)phthalazin-1(2H)-one·hydrochloride | 167.53 |
| 135 | N-(1-(3-(4-fluorophenyl)-4-oxo-3,4-dihydrophthalazin-1-yl)piperidin-3-yl)ethanesulfonamide | 174.93 |
| 136 | N-(3-(3-(oxetan-3-yl)-4-oxo-3,4-dihydrophthalazin-1-yl)phenyl)ethanesulfonamide | 101.27 |
| 137 | N-(1-(3-(4-fluorophenyl)-4-oxo-3,4-dihydrophthalazin-1-yl)pyrrolidin3-yl)ethanesulfonamide | 112.58 |
| 138 | (S)-N-(1-(3-(4-fluorophenyl)-4-oxo-3,4-dihydrophthalazin-1-yl)piperidin-3-yl)ethanesulfonamide | 175.98 |
| 139 | (R)-N-(1-(3-(4-fluorophenyl)-4-oxo-3,4-dihydrophthalazin-1-yl)piperidin-3-yl)ethanesulfonamide | 130.07 |
| 140 | N-(3-(3-(4,4-difluorocyclohexyl)-4-oxo-3,4-dihydrophthalazin-1-yl)phenyl)ethanesulfonamide | 134.41 |
| 141 | N-(1-(3-(4-fluorophenyl)-4-oxo-3,4-dihydrophthalazin-1-yl)azepan-3-yl)ethanesulfonamide | 212.55 |
| 142 | 3-(3-cyclohexyl-4-oxo-3,4-dihydrophthalazin-1-yl)benzenesulfonamide | 162.3 |
| 143 | 4-(3-aminoazepan-1-yl)-2-cyclohexylphthalazin-1(2H)-one·hydrochloride | 238.3 |
| 144 | N-(1-(3-cyclohexyl-4-oxo-3,4-dihydrophthalazin-1-yl)azepan-3-yl)ethanesulfonamide | 164.1 |
| 145 | N-(3-(3-(4-methylcyclohexyl)-4-oxo-3,4-dihydrophthalazin-1-yl)phenyl)ethanesulfonamide | 136.6 |
| 146 | (S)-4-(3-aminopiperidin-1-yl)-2-cyclohexylphthalazin-1(2H)-one·hydrochloride | 135.2 |
| 147 | (S)-N-(1-(3-cyclohexyl-4-oxo-3,4-dihydrophthalazin-1-yl)piperidin-3-yl)ethanesulfonamide | 127.7 |
| 148 | 4-(3-aminoazepan-1-yl)-2-(4-fluorophenyl)phthalazin-1(2H)-one·hydrochloride | 184.3 |
| 149 | N-(3-(2-(4-fluorophenyl)-1-oxo-1,2-dihydropyrido[3,4-d]pyridin-4-yl)phenyl)ethanesulfonamide | 114.9 |
| 150 | N-(3-(3-(4-fluorophenyl)-8-methyl-4-oxo-3,4-dihydrophthalazin-1-yl)phenyl)ethanesulfonamide | 104.7 |
| 151 | N-(3-(4-oxo-3-(tetrahydro-2H-pyran-4-yl)-3,4-dihydrophthalazin-1-yl)phenyl)ethanesulfonamide | 145.64 |
| 152 | 2-(3-(3-(4-fluorophenyl)-4-oxo-3,4-dihydrophthalazin-1-yl)phenyl)-2-methylpropanoic acid | 195.5 |
| 153 | 2-(3-(3-(4-fluorophenyl)-4-oxo-3,4-dihydrophthalazin-1-yl)phenyl)-N,2-dimethylpropanamide | 280.4 |
| 154 | 2-cyclohexyl-4-(3-(methylsulfonyl)phenyl)phthalazin-1(2H)-one | 263.7 |
| 155 | (S)-4-(3-aminoazepan-1-yl)-2-cyclohexylphthalazin-1(2H)-one·hydrochloride | 230.9 |
| 156 | (S)-2-cyclohexyl-4-(3-(methylamino)azepan-1-yl)phthalazin-1(2H)-one·hydrochloride | 223.9 |
| 157 | (S)-4-(3-aminoazepan-1-yl)-2-(2,4-difluorophenyl)phthalazin-1(2H)-one·hydrochloride | 244.1 |
| 158 | (S)-4-(3-aminoazepan-1-yl)-2-(4-fluorophenyl)phthalazin-1(2H)-one·hydrochloride | 164.7 |
| 159 | (R)-4-(3-aminoazepan-1-yl)-2-cyclohexylphthalazin-1(2H)-one·hydrochloride | 398.8 |
| 160 | 2-(3-(3-cyclohexyl-4-oxo-3,4-dihydrophthalazin-1-yl)phenyl)-2-methylpropanoic acid | 289.8 |
| 161 | 3-(3-(3-(4-fluorophenyl)-4-oxo-3,4-dihydrophthalazin-1-yl)phenyl)oxazolidin-2-one | 177 |
| 162 | (R)-4-(3-aminoazepan-1-yl)-2-(4-fluorophenyl)phthalazin-1(2H)-one·hydrochloride | 205.5 |
| 163 | 2-(3-(3-cyclohexyl-4-oxo-3,4-dihydrophthalazin-1-yl)phenyl)-2-methylpropanamide | 277 |
| 164 | 2-(3-(3-cyclohexyl-4-oxo-3,4-dihydrophthalazin-1-yl)phenyl)-N,2-dimethylpropanamide | 285.5 |
| 165 | (R)-4-(3-aminoazepan-1-yl)-2-(2,4-difluorophenyl)phthalazin-1(2H)-one·hydrochloride | 277.1 |
| 166 | 2-(3-(3-(2,4-difluorophenyl)-4-oxo-3,4-dihydrophthalazin-1-yl)phenyl)-2-methylpropanoic acid | 239.2 |
| 167 | 4-(3-aminoazepan-1-yl)-2-(2,4-difluorophenyl)phthalazin-1(2H)-one·hydrochloride | 249.4 |
| 168 | 2-(3-(3-(2,4-difluorophenyl)-4-oxo-3,4-dihydrophthalazin-1-yl)phenyl)-2-methylpropanenitrile | 155.7 |
| 169 | 4-(3-(2-(1H-tetrazol-5-yl)propan-2-yl)phenyl)-2-(2,4-difluorophenyl)phthalazin-1(2H)-one | 211.3 |
| 170 | (E)-2-(3-(3-(2,4-difluorophenyl)-4-oxo-3,4-dihydrophthalazin-1-yl)phenyl)-N'-hydroxy-2-methylpropanimideamide | 262.7 |
| 171 | 2-(3-(3-(2-fluorophenyl)-4-oxo-3,4-dihydrophthalazin-1-yl)phenyl)-2-methylpropanoic acid | 134.7 |
| 172 | 4-(3-aminoazepan-1-yl)-2-cyclobutylphthalazin-1(2H)-one·hydrochloride | 99.5 |
| 173 | 4-(3-aminoazepan-1-yl)-2-cyclopentylphthalazin-1(2H)-one·hydrochloride | 112.2 |
| 174 | 2-(2,4-difluorophenyl)-4-(3-(2-(5-methyl-1,2,4-oxadiazol-3-yl)propan-2-yl)phenyl)phthalazin-1(2H)-one | 114.2 |
| 175 | (R)-4-(3-aminoazepan-1-yl)-2-(2-fluorophenyl)phthalazin-1(2H)-one·hydrochloride | 155.3 |
| 176 | 2-(3-(3-(2,4-difluorophenyl)-4-oxo-3,4-dihydrophthalazin-1-yl)phenyl)-N-isopropyl-2-methylpropanamide | 244.30 |
| 177 | 2-(2,4-difluorophenyl)-4-(3-(2-methyl-1-oxo-1-(pyrrolidin1-yl)propan-2-yl)phenyl)phthalazin-1(2H)-one | 241.6 |
| 178 | N-cyclopropyl-2-(3-(3-(2,4-difluorophenyl)-4-oxo-3,4-dihydrophthalazin-1-yl)phenyl)-2-methylpropanamide | 331.00 |
| 179 | 2-(2,4-difluorophenyl)-4-(3-(2-methyl-1-morpholino-1-oxopropan-2-yl)phenyl)phthalazin-1(2H)-one | 221.60 |
| 180 | N-cyclohexyl-2-(3-(3-(2,4-difluorophenyl)-4-oxo-3,4-dihydrophthalazin-1-yl)phenyl)-2-methylpropanamide | 133.30 |
| 181 | 2-(3-(3-(2,4-difluorophenyl)-4-oxo-3,4-dihydrophthalazin-1-yl)phenyl)-2-methyl-N-phenylpropanamide | 122.10 |
| 182 | 2-(3-(3-(2,4-difluorophenyl)-4-oxo-3,4-dihydrophthalazin-1-yl)phenyl)-2-methyl-N-(1-methyl-1H-pyrazol-4-yl)propanamide | 219.60 |
| 183 | 1-(3-(3-(2,4-difluorophenyl)-4-oxo-3,4-dihydrophthalazin-1-yl)phenyl)cyclopropan-1-carboxylic acid | 194.70 |
| 184 | 4-(1,4-diazepan-1-yl)-2-(2,4-difluorophenyl)phthalazin-1(2H)-one hydrochloride | 176.80 |
| 185 | (R)-4-(3-aminoazepan-1-yl)-2-(4-chlorophenyl)phthalazin-1(2H)-one hydrochloride | 273.80 |
| 186 | 2-(3-(3-(2,4-difluorophenyl)-4-oxo-3,4-dihydrophthalazin-1-yl)phenyl)-N-(2-hydroxyethyl)-2-methylpropanamide | 305.80 |
| 187 | (R)-N-(1-(3-(2,4-difluorophenyl)-4-oxo-3,4-dihydrophthalazin-1-yl)azepan-3-yl)ethanesulfonamide | 303.80 |
| 188 | 2-(3-(3-(2,4-difluorophenyl)-4-oxo-3,4-dihydrophthalazin-1-yl)phenyl)-2-methylpropanamide | 358.40 |
| 189 | (R)-2-(2,4-difluorophenyl)-4-(3-(methylamino)azepan-1-yl)phthalazin-1(2H)-one hydrochloride | 273.70 |
| 190 | 2-(2,4-difluorophenyl)-4-(3-hydroxyazepan-1-yl)phthalazin-1(2H)-one | 332.60 |
| 191 | (R)-2-(2,4-difluorophenyl)-4-(3-((2-hydroxyethyl)amino)azepan-1-yl)phthalazin-1(2H)-one hydrochloride | 312.70 |
| 192 | (R)-N-(1-(3-(2,4-difluorophenyl)-4-oxo-3,4-dihydrophthalazin-1-yl)azepan-3-yl)acetamide | 286.60 |
| 193 | 2-(2,4-difluorophenyl)-4-(3-oxoazepan-1-yl)phthalazin-1(2H)-one | 206.10 |
| 194 | 2-(3-(3-(4-chlorophenyl)-4-oxo-3,4-dihydrophthalazin-1-yl)phenyl)-2-methylpropanoic acid | 350.20 |
| 195 | 1-(3-(3-(4-chlorophenyl)-4-oxo-3,4-dihydrophthalazin-1-yl)phenyl)cyclopropan-1-carboxylic acid | 287.30 |
| 196 | 2-(4-chlorophenyl)-4-(3-hydroxyazepan-1-yl)phthalazin-1(2H)-one | 135.10 |
| 197 | 2-(3-(3-(4-chlorophenyl)-4-oxo-3,4-dihydrophthalazin-1-yl)phenyl)-2-methylpropanamide | 153.60 |
| 198 | 1-(3-(3-(4-chlorophenyl)-4-oxo-3,4-dihydrophthalazin-1-yl)phenyl)cyclopropan-1-carboxyamide | 225.40 |
| 199 | 2-(3-(3-(2,4-difluorophenyl)-4-oxo-3,4-dihydrophthalazin-1-yl)phenyl)acetic acid | 149.41 |
| 200 | 4-(3-(2-aminopropan-2-yl)phenyl)-2-(2,4-difluorophenyl)phthalazin-1(2H)-one hydrochloride | 255.33 |
| 201 | 2-(3-(3-(4-chloro-2-fluorophenyl)-4-oxo-3,4-dihydrophthalazin-1-yl)phenyl)-2-methylpropanoic acid | 204.58 |
| 202 | (R)-4-(3-aminoazepan-1-yl)-2-(4-chloro-2-fluorophenyl)phthalazin-1-(2H)-one hydrochloride | 261.73 |
| 203 | 1-(3-(4-chloro-2-fluorophenyl)-4-oxo-3,4-dihydrophthalazin-1-yl)azepan-3-carbonitrile | 109.84 |
| 204 | 4-(3-(1-amino-2-methylpropan-2-yl)phenyl)-2-(2,4-difluorophenyl)phthalazin-1(2H)-one hydrochloride | 161.50 |
| 205 | 2-(1-(3-(2,4-difluorophenyl)-4-oxo-3,4-dihydrophthalazin-1-yl)piperidin-3-yl)-2-methylpropanoic acid | 105.70 |
| 206 | 2-(3-(3-(4-cyclopropylphenyl)-4-oxo-3,4-dihydrophthalazin-1-yl)phenyl)-2-methylpropanoic acid | 182.70 |
| 207 | 1-(3-(3-(2,4-difluorophenyl)-4-oxo-3,4-dihydrophthalazin-1-yl)phenyl)cyclobutane-1-carboxylic acid | 309.90 |
| 208 | 1-(3-(3-(2,4-difluorophenyl)-4-oxo-3,4-dihydrophthalazin-1-yl)phenyl)cyclopentane-1-carboxylic acid | 117.30 |
| 209 | 2-(1-(3-(4-chlorophenyl)-4-oxo-3,4-dihydrophthalazin-1-yl)piperidin-3-yl)-2-methylpropanoic acid | 169.10 |
| 210-1 | 2-(1-(3-(2,4-difluorophenyl)-4-oxo-3,4-dihydrophthalazin-1-yl)piperidin-3-yl)-2-methylpropanoic acid | 199.00 |
| 210-2 | 2-(1-(3-(2,4-difluorophenyl)-4-oxo-3,4-dihydrophthalazin-1-yl)piperidin-3-yl)-2-methylpropanoic acid | 100.50 |
| 211 | methyl 1-(1-(3-(2,4-difluorophenyl)-4-oxo-3,4-dihydrophthalazin-1-yl)piperidin-3-yl)cyclopropan-1-carboxylate | 170.00 |
| 212 | 1-(1-(3-(2,4-difluorophenyl)-4-oxo-3,4-dihydrophthalazin-1-yl)piperidin-3-yl)cyclopropan-1-carboxylic acid | 170.10 |
| 213-1 | 1-(1-(3-(2,4-difluorophenyl)-4-oxo-3,4-dihydrophthalazin-1-yl)piperidin-3-yl)cyclopropan-1-carboxylic acid | 168.10 |
| 213-2 | 1-(1-(3-(2,4-difluorophenyl)-4-oxo-3,4-dihydrophthalazin-1-yl)piperidin-3-yl)cyclopropan-1-carboxylic acid | 102.60 |
| 214 | 2-(2,4-difluorophenyl)-4-(3-(pyrrolidin1-ylsulfonyl)phenyl)phthalazin-1(2H)-one | 233.90 |
| 215 | 2-(2,4-difluorophenyl)-4-(1,2,3,6-tetrahydropyridin-4-yl)phthalazin-1(2H)-one hydrochloride | 226.60 |
| 216 | 3-(3-(2,4-difluorophenyl)-4-oxo-3,4-dihydrophthalazin-1-yl)-N-ethyl-N-methylbenzenesulfonamide | 184.80 |
| 217 | 3-(3-(2,4-difluorophenyl)-4-oxo-3,4-dihydrophthalazin-1-yl)-N,N-diethylbenzenesulfonamide | 185.10 |
| 218 | 2-(2,4-difluorophenyl)-4-(1-(ethylsulfonyl)-1,2,3,6-tetrahydropyridin-4-yl)phthalazin-1(2H)-one | 176.50 |
| 219 | 2-(4-(3-(2,4-difluorophenyl)-4-oxo-3,4-dihydrophthalazin-1-yl)piperazin-2-yl)-2-methylpropanoic acid | 114.60 |
| 220 | 2-(4-(3-(2,4-difluorophenyl)-4-oxo-3,4-dihydrophthalazin-1-yl)-1-methylpiperazin-2-yl)-2-methylpropanoic acid | 125.80 |
| 221 | 2-(4-(3-(2,4-difluorophenyl)-4-oxo-3,4-dihydrophthalazin-1-yl)morpholin-2-yl)-2-methylpropanoic acid | 125.50 |

## Claims

1. A compound or a racemate, a steroisomer or a pharmaceutically acceptable salt thereof, wherein the compound is selected from the group consisting of:
1) N-(3-(4-Oxo-3-phenyl-3,4-dihydrophthalazin-1-yl)phenyl)ethanesulfonamide;
2) 4-(3-Aminophenyl)-2-(4-(trifluoromethyl)phenyl)phthalazin-1(2H)-one;
3) N-(3-(4-Oxo-3-(4-(trifluoromethyl)phenyl)-3,4-dihydrophthalazin-1-yl)phenyl)ethanesulfonamide;
4) 4-(3-(Ethylamino)phenyl)-2-(4-(trifluoromethyl) phenyl)phthalazin-1(2H)-one;
5) N-Methyl-N-(3-(4-oxo-3-(4-(trifluoromethyl)phenyl)-3,4-dihydrophthalazin-1-yl)phenyl)ethanesulfonamide;
6) N-(3-(4-Oxo-3-phenyl-3,4-dihydrophthalazin-1-yl)phenyl)methanesulfonamide;
7) N-Methyl-N-(3-(4-oxo-3-phenyl-3,4-dihydrophthalazin-1-yl)phenyl)methanesulfonamide;
8) N-(4-(4-Oxo-3-phenyl-3,4-dihydrophthalazin-1-yl)phenyl)ethanesulfonamide;
9) N-(3-(3-Phenyl)-4-oxo-3,4-dihydrophthalazin-1-yl)phenyl)dimethylsulfamoylamide;
10) N-(3-(3-(4-Fluorophenyl)-4-oxo-3,4-dihydrophthalazin-1-yl)phenyl)methanesulfonamide;
11) N-(3-(3-(4-Fluorophenyl)-4-oxo-3,4-dihydrophthalazin-1-yl)phenyl)ethanesulfonamide;
12) N-(3-(3-(4-Fluorophenyl)-4-oxo-3,4-dihydrophthalazin-1-yl)phenyl)dimethylsulfamoylamide;
13) (Z)-N-Methyl-1-(3-(4-oxo-3-phenyl-3,4-dihydrophthalazin-1-yl)phenyl)methanimine oxide;
14) (Z)-N-Isopropyl-1-(3-(4-oxo-3-phenyl-3,4-dihydrophthalazin-1-yl)phenyl)methanimine oxide;
15) (Z)-1-(3-(3-(4-Fluorophenyl)-4-oxo-3,4-dihydrophthalazin-1-yl)phenyl)-N-methylmethanimine oxide;
16) (Z)-N-Ethyl-1-(3-(3-(4-fluorophenyl)-4-oxo-3,4-dihydrophthalazin-1-yl)phenyl)methanimine oxide;
17) (Z)-1-(3-(3-(4-Fluorophenyl)-4-oxo-3,4-dihydrophthalazin-1-yl)phenyl)-N-isopropylmethanimine oxide;
18) N-(2-Chloro-5-(4-oxo-3-phenyl-3,4-dihydrophthalazin-1-yl)phenyl)-N-(methylsulfonyl)methane sulfonamide;
19) N-(3-(3-(Phenyl)-4-oxo-3,4-dihydrophthalazin-1-yl)-6-chlorophenyl)dimethylsulfamoylamide;
20) Methyl (N-(3-(3-(4-fluorophenyl)-4-oxo-3,4-dihydrophthalazin-1-yl)phenyl)sulfamoyl)carbamate;
21) (Z)-1-(3-(3-(2,4-Difluorophenyl)-4-oxo-3,4-dihydrophthalazin-1-yl)phenyl)-N-methylmethanimine oxide;
22) (Z)-1-(3-(3-(2,4-Difluorophenyl)-4-oxo-3,4-dihydrophthalazin-1-yl)phenyl)-N-isopropylmethanimine oxide;
23) (Z)-N-tert-Butyl-1-(3-(3-(2,4-difluorophenyl)-4-oxo-3,4-dihydrophthalazin-1-yl)phenyl)methanimine oxide;
24) 4-(3-Aminophenyl)-2-(2,4-difluorophenyl)phthalazin-1(2H)-one;
25) N-(3-(3-(2,4-Difluorophenyl)-4-oxo-3,4-dihydrophthalazin-1-yl)phenyl)ethanesulfonamide;
26) Methyl (N-(3-(3-(2,4-difluorophenyl)-4-oxo-3,4-dihydrophthalazin-1-yl)phenyl)sulfamoyl)carbamate;
27) (Z)-1-(3-(3-(3-Fluorophenyl)-4-oxo-3,4-dihydrophthalazin-1-yl)phenyl)-N-methylmethanimine oxide;
28) (Z)-1-(3-(3-(3-Fluorophenyl)-4-oxo-3,4-dihydrophthalazin-1-yl)phenyl)-N-isopropylmethanimine oxide;
29) (Z)-N-tert-Butyl-1-(3-(3-(3-fluorophenyl)-4-oxo-3,4-dihydrophthalazin-1-yl)phenyl)methanimine oxide;
30) N-(3-(3-(3-Fluorophenyl)-4-oxo-3,4-dihydrophthalazin-1-yl)phenyl)ethanesulfonamide;
31) (Z)-1-(3-(3-(3,5-Difluorophenyl)-4-oxo-3,4-dihydrophthalazin-1-yl)phenyl)-N-isopropylmethanimine oxide;
32) (Z)-N-Isopropyl-1-(3-(3-(4-methoxyphenyl)-4-oxo-3,4-dihydrophthalazin-1-yl)phenyl)methanimine oxide;
33) (Z)-1-(3-(3-(3-Chloro-4-fluorophenyl)-4-oxo-3,4-dihydrophthalazin-1-yl)phenyl)-N-isopropylmethanimine oxide;
34) (Z)-N-tert-Butyl-1-(3-(3-(3-chloro-4-fluoro phenyl)-4-oxo-3,4-dihydrophthalazin-1-yl)phenyl)methanimine oxide;
35) (Z)-N-Benzyl-1-(3-(4-oxo-3-phenyl-3,4-dihydrophthalazin-1-yl)phenyl)methanimine oxide;
36) (E)-1-(4-(3-(4-Fluorophenyl)-4-oxo-3,4-dihydrophthalazin-1-yl)phenyl)-N-methylmethanimine oxide;
37) (Z)-1-(3-(3-(4-Fluorophenyl)-4-oxo-3,4-dihydrophthalazin-1-yl)phenyl)-N-isopropylmethanimine oxide;
38) (E)-1-(3-Fluoro-4-(3-(4-fluorophenyl)-4-oxo-3,4-dihydrophthalazin-1-yl)phenyl)-N-methylmethanimine oxide;
39) (E)-1-(3-Fluoro-4-(3-(4-fluorophenyl)-4-oxo-3,4-dihydrophthalazin-1-yl)phenyl)-N-isopropylmethanimine oxide;
40) (Z)-1-(2-(3-(4-Fluorophenyl)-4-oxo-3,4-dihydrophthalazin-1-yl)phenyl)-N-methylmethanimine oxide;
41) (Z)-1-(2-(3-(4-Fluorophenyl)-4-oxo-3,4-dihydrophthalazin-1-yl)phenyl)-N-isopropylmethanimine oxide;
42) (Z)-N-tert-Butyl-1-(2-(3-(4-fluorophenyl)-4-oxo-3,4-dihydrophthalazin-1-yl)phenyl)methanimine oxide;
43) (Z)-N-tert-Butyl-1-(3-(3-(4-fluorophenyl)-4-oxo-3,4-dihydrophthalazin-1-yl)phenyl)methanimine oxide;
44) (Z)-N-Benzyl-1-(3-(3-(4-fluorophenyl)-4-oxo-3,4-dihydrophthalazin-1-yl)phenyl)methanimine oxide;
45) (Z)-1-(5-(3-(4-Fluorophenyl)-4-oxo-3,4-dihydrophthalazin-1-yl)thiophen-2-yl)-N-methylmethanimine oxide;
46) (Z)-1-(5-(3-(4-Fluorophenyl)-4-oxo-3,4-dihydrophthalazin-1-yl)thiophen-2-yl)-N-isopropylmethanimine oxide;
47) (Z)-N-tert-Butyl-1-(5-(3-(4-fluorophenyl)-4-oxo-3,4-dihydrophthalazin-1-yl)thiophen-2-yl)methanimine oxide;
48) (Z)-1-(5-(3-(4-Fluorophenyl)-4-oxo-3,4-dihydrophthalazin-1-yl)furan-2-yl)-N-isopropylmethanimine oxide;
49) (Z)-N-tert-Butyl-1-(5-(3-(4-fluorophenyl)-4-oxo-3,4-dihydrophthalazin-1-yl)-2-methoxyphenyl)methanimine oxide;
50) (Z)-N-Benzyl-1-(5-(3-(4-fluorophenyl)-4-oxo-3,4-dihydrophthalazin-1-yl)-2-methoxyphenyl)methanimine oxide;
51) (Z)-1-(2-Fluoro-5-(3-(4-fluorophenyl)-4-oxo-3,4-dihydrophthalazin-1-yl)phenyl)-N-methylmethanimine oxide;
52) (Z)-1-(2-Fluoro-5-(3-(4-fluorophenyl)-4-oxo-3,4-dihydrophthalazin-1-yl)phenyl)-N-isopropylmethanimine oxide;
53) (Z)-N-tert-Butyl-1-(2-fluoro-5-(3-(4-fluoro phenyl)-4-oxo-3,4-dihydrophthalazin-1-yl)phenyl)methanimine oxide;
54) (Z)-N-Benzyl-1-(2-fluoro-5-(3-(4-fluorophenyl)-4-oxo-3,4-dihydrophthalazin-1-yl)phenyl)methanimine oxide;
55) (Z)-1-(4-Fluoro-3-(3-(4-fluorophenyl)-4-oxo-3,4-dihydrophthalazin-1-yl)phenyl)-N-methylmethanimine oxide;
56) (Z)-1-(4-Fluoro-3-(3-(4-fluorophenyl)-4-oxo-3,4-dihydrophthalazin-1-yl)phenyl)-N-isopropylmethanimine oxide;
57) (Z)-N-tert-Butyl-1-(4-fluoro-3-(3-(4-fluoro phenyl)-4-oxo-3,4-dihydrophthalazin-1-yl)phenyl)methanimine oxide;
58) (Z)-N-Benzyl-1-(4-fluoro-3-(3-(4-fluorophenyl)-4-oxo-3,4-dihydrophthalazin-1-yl)phenyl)methanimine oxide;
59) 4-(4-Chloro-3-nitrophenyl)-2-(4-fluorophenyl)phthalazin-1(2H)-one;
60) 4-(3-Aminophenyl)-2-(4-fluorophenyl)phthalazin-1(2H)-one;
61) N-(3-(3-Benzyl-4-oxo-3,4-dihydrophthalazin-1-yl)phenyl)ethanesulfonamide;
62) N-(3-(4-Oxo-3-phenethyl-3,4-dihydrophthalazin-1-yl)phenyl)ethanesulfonamide;
63) N-(3-(3-(4-Fluorobenzyl)-4-oxo-3,4-dihydrophthalazin-1-yl)phenyl)ethanesulfonamide;
64) N-(3-(3-([1,1'-Biphenyl]-4-yl)-4-oxo-3,4-dihydrophthalazin-1-yl)phenyl)ethanesulfonamide;
65) N-(3-(3-([1,1'-Biphenyl]-4-yl)-4-oxo-3,4-dihydrophthalazin-1-yl)phenyl)-N-methylethanesulfonamide;
66) Methyl 3-(3-(4-fluorophenyl)-4-oxo-3,4-dihydrophthalazin-1-yl)benzoate;
67) N-(3-(3-(4-Fluorophenyl)-4-oxo-3,4-dihydrophthalazin-1-yl)phenyl)propan-2-sulfonamide;
68) N-(3-(3-(4-Fluorophenyl)-4-oxo-3,4-dihydrophthalazin-1-yl)phenyl)propan-1-sulfonamide;
69) N-(3-(3-(4-Fluorophenyl)-4-oxo-3,4-dihydrophthalazin-1-yl)phenyl)-N-methylmethanesulfonamide;
70) N-(3-(3-(4-Fluorophenyl)-4-oxo-3,4-dihydrophthalazin-1-yl)phenyl)-N-methylethanesulfonamide;
71) N-(3-(3-(4-Fluorophenyl)-4-oxo-3,4-dihydrophthalazin-1-yl)phenyl)cyclopropanesulfonamide;
72) N-(3-(3-(4-Fluorophenyl)-4-oxo-3,4-dihydrophthalazin-1-yl)phenyl)-N-methylpropan-1-sulfonamide;
73) N-(3-(3-(4-Fluorophenyl)-4-oxo-3,4-dihydrophthalazin-1-yl)phenyl)-N-methylpropan-2-sulfonamide;
74) N-(3-(3-(4-Fluorophenyl)-4-oxo-3,4-dihydrophthalazin-1-yl)phenyl)-N-methylcyclopropanesulfonamide;
75) N-(3-(3-(4-Fluorophenyl)-4-oxo-3,4-dihydrophthalazin-1-yl)phenyl)-N-methyl-dimethylsulfamoylamide;
76) Methyl (N-(3-(3-(4-fluorophenyl)-4-oxo-3,4-dihydrophthalazin-1-yl)phenyl)-N-methylsulfamoyl) (methyl)carbamate;
77) 1,1,1-Trifluoro-N-(3-(3-(4-fluorophenyl)-4-oxo-3,4-dihydrophthalazin-1-yl)phenyl)methanesulfonamide;
78) 1,1,1-Trifluoro-N-(3-(3-(4-fluorophenyl)-4-oxo-3,4-dihydrophthalazin-1-yl)phenyl)-N-methylmethane sulfonamide;
79) 3-(3-(4-Fluorophenyl)-4-oxo-3,4-dihydrophthalazin-1-yl)-N-((tetrahydro-2H-pyran-2-yl)oxy)benzamide;
80) N-(3-(3-(4-Fluorophenyl)-4-oxo-3,4-dihydrophthalazin-1-yl)phenyl)-N-methyl-methylsulfamoylamide;
81) 4-(3-(1H-Tetrazol-5-yl)phenyl)-2-(4-fluoro phenyl)phthalazin-1(2H)-one;
82) N-(5-(3-(4-Fluorophenyl)-4-oxo-3,4-dihydrophthalazin-1-yl)pyridin-3-yl)ethanesulfonamide;
83) 3-(3-(4-Fluorophenyl)-4-oxo-3,4-dihydrophthalazin-1-yl)-N-hydroxybenzamide;
84) (Z)-1-(3-(3-([1,1'-Biphenyl]-4-yl)-4-oxo-3,4-dihydrophthalazin-1-yl)phenyl)-N-methylmethanimine oxide;
85) (Z)-1-(3-(3-([1,1'-Biphenyl]-4-yl)-4-oxo-3,4-dihydrophthalazin-1-yl)phenyl)-N-isopropylmethanimine oxide;
86) 3-(3-(4-Fluorophenyl)-4-oxo-3,4-dihydrophthalazin-1-yl)-N-methylbenzenesulfonamide;
87) N-Ethyl-3-(3-(4-fluorophenyl)-4-oxo-3,4-dihydrophthalazin-1-yl)benzenesulfonamide;
88) 3-(3-(4-Fluorophenyl)-4-oxo-3,4-dihydrophthalazin-1-yl)-N,N-dimethylbenzenesulfonamide;
89) 2-(2,4-Difluorophenyl)-4-(3-(methylsulfonyl)phenyl)phthalazin-1(2H)-one;
90) 2-(2,4-Difluorophenyl)-4-(3-(ethylsulfonyl)phenyl)phthalazin-1(2H)-one;
91) 3-(3-(2,4-Difluorophenyl)-4-oxo-3,4-dihydrophthalazin-1-yl)-N-methylbenzenesulfonamide;
92) 3-(3-(2,4-Difluorophenyl)-4-oxo-3,4-dihydrophthalazin-1-yl)-N-ethylbenzenesulfonamide;
93) 3-(3-(2,4-Difluorophenyl)-4-oxo-3,4-dihydrophthalazin-1-yl)-N,N-dimethylbenzenesulfonamide;
94) (Z)-1-(3-(3-(2-Fluorophenyl)-4-oxo-3,4-dihydrophthalazin-1-yl)phenyl)-N-methylmethanimine oxide;
95) (Z)-1-(3-(3-(2-Fluorophenyl)-4-oxo-3,4-dihydrophthalazin-1-yl)phenyl)-N-isopropylmethanimine oxide;
96) (Z)-N-tert-Butyl-1-(3-(3-(2-fluorophenyl)-4-oxo-3,4-dihydrophthalazin-1-yl)phenyl)methanimine oxide;
97) 2-(2-Fluorophenyl)-4-(3-(methylsulfonyl)phenyl)phthalazin-1(2H)-one;
98) 4-(3-(Ethylsulfonyl)phenyl)-2-(2-fluorophenyl)phthalazin-1(2H)-one;
99) 3-(3-(2-Fluorophenyl)-4-oxo-3,4-dihydrophthalazin-1-yl)-N-methylbenzenesulfonamide;
100) N-Ethyl-3-(3-(2-fluorophenyl)-4-oxo-3,4-dihydrophthalazin-1-yl)benzenesulfonamide;
101) 3-(3-(2-Fluorophenyl)-4-oxo-3,4-dihydrophthalazin-1-yl)-N,N-dimethylbenzenesulfonamide;
102) (Z)-1-(3-(3-(2,6-Difluorophenyl)-4-oxo-3,4-dihydrophthalazin-1-yl)phenyl)-N-methylmethanimine oxide;
103) (Z)-1-(3-(3-(2,6-Difluorophenyl)-4-oxo-3,4-dihydrophthalazin-1-yl)phenyl)-N-isopropylmethanimine oxide;
104) (Z)-N-tert-Butyl-1-(3-(3-(2,6-difluorophenyl)-4-oxo-3,4-dihydrophthalazin-1-yl)phenyl)methanimine oxide;
105) 2-(2,6-Difluorophenyl)-4-(3-(methylsulfonyl) phenyl)phthalazin-1(2H)-one;
106) 2-(2,6-Difluorophenyl)-4-(3-(ethylsulfonyl) phenyl)phthalazin-1(2H)-one;
107) 3-(3-(2,6-Difluorophenyl)-4-oxo-3,4-dihydrophthalazin-1-yl)-N-methylbenzenesulfonamide;
108) 3-(3-(2,6-Difluorophenyl)-4-oxo-3,4-dihydrophthalazin-1-yl)-N-ethylbenzenesulfonamide;
109) 3-(3-(2,6-Difluorophenyl)-4-oxo-3,4-dihydrophthalazin-1-yl)-N,N-dimethylbenzenesulfonamide;
110) 6-Fluoro-4-(3-(methylsulfonyl)phenyl)-2-phenyl phthalazin-1(2H)-one;
111) 3-(7-Fluoro-4-oxo-3-phenyl-3,4-dihydrophthalazin-1-yl)-N-methylbenzenesulfonamide;
112) 7-Fluoro-4-(3-(methylsulfonyl)phenyl)-2-phenyl phthalazin-1(2H)-one;
113) 3-(6-Fluoro-4-oxo-3-phenyl-3,4-dihydrophthalazin-1-yl)-N-methylbenzenesulfonamide;
114) 3-(3-(4-Fluorophenyl)-4-oxo-3,4-dihydrophthalazin-1-yl)pyridine 1-oxide;
115) 2-(2,4-Difluorophenyl)-4-(pyridin-3-yl)phthalazin-1(2H)-one;
116) 2-(2,6-Difluorophenyl)-4-(pyridin-3-yl)phthalazin-1(2H)-one;
117) 3-(3-(2,4-Difluorophenyl)-4-oxo-3,4-dihydrophthalazin-1-yl) pyridine 1-oxide;
118) 3-(3-(2,6-Difluorophenyl)-4-oxo-3,4-dihydrophthalazin-1-yl) pyridine 1-oxide;
119) 2-(2,4-Difluorophenyl)-6-fluoro-4-(3-(methyl sulfonyl)phenyl)phthalazin-1(2H)-one;
120) 2-(2,4-Difluorophenyl)-7-fluoro-4-(3-(methyl sulfonyl)phenyl)phthalazin-1(2H)-one;
121) 3-(3-(2,4-Difluorophenyl)-7-fluoro-4-oxo-3,4-dihydrophthalazin-1-yl)-N-methylbenzenesulfonamide;
122) 3-(3-(2,4-Difluorophenyl)-6-fluoro-4-oxo-3,4-dihydrophthalazin-1-yl)-N-methylbenzenesulfonamide;
123) 2-(2,4-Difluorophenyl)-4-(3-(ethylsulfonyl) phenyl)-6-fluorophthalazin-1(2H)-one;
124) 3-(3-(2,4-Difluorophenyl)-7-fluoro-4-oxo-3,4-dihydrophthalazin-1-yl)-N-ethylbenzenesulfonamide;
125) 3-(3-(2,4-Difluorophenyl)-7-fluoro-4-oxo-3,4-dihydrophthalazin-1-yl)-N,N-dimethylbenzenesulfonamide;
126) 3-(3-(2,4-Difluorophenyl)-4-oxo-3,4-dihydrophthalazin-1-yl)benzenesulfonamide;
127) 3-(3-(2,4-Difluorophenyl)-4-oxo-3,4-dihydrophthalazin-1-yl)-N-(2-hydroxyethyl)benzenesulfonamide;
128) N-(3-(3-Cyclohexyl-4-oxo-3,4-dihydrophthalazin-1-yl)phenyl)ethanesulfonamide;
129) N-(3-(3-(1-Methylpiperidin-4-yl)-4-oxo-3,4-dihydrophthalazin-1-yl)phenyl)ethanesulfonamide;
130) 3-(3-(4-Fluorophenyl)-4-oxo-3,4-dihydrophthalazin-1-yl)benzonitrile;
131) (E)-3-(3-(4-Fluorophenyl)-4-oxo-3,4-dihydrophthalazin-1-yl)-N'-hydroxybenzimidamide;
132) N-(3-(3-Isopropyl-4-oxo-3,4-dihydrophthalazin-1-yl)phenyl)ethanesulfonamide;
133) N-(3-(3-(1-Methyl-1H-pyrazol-4-yl)-4-oxo-3,4-dihydrophthalazin-1-yl)phenyl)ethanesulfonamide;
134) 4-(3-Aminopiperidin-1-yl)-2-(4-fluorophenyl)phthalazin-1(2H)-one·hydrochloride;
135) N-(1-(3-(4-Fluorophenyl)-4-oxo-3,4-dihydrophthalazin-1-yl)piperidin-3-yl)ethanesulfonamide;
136) N-(3-(3-(Oxetan-3-yl)-4-oxo-3,4-dihydrophthalazin-1-yl)phenyl)ethanesulfonamide;
137) N-(1-(3-(4-Fluorophenyl)-4-oxo-3,4-dihydrophthalazin-1-yl)pyrrolidin-3-yl)ethanesulfonamide;
138) (S)-N-(1-(3-(4-Fluorophenyl)-4-oxo-3,4-dihydrophthalazin-1-yl)piperidin-3-yl)ethanesulfonamide;
139) (R)-N-(1-(3-(4-Fluorophenyl)-4-oxo-3,4-dihydrophthalazin-1-yl)piperidin-3-yl)ethanesulfonamide;
140) N-(3-(3-(4,4-Difluorocyclohexyl)-4-oxo-3,4-dihydrophthalazin-1-yl)phenyl)ethanesulfonamide;
141) N-(1-(3-(4-Fluorophenyl)-4-oxo-3,4-dihydrophthalazin-1-yl)azepan-3-yl)ethanesulfonamide;
142) 3-(3-Cyclohexyl-4-oxo-3,4-dihydrophthalazin-1-yl)benzenesulfonamide;
143) 4-(3-Aminoazepan-1-yl)-2-cyclohexylphthalazin-1(2H)-one·hydrochloride;
144) N-(1-(3-Cyclohexyl-4-oxo-3,4-dihydrophthalazin-1-yl)azepan-3-yl)ethanesulfonamide;
145) N-(3-(3-(4-Methylcyclohexyl)-4-oxo-3,4-dihydrophthalazin-1-yl)phenyl)ethanesulfonamide;
146) (S)-4-(3-Aminopiperidin-1-yl)-2-cyclohexyl phthalazin-1(2H)-one·hydrochloride;
147) (S)-N-(1-(3-Cyclohexyl-4-oxo-3,4-dihydrophthalazin-1-yl)piperidin-3-yl)ethanesulfonamide;
148) 4-(3-Aminoazepan-1-yl)-2-(4-fluorophenyl)phthalazin-1(2H)-onehydrochloride;
149) N-(3-(2-(4-Fluorophenyl)-1-oxo-1,2-dihydro pyrido[3,4-d]pyridin-4-yl)phenyl)ethanesulfonamide;
150) N-(3-(3-(4-Fluorophenyl)-8-methyl-4-oxo-3,4-dihydrophthalazin-1-yl)phenyl)ethanesulfonamide;
151) N-(3-(4-Oxo-3-(tetrahydro-2H-pyran-4-yl)-3,4-dihydrophthalazin-1-yl)phenyl)ethanesulfonamide;
152) 2-(3-(3-(4-Fluorophenyl)-4-oxo-3,4-dihydrophthalazin-1-yl)phenyl)-2-methylpropanoic acid;
153) 2-(3-(3-(4-Fluorophenyl)-4-oxo-3,4-dihydrophthalazin-1-yl)phenyl)-N,2-dimethylpropanamide;
154) 2-Cyclohexyl-4-(3-(methylsulfonyl)phenyl)phthalazin-1(2H)-one;
155) (S)-4-(3-Aminoazepan-1-yl)-2-cyclohexyl phthalazin-1(2H)-one-hydrochloride;
156) (S)-2-Cyclohexyl-4-(3-(methylamino)azepan-1-yl)phthalazin-1(2H)-one-hydrochloride;
157) (S)-4-(3-Aminoazepan-1-yl)-2-(2,4-difluorophenyl)phthalazin-1(2H)-onehydrochloride;
158) (S)-4-(3-Aminoazepan-1-yl)-2-(4-fluorophenyl)phthalazin-1(2H)-one·hydrochloride;
159) (R)-4-(3-Aminoazepan-1-yl)-2-cyclohexyl phthalazin-1(2H)-one-hydrochloride;
160) 2-(3-(3-Cyclohexyl-4-oxo-3,4-dihydrophthalazin-1-yl)phenyl)-2-methylpropanoic acid;
161) 3-(3-(3-(4-Fluorophenyl)-4-oxo-3,4-dihydrophthalazin-1-yl)phenyl)oxazolidin-2-one;
162) (R)-4-(3-Aminoazepan-1-yl)-2-(4-fluorophenyl)phthalazin-1(2H)-onehydrochloride;
163) 2-(3-(3-Cyclohexyl-4-oxo-3,4-dihydrophthalazin-1-yl)phenyl)-2-methylpropanamide;
164) 2-(3-(3-Cyclohexyl-4-oxo-3,4-dihydrophthalazin-1-yl)phenyl)-N,2-dimethylpropanamide;
165) (R)-4-(3-Aminoazepan-1-yl)-2-(2,4-difluorophenyl)phthalazin-1(2H)-onehydrochloride;
166) 2-(3-(3-(2,4-Difluorophenyl)-4-oxo-3,4-dihydrophthalazin-1-yl)phenyl)-2-methylpropanoic acid;
167) 4-(3-Aminoazepan-1-yl)-2-(2,4-difluoro phenyl)phthalazin-1(2H)-onehydrochloride;
168) 2-(3-(3-(2,4-Difluorophenyl)-4-oxo-3,4-dihydrophthalazin-1-yl)phenyl)-2-methylpropanenitrile;
169) 4-(3-(2-(1H-Tetrazol-5-yl)propan-2-yl)phenyl)-2-(2,4-difluorophenyl)phthalazin-1(2H)-one;
170) (E)-2-(3-(3-(2,4-Difluorophenyl)-4-oxo-3,4-dihydrophthalazin-1-yl)phenyl)-N'-hydroxy-2-methylpropanimideamide;
171) 2-(3-(3-(2-Fluorophenyl)-4-oxo-3,4-dihydrophthalazin-1-yl)phenyl)-2-methylpropanoic acid;
172) 4-(3-Aminoazepan-1-yl)-2-cyclobutylphthalazin-1(2H)-one-hydrochloride;
173) 4-(3-Aminoazepan-1-yl)-2-cyclopentylphthalazin-1(2H)-one-hydrochloride;
174) 2-(2,4-Difluorophenyl)-4-(3-(2-(5-methyl-1,2,4-oxadiazol-3-yl)propan-2-yl)phenyl)phthalazin-1(2H)-one;
175) (R)-4-(3-Aminoazepan-1-yl)-2-(2-fluorophenyl)phthalazin-1(2H)-one-hydrochloride;
176) 2-(3-(3-(2,4-Difluorophenyl)-4-oxo-3,4-dihydrophthalazin-1-yl)phenyl)-N-isopropyl-2-methylpropanamide;
177) 2-(2,4-Difluorophenyl)-4-(3-(2-methyl-1-oxo-1-(pyrrolidin-1-yl)propan-2-yl)phenyl)phthalazin-1(2H)-one;
178) N-Cyclopropyl-2-(3-(3-(2,4-difluorophenyl)-4-oxo-3,4-dihydrophthalazin-1-yl)phenyl)-2-methylpropanamide;
179) 2-(2,4-Difluorophenyl)-4-(3-(2-methyl-1-morpholino-1-oxopropan-2-yl)phenyl)phthalazin-1(2H)-one;
180) N-Cyclohexyl-2-(3-(3-(2,4-difluorophenyl)-4-oxo-3,4-dihydrophthalazin-1-yl)phenyl)-2-methylpropanamide;
181) 2-(3-(3-(2,4-difluorophenyl)-4-oxo-3,4-dihydrophthalazin-1-yl)phenyl)-2-methyl-N-phenylpropanamide;
182) 2-(3-(3-(2,4-Difluorophenyl)-4-oxo-3,4-dihydrophthalazin-1-yl)phenyl)-2-methyl-N-(1-methyl-1H-pyrazol-4-yl)propanamide;
183) 1-(3-(3-(2,4-Difluorophenyl)-4-oxo-3,4-dihydrophthalazin-1-yl)phenyl)cyclopropan-1-carboxylic acid;
184) 4-(1,4-Diazepan-1-yl)-2-(2,4-difluorophenyl)phthalazin-1(2H)-onehydrochloride;
185) (R)-4-(3-Aminoazepan-1-yl)-2-(4-chlorophenyl)phthalazin-1(2H)-one·hydrochloride;
186) 2-(3-(3-(2,4-Difluorophenyl)-4-oxo-3,4-dihydrophthalazin-1-yl)phenyl)-N-(2-hydroxyethyl)-2-methylpropanamide;
187) (R)-N-(1-(3-(2,4-Difluorophenyl)-4-oxo-3,4-dihydrophthalazin-1-yl)azepan-3-yl)ethanesulfonamide;
188) 2-(3-(3-(2,4-Difluorophenyl)-4-oxo-3,4-dihydrophthalazin-1-yl)phenyl)-2-methylpropanamide;
189) (R)-2-(2,4-Difluorophenyl)-4-(3-(methylamino) azepan-1-yl)phthalazin-1(2H)-one-hydrochloride;
190) 2-(2,4-Difluorophenyl)-4-(3-hydroxyazepan-1-yl)phthalazin-1(2H)-one;
191) (R)-2-(2,4-Difluorophenyl)-4-(3-((2-hydroxyethyl) amino)azepan-1-yl)phthalazin-1(2H)-one-hydrochloride;
192) (R)-N-(1-(3-(2,4-Difluorophenyl)-4-oxo-3,4-dihydrophthalazin-1-yl)azepan-3-yl)acetamide;
193) 2-(2,4-Difluorophenyl)-4-(3-oxoazepan-1-yl)phthalazin-1(2H)-one;
194) 2-(3-(3-(4-Chlorophenyl)-4-oxo-3,4-dihydrophthalazin-1-yl)phenyl)-2-methylpropanoic acid;
195) 1-(3-(3-(4-Chlorophenyl)-4-oxo-3,4-dihydrophthalazin-1-yl)phenyl)cyclopropan-1-carboxylic acid;
196) 2-(4-Chlorophenyl)-4-(3-hydroxyazepan-1-yl)phthalazin-1(2H)-one;
197) 2-(3-(3-(4-Chlorophenyl)-4-oxo-3,4-dihydrophthalazin-1-yl)phenyl)-2-methylpropanamide;
198) 1-(3-(3-(4-Chlorophenyl)-4-oxo-3,4-dihydrophthalazin-1-yl)phenyl)cyclopropan-1-carboxyamide;
199) 2-(3-(3-(2,4-Difluorophenyl)-4-oxo-3,4-dihydrophthalazin-1-yl)phenyl)acetic acid;
200) 4-(3-(2-Aminopropan-2-yl)phenyl)-2-(2,4-difluoro phenyl)phthalazin-1(2H)-onehydrochloride;
201) 2-(3-(3-(4-Chloro-2-fluorophenyl)-4-oxo-3,4-dihydrophthalazin-1-yl)phenyl)-2-methylpropanoic acid;
202) (R)-4-(3-Aminoazepan-1-yl)-2-(4-chloro-2-fluoro phenyl)phthalazin-1-(2H)-one-hydrochloride;
203) 1-(3-(4-Chloro-2-fluorophenyl)-4-oxo-3,4-dihydrophthalazin-1-yl)azepane-3-carbonitrile;
204) 4-(3-(1-Amino-2-methylpropan-2-yl)phenyl)-2-(2,4-difluorophenyl)phthalazin-1(2H)-one·hydrochloride;
205) 2-(1-(3-(2,4-Difluorophenyl)-4-oxo-3,4-dihydrophthalazin-1-yl)piperidin-3-yl)-2-methylpropanoic acid;
206) 2-(3-(3-(4-Cyclopropylphenyl)-4-oxo-3,4-dihydrophthalazin-1-yl)phenyl)-2-methylpropanoic acid;
207) 1-(3-(3-(2,4-Difluorophenyl)-4-oxo-3,4-dihydrophthalazin-1-yl)phenyl)cyclobutane-1-carboxylic acid;
208) 1-(3-(3-(2,4-Difluorophenyl)-4-oxo-3,4-dihydrophthalazin-1-yl)phenyl)cyclopentane-1-carboxylic acid;
209) 2-(1-(3-(4-Chlorophenyl)-4-oxo-3,4-dihydrophthalazin-1-yl)piperidin-3-yl)-2-methylpropanoic acid;
210-1 and 210-2) 2-(1-(3-(2,4-Difluorophenyl)-4-oxo-3,4-dihydrophthalazin-1-yl)piperidin-3-yl)-2-methyl propanoic acid;
211) Methyl 1-(1-(3-(2,4-difluorophenyl)-4-oxo-3,4-dihydrophthalazin-1-yl)piperidin-3-yl)cyclopropan-1-carboxylate;
212) 1-(1-(3-(2,4-Difluorophenyl)-4-oxo-3,4-dihydrophthalazin-1-yl)piperidin-3-yl)cyclopropan-1-carboxylic acid;
213-1 and 213-2) 1-(1-(3-(2,4-Difluorophenyl)-4-oxo-3,4-dihydrophthalazin-1-yl)piperidin-3-yl)cyclopropan-1-carboxylic acid;
214) 2-(2,4-Difluorophenyl)-4-(3-(pyrrolidin-1-ylsulfonyl)phenyl)phthalazin-1(2H)-one;
215) 2-(2,4-Difluorophenyl)-4-(1,2,3,6-tetrahydro pyridin-4-yl)phthalazin-1(2H)-one·hydrochloride;
216) 3-(3-(2,4-Difluorophenyl)-4-oxo-3,4-dihydrophthalazin-1-yl)-N-ethyl-N-methylbenzenesulfonamide;
217) 3-(3-(2,4-Difluorophenyl)-4-oxo-3,4-dihydrophthalazin-1-yl)-N,N-diethylbenzenesulfonamide;
218) 2-(2,4-Difluorophenyl)-4-(1-(ethylsulfonyl)-1,2,3,6-tetrahydropyridin-4-yl)phthalazin-1(2H)-one;
219) 2-(4-(3-(2,4-Difluorophenyl)-4-oxo-3,4-dihydrophthalazin-1-yl)piperazin-2-yl)-2-methylpropanoic acid;
220) 2-(4-(3-(2,4-Difluorophenyl)-4-oxo-3,4-dihydrophthalazin-1-yl)-1-methylpiperazin-2-yl)-2-methylpropanoic acid; and
221) 2-(4-(3-(2,4-Difluorophenyl)-4-oxo-3,4-dihydrophthalazin-1-yl)morpholin-2-yl)-2-methylpropanoic acid.

2. A pharmaceutical composition for preventing or treating a Sirt6-related disease, comprising a compound, or a racemate, a stereoisomer or a pharmaceutically acceptable salt thereof according to claim 1, wherein the Sirt6-related disease is selected from the group consisting of a cancer, fatty liver, hepatocirrhosis /inflammation, decreased insulin secretion and diseases caused by aging.

## Patentansprüche

1. Eine Verbindung oder ein Racemat, ein Stereoisomer oder ein pharmazeutisch verträgliches Salz davon, wobei die Verbindung aus der Gruppe ausgewählt ist, bestehend aus:
1) N-(3-(4-Oxo-3-phenyl-3,4-dihydrophthalazin-1-yl)phenyl)ethansulfonamid;
2) 4-(3-Aminophenyl)-2-(4-(trifluormethyl)phenyl)phthalazin-1(2H)-on;
3) N-(3-(4-Oxo-3-(4-(trifluormethyl)phenyl)-3,4-dihydrophthalazin-1-yl)phenyl)ethansulfonamid;
4) 4-(3-(Ethylamino)phenyl)-2-(4-(trifluormethyl)phenyl)phthalazin-1(2H)-on;
5) N-Methyl-N-(3-(4-oxo-3-(4-(trifluormethyl)phenyl)-3,4-dihydrophthalazin-1-yl)phenyl)ethansulfonamid;
6) N-(3-(4-Oxo-3-phenyl-3,4-dihydrophthalazin-1-yl)phenyl)methansulfonamid;
7) N-Methyl-N-(3-(4-oxo-3-phenyl-3,4-dihydrophthalazin-1-yl)phenyl)methansulfonamid;
8) N-(4-(4-Oxo-3-phenyl-3,4-dihydrophthalazin-1-yl)phenyl)ethansulfonamid;
9) N-(3-(3-Phenyl)-4-oxo-3,4-dihydrophthalazin-1-yl)phenyl)dimethylsulfamoylamid;
10) N-(3-(3-(4-Fluorphenyl)-4-oxo-3,4-dihydrophthalazin-1-yl)phenyl)methansulfonamid;
11) N-(3-(3-(4-Fluorphenyl)-4-oxo-3,4-dihydrophthalazin-1-yl)phenyl)ethansulfonamid;
12) N-(3-(3-(4-Fluorphenyl)-4-oxo-3,4-dihydrophthalazin-1-yl)phenyl)dimethylsulfamoylamid;
13) (Z)-N-Methyl-1-(3-(4-oxo-3-phenyl-3,4-dihydrophthalazin-1-yl)phenyl)methaniminoxid;
14) (Z)-N-Isopropyl-1-(3-(4-oxo-3-phenyl-3,4-dihydrophthalazin-1-yl)phenyl)methaniminoxid;
15) (Z)-1-(3-(3-(4-Fluorphenyl)-4-oxo-3,4-dihydrophthalazin-1-yl)phenyl)-N-methylmethaniminoxid;
16) (Z)-N-Ethyl-1-(3-(3-(4-fluorphenyl)-4-oxo-3,4-dihydrophthalazin-1-yl)phenyl)methaniminoxid;
17) (Z)-1-(3-(3-(4-Fluorphenyl)-4-oxo-3,4-dihydrophthalazin-1-yl)phenyl)-N-isopropylmethaniminoxid;
18) N-(2-Chlor-5-(4-oxo-3-phenyl-3,4-dihydrophthalazin-1-yl)phenyl)-N-(methylsulfonyl)methansulfonamid;
19) N-(3-(3-(Phenyl)-4-oxo-3,4-dihydrophthalazin-1-yl)-6-chlorphenyl)dimethylsulfamoylamid;
20) Methyl-(N-(3-(3-(4-fluorphenyl)-4-oxo-3,4-dihydrophthalazin-1-yl)phenyl)sulfamoyl)carbamat;
21) (Z)-1-(3-(3-(2,4-Difluorphenyl)-4-oxo-3,4-dihydrophthalazin-1-yl)phenyl)-N-methylmethaniminoxid;
22) (Z)-1-(3-(3-(2,4-Difluorphenyl)-4-oxo-3,4-dihydrophthalazin-1-yl)phenyl)-N-isopropylmethaniminoxid;
23) (Z)-N-tert-Butyl-1-(3-(3-(2,4-difluorphenyl)-4-oxo-3,4-dihydrophthalazin-1-yl)phenyl)methaniminoxid;
24) 4-(3-Aminophenyl)-2-(2,4-difluorphenyl)phthalazin-1(2H)-on;
25) N-(3-(3-(2,4-Difluorphenyl)-4-oxo-3,4-dihydrophthalazin-1-yl)phenyl)ethansulfonamid;
26) Methyl-(N-(3-(3-(2,4-difluorphenyl)-4-oxo-3,4-dihydrophthalazin-1-yl)phenyl)sulfamoyl)carbamat;
27) (Z)-1-(3-(3-(3-Fluorphenyl)-4-oxo-3,4-dihydrophthalazin-1-yl)phenyl)-N-methylmethaniminoxid;
28) (Z)-1-(3-(3-(3-Fluorphenyl)-4-oxo-3,4-dihydrophthalazin-1-yl)phenyl)-N-isopropylmethaniminoxid;
29) (Z)-N-tert-Butyl-1-(3-(3-(3-fluorphenyl)-4-oxo-3,4-dihydrophthalazin-1-yl)phenyl)methaniminoxid;
30) N-(3-(3-(3-Fluorphenyl)-4-oxo-3,4-dihydrophthalazin-1-yl)phenyl)ethansulfonamid;
31) (Z)-1-(3-(3-(3,5-Difluorphenyl)-4-oxo-3,4-dihydrophthalazin-1-yl)phenyl)-N-isopropylmethaniminoxid;
32) (Z)-N-Isopropyl-1-(3-(3-(4-methoxyphenyl)-4-oxo-3,4-dihydrophthalazin-1-yl)phenyl)methaniminoxid;
33) (Z)-1-(3-(3-(3-Chlor-4-fluorphenyl)-4-oxo-3,4-dihydrophthalazin-1-yl)phenyl)-N-isopropylmethaniminoxid;
34) (Z)-N-*tert*-Butyl-1-(3-(3-(3-chlor-4-fluorphenyl)-4-oxo-3,4-dihydrophthalazin-1-yl)phenyl)methaniminoxid;
35) (Z)-N-Benzyl-1-(3-(4-oxo-3-phenyl-3,4-dihydrophthalazin-1-yl)phenyl)methaniminoxid;
36) (E)-1-(4-(3-(4-Fluorphenyl)-4-oxo-3,4-dihydrophthalazin-1-yl)phenyl)-N-methylmethaniminoxid;
37) (Z)-1-(3-(3-(4-Fluorphenyl)-4-oxo-3,4-dihydrophthalazin-1-yl)phenyl)-N-isopropylmethaniminoxid;
38) (E)-1-(3-Fluor-4-(3-(4-fluorphenyl)-4-oxo-3,4-dihydrophthalazin-1-yl)phenyl)-N-methylmethaniminoxid;
39) (E)-1-(3-Fluor-4-(3-(4-fluorphenyl)-4-oxo-3,4-dihydrophthalazin-1-yl)phenyl)-N-isopropylmethaniminoxid;
40) (Z)-1-(2-(3-(4-Fluorphenyl)-4-oxo-3,4-dihydrophthalazin-1-yl)phenyl)-N-methylmethaniminoxid;
41) (Z)-1-(2-(3-(4-Fluorphenyl)-4-oxo-3,4-dihydrophthalazin-1-yl)phenyl)-N-isopropylmethaniminoxid;
42) (Z)-N-*tert*-Butyl-1-(2-(3-(4-fluorphenyl)-4-oxo-3,4-dihydrophthalazin-1-yl)phenyl)methaniminoxid;
43) (Z)-N-*tert*-Butyl-1-(3-(3-(4-fluorphenyl)-4-oxo-3,4-dihydrophthalazin-1-yl)phenyl)methaniminoxid;
44) (Z)-N-Benzyl-1-(3-(3-(4-fluorphenyl)-4-oxo-3,4-dihydrophthalazin-1-yl)phenyl)methaniminoxid;
45) (Z)-1-(5-(3-(4-Fluorphenyl)-4-oxo-3,4-dihydrophthalazin-1-yl)thiophen-2-yl)-N-methylmethaniminoxid;
46) (Z)-1-(5-(3-(4-Fluorphenyl)-4-oxo-3,4-dihydrophthalazin-1-yl)thiophen-2-yl)-N-isopropylmethaniminoxid;
47) (Z)-N-*tert*-Butyl-1-(5-(3-(4-fluorphenyl)-4-oxo-3,4-dihydrophthalazin-1-yl)thiophen-2-yl)methaniminoxid;
48) (Z)-1-(5-(3-(4-Fluorphenyl)-4-oxo-3,4-dihydrophthalazin-1-yl)furan-2-yl)-N-isopropylmethaniminoxid;
49) (Z)-N-*tert*-Butyl-1-(5-(3-(4-fluorphenyl)-4-oxo-3,4-dihydrophthalazin-1-yl)-2-methoxyphenyl)methaniminoxid;
50) (Z)-N-Benzyl-1-(5-(3-(4-fluorphenyl)-4-oxo-3,4-dihydrophthalazin-1-yl)-2-methoxyphenyl)methaniminoxid;
51) (Z)-1-(2-Fluor-5-(3-(4-fluorphenyl)-4-oxo-3,4-dihydrophthalazin-1-yl)phenyl)-N-methylmethaniminoxid;
52) (Z)-1-(2-Fluor-5-(3-(4-fluorphenyl)-4-oxo-3,4-dihydrophthalazin-1-yl)phenyl)-N-isopropylmethaniminoxid;
53) (Z)-N-*tert*-Butyl-1-(2-fluor-5-(3-(4-fluorphenyl)-4-oxo-3,4-dihydrophthalazin-1-yl)phenyl)methaniminoxid;
54) (Z)-N-Benzyl-1-(2-fluor-5-(3-(4-fluorphenyl)-4-oxo-3,4-dihydrophthalazin-1-yl)phenyl)methaniminoxid;
55) (Z)-1-(4-Fluor-3-(3-(4-fluorphenyl)-4-oxo-3,4-dihydrophthalazin-1-yl)phenyl)-N-methylmethaniminoxid;
56) (Z)-1-(4-Fluor-3-(3-(4-fluorphenyl)-4-oxo-3,4-dihydrophthalazin-1-yl)phenyl)-N-isopropylmethaniminoxid;
57) (Z)-N-*tert*-Butyl-1-(4-fluor-3-(3-(4-fluorphenyl)-4-oxo-3,4-dihydrophthalazin-1-yl)phenyl)methaniminoxid;
58) (Z)-N-Benzyl-1-(4-fluor-3-(3-(4-fluorphenyl)-4-oxo-3,4-dihydrophthalazin-1-yl)phenyl)methaniminoxid;
59) 4-(4-Chlor-3-nitrophenyl)-2-(4-fluorphenyl)phthalazin-1(2H)-on;
60) 4-(3-Aminophenyl)-2-(4-fluorphenyl)phthalazin-1(2H)-on;
61) N-(3-(3-Benzyl-4-oxo-3,4-dihydrophthalazin-1-yl)phenyl)ethansulfonamid;
62) N-(3-(4-Oxo-3-phenethyl-3,4-dihydrophthalazin-1-yl)phenyl)ethansulfonamid;
63) N-(3-(3-(4-Fluorbenzyl)-4-oxo-3,4-dihydrophthalazin-1-yl)phenyl)ethansulfonamid;
64) N-(3-(3-([1,1'-Biphenyl]-4-yl)-4-oxo-3,4-dihydrophthalazin-1-yl)phenyl)ethansulfonamid;
65) N-(3-(3-([1,1'-Biphenyl]-4-yl)-4-oxo-3,4-dihydrophthalazin-1-yl)phenyl)-N-methylethansulfonamid;
66) Methyl-3-(3-(4-fluorphenyl)-4-oxo-3,4-dihydrophthalazin-1-yl)benzoat;
67) N-(3-(3-(4-Fluorphenyl)-4-oxo-3,4-dihydrophthalazin-1-yl)phenyl)propan-2-sulfonamid;
68) N-(3-(3-(4-Fluorphenyl)-4-oxo-3,4-dihydrophthalazin-1-yl)phenyl)propan-1-sulfonamid;
69) N-(3-(3-(4-Fluorphenyl)-4-oxo-3,4-dihydrophthalazin-1-yl)phenyl)-N-methylmethansulfonamid;
70) N-(3-(3-(4-Fluorphenyl)-4-oxo-3,4-dihydrophthalazin-1-yl)phenyl)-N-methylethansulfonamid;
71) N-(3-(3-(4-Fluorphenyl)-4-oxo-3,4-dihydrophthalazin-1-yl)phenyl)cyclopropansulfonamid;
72) N-(3-(3-(4-Fluorphenyl)-4-oxo-3,4-dihydrophthalazin-1-yl)phenyl)-N-methylpropan-1-sulfonamid;
73) N-(3-(3-(4-Fluorphenyl)-4-oxo-3,4-dihydrophthalazin-1-yl)phenyl)-N-methylpropan-2-sulfonamid;
74) N-(3-(3-(4-Fluorphenyl)-4-oxo-3,4-dihydrophthalazin-1-yl)phenyl)-N-methylcyclopropansulfonamid;
75) N-(3-(3-(4-Fluorphenyl)-4-oxo-3,4-dihydrophthalazin-1-yl)phenyl)-N-methyl-dimethylsulfamoylamid;
76) Methyl-(N-(3-(3-(4-fluorphenyl)-4-oxo-3,4-dihydrophthalazin-1-yl)phenyl)-N-methylsulfamoyl) (methyl)carbamat;
77) 1,1,1-Trifluor-N-(3-(3-(4-fluorphenyl)-4-oxo-3,4-dihydrophthalazin-1-yl)phenyl)methansulfonamid;
78) 1,1,1-Trifluor-N-(3-(3-(4-fluorphenyl)-4-oxo-3,4-dihydrophthalazin-1-yl)phenyl)-N-methylmethansulfonamid;
79) 3-(3-(4-Fluorphenyl)-4-oxo-3,4-dihydrophthalazin-1-yl)-N-((tetrahydro-2H-pyran-2-yl)oxy)benzamid;
80) N-(3-(3-(4-Fluorphenyl)-4-oxo-3,4-dihydrophthalazin-1-yl)phenyl)-N-methyl-methylsulfamoylamid;
81) 4-(3-(1H-Tetrazol-5-yl)phenyl)-2-(4-fluorphenyl)phthalazin-1(2H)-on;
82) N-(5-(3-(4-Fluorphenyl)-4-oxo-3,4-dihydrophthalazin-1-yl)pyridin-3-yl)ethansulfonamid;
83) 3-(3-(4-Fluorphenyl)-4-oxo-3,4-dihydrophthalazin-1-yl)-N-hydroxybenzamid;
84) (Z)-1-(3-(3-([1,1'-Biphenyl]-4-yl)-4-oxo-3,4-dihydrophthalazin-1-yl)phenyl)-N-methylmethaniminoxid;
85) (Z)-1-(3-(3-([1,1'-Biphenyl]-4-yl)-4-oxo-3,4-dihydrophthalazin-1-yl)phenyl)-N-isopropylmethaniminoxid;
86) 3-(3-(4-Fluorphenyl)-4-oxo-3,4-dihydrophthalazin-1-yl)-N-methylbenzolsulfonamid;
87) N-Ethyl-3-(3-(4-fluorphenyl)-4-oxo-3,4-dihydrophthalazin-1-yl)benzolsulfonamid;
88) 3-(3-(4-Fluorphenyl)-4-oxo-3,4-dihydrophthalazin-1-yl)-N,N-dimethylbenzolsulfonamid;
89) 2-(2,4-Difluorphenyl)-4-(3-(methylsulfonyl)phenyl)phthalazin-1(2H)-on;
90) 2-(2,4-Difluorphenyl)-4-(3-(ethylsulfonyl)phenyl)phthalazin-1(2H)-on;
91) 3-(3-(2,4-Difluorphenyl)-4-oxo-3,4-dihydrophthalazin-1-yl)-N-methylbenzolsulfonamid;
92) 3-(3-(2,4-Difluorphenyl)-4-oxo-3,4-dihydrophthalazin-1-yl)-N-ethylbenzolsulfonamid;
93) 3-(3-(2,4-Difluorphenyl)-4-oxo-3,4-dihydrophthalazin-1-yl)-N,N-dimethylbenzolsulfonamid;
94) (Z)-1-(3-(3-(2-Fluorphenyl)-4-oxo-3,4-dihydrophthalazin-1-yl)phenyl)-N-methylmethaniminoxid;
95) (Z)-1-(3-(3-(2-Fluorphenyl)-4-oxo-3,4-dihydrophthalazin-1-yl)phenyl)-N-isopropylmethaniminoxid;
96) (Z)-N-*tert*-Butyl-1-(3-(3-(2-fluorphenyl)-4-oxo-3,4-dihydrophthalazin-1-yl)phenyl)methaniminoxid;
97) 2-(2-Fluorphenyl)-4-(3-(methylsulfonyl)phenyl)phthalazin-1(2H)-on;
98) 4-(3-(Ethylsulfonyl)phenyl)-2-(2-fluorphenyl)phthalazin-1(2H)-on;
99) 3-(3-(2-Fluorphenyl)-4-oxo-3,4-dihydrophthalazin-1-yl)-N-methylbenzolsulfonamid;
100) N-Ethyl-3-(3-(2-fluorphenyl)-4-oxo-3,4-dihydrophthalazin-1-yl)benzolsulfonamid;
101) 3-(3-(2-Fluorphenyl)-4-oxo-3,4-dihydrophthalazin-1-yl)-N,N-dimethylbenzolsulfonamid;
102) (Z)-1-(3-(3-(2,6-Difluorphenyl)-4-oxo-3,4-dihydrophthalazin-1-yl)phenyl)-N-methylmethaniminoxid;
103) (Z)-1-(3-(3-(2,6-Difluorphenyl)-4-oxo-3,4-dihydrophthalazin-1-yl)phenyl)-N-isopropylmethaniminoxid;
104) (Z)-N-*tert*-Butyl-1-(3-(3-(2,6-difluorphenyl)-4-oxo-3,4-dihydrophthalazin-1-yl)phenyl)methaniminoxid;
105) 2-(2,6-Difluorphenyl)-4-(3-(methylsulfonyl)phenyl)phthalazin-1(2H)-on;
106) 2-(2,6-Difluorphenyl)-4-(3-(ethylsulfonyl)phenyl)phthalazin-1(2H)-on;
107) 3-(3-(2,6-Difluorphenyl)-4-oxo-3,4-dihydrophthalazin-1-yl)-N-methylbenzolsulfonamid;
108) 3-(3-(2,6-Difluorphenyl)-4-oxo-3,4-dihydrophthalazin-1-yl)-N-ethylbenzolsulfonamid;
109) 3-(3-(2,6-Difluorphenyl)-4-oxo-3,4-dihydrophthalazin-1-yl)-N,N-dimethylbenzolsulfonamid;
110) 6-Fluor-4-(3-(methylsulfonyl)phenyl)-2-phenylphthalazin-1(2H)-on;
111) 3-(7-Fluor-4-oxo-3-phenyl-3,4-dihydrophthalazin-1-yl)-N-methylbenzolsulfonamid;
112) 7-Fluor-4-(3-(methylsulfonyl)phenyl)-2-phenyl- - phthalazin-1(2H)-on;
113) 3-(6-Fluor-4-oxo-3-phenyl-3,4-dihydrophthalazin-1-yl)-N-methylbenzolsulfonamid;
114) 3-(3-(4-Fluorphenyl)-4-oxo-3,4-dihydrophthalazin-1-yl)pyridin-1-oxid;
115) 2-(2,4-Difluorphenyl)-4-(pyridin-3-yl)phthalazin-1(2H)-on;
116) 2-(2,6-Difluorphenyl)-4-(pyridin-3-yl)phthalazin-1(2H)-on;
117) 3-(3-(2,4-Difluorphenyl)-4-oxo-3,4-dihydrophthalazin-1-yl)pyridin-1-oxid;
118) 3-(3-(2,6-Difluorphenyl)-4-oxo-3,4-dihydrophthalazin-1-yl)pyridin-1-oxid;
119) 2-(2,4-Difluorphenyl)-6-fluor-4-(3-(methylsulfonyl)phenyl)phthalazin-1(2H)-on;
120) 2-(2,4-Difluorphenyl)-7-fluor-4-(3-(methylsulfonyl)phenyl)phthalazin-1(2H)-on;
121) 3-(3-(2,4-Difluorphenyl)-7-fluor-4-oxo-3,4-dihydrophthalazin-1-yl)-N-methylbenzolsulfonamid;
122) 3-(3-(2,4-Difluorphenyl)-6-fluor-4-oxo-3,4-dihydrophthalazin-1-yl)-N-methylbenzolsulfonamid;
123) 2-(2,4-Difluorphenyl)-4-(3-(ethylsulfonyl)phenyl)-6-fluorphthalazin-1(2H)-on;
124) 3-(3-(2,4-Difluorphenyl)-7-fluor-4-oxo-3,4-dihydrophthalazin-1-yl)-N-ethylbenzolsulfonamid;
125) 3-(3-(2,4-Difluorphenyl)-7-fluor-4-oxo-3,4-dihydrophthalazin-1-yl)-N,N-dimethylbenzolsulfonamid;
126) 3-(3-(2,4-Difluorphenyl)-4-oxo-3,4-dihydrophthalazin-1-yl)benzolsulfonamid;
127) 3-(3-(2,4-Difluorphenyl)-4-oxo-3,4-dihydrophthalazin-1-yl)-N-(2-hydroxyethyl)benzolsulfonamid;
128) N-(3-(3-Cyclohexyl-4-oxo-3,4-dihydrophthalazin-1-yl)phenyl)ethansulfonamid;
129) N-(3-(3-(1-Methylpiperidin-4-yl)-4-oxo-3,4-dihydrophthalazin-1-yl)phenyl)ethansulfonamid;
130) 3-(3-(4-Fluorphenyl)-4-oxo-3,4-dihydrophthalazin-1-yl)benzonitril;
131) (E)-3-(3-(4-Fluorphenyl)-4-oxo-3,4-dihydrophthalazin-1-yl)-N'-hydroxybenzimidamid;
132) N-(3-(3-Isopropyl-4-oxo-3,4-dihydrophthalazin-1-yl)phenyl)ethansulfonamid;
133) N-(3-(3-(1-Methyl-1H-pyrazol-4-yl)-4-oxo-3,4-dihydrophthalazin-1-yl)phenyl)ethansulfonamid;
134) 4-(3-Aminopiperidin-1-yl)-2-(4-fluorphenyl)phthalazin-1(2H)-on·Hydrochlorid;
135) N-(1-(3-(4-Fluorphenyl)-4-oxo-3,4-dihydrophthalazin-1-yl)piperidin-3-yl)ethansulfonamid;
136) N-(3-(3-(Oxetan-3-yl)-4-oxo-3,4-dihydrophthalazin-1-yl)phenyl)ethansulfonamid;
137) N-(1-(3-(4-Fluorphenyl)-4-oxo-3,4-dihydrophthalazin-1-yl)pyrrolidin-3-yl)ethansulfonamid;
138) (S)-N-(1-(3-(4-Fluorphenyl)-4-oxo-3,4-dihydrophthalazin-1-yl)piperidin-3-yl)ethansulfonamid;
139) (R)-N-(1-(3-(4-Fluorphenyl)-4-oxo-3,4-dihydrophthalazin-1-yl)piperidin-3-yl)ethansulfonamid;
140) N-(3-(3-(4,4-Difluorcyclohexyl)-4-oxo-3,4-dihydrophthalazin-1-yl)phenyl)ethansulfonamid;
141) N-(1-(3-(4-Fluorphenyl)-4-oxo-3,4-dihydrophthalazin-1-yl)azepan-3-yl)ethansulfonamid;
142) 3-(3-Cyclohexyl-4-oxo-3,4-dihydrophthalazin-1-yl)benzolsulfonamid;
143) 4-(3-Aminoazepan-1-yl)-2-cyclohexylphthalazin-1(2H)-on·Hydrochlorid;
144) N-(1-(3-Cyclohexyl-4-oxo-3,4-dihydrophthalazin-1-yl)azepan-3-yl)ethansulfonamid;
145) N-(3-(3-(4-Methylcyclohexyl)-4-oxo-3,4-dihydrophthalazin-1-yl)phenyl)ethansulfonamid;
146) (S)-4-(3-Aminopiperidin-1-yl)-2-cyclohexylphthalazin-1(2H)-on·Hydrochlorid;
147) (S)-N-(1-(3-Cyclohexyl-4-oxo-3,4-dihydrophthalazin-1-yl)piperidin-3-yl)ethansulfonamid;
148) 4-(3-Aminoazepan-1-yl)-2-(4-fluorphenyl)phthalazin-1(2H)-on·Hydrochlorid;
149) N-(3-(2-(4-Fluorphenyl)-1-oxo-1,2-dihydro pyrido[3,4-d]pyridin-4-yl)phenyl)ethansulfonamid;
150) N-(3-(3-(4-Fluorphenyl)-8-methyl-4-oxo-3,4-dihydrophthalazin-1-yl)phenyl)ethansulfonamid;
151) N-(3-(4-Oxo-3-(tetrahydro-2H-pyran-4-yl)-3,4-dihydrophthalazin-1-yl)phenyl)ethansulfonamid;
152) 2-(3-(3-(4-Fluorphenyl)-4-oxo-3,4-dihydrophthalazin-1-yl)phenyl)-2-methylpropansäure;
153) 2-(3-(3-(4-Fluorphenyl)-4-oxo-3,4-dihydrophthalazin-1-yl)phenyl)-N,2-dimethylpropanamid;
154) 2-Cyclohexyl-4-(3-(methylsulfonyl)phenyl)phthalazin-1(2H)-on;
155) (S)-4-(3-Aminoazepan-1-yl)-2-cyclohexylphthalazin-1(2H)-on·Hydrochlorid;
156) (S)-2-Cyclohexyl-4-(3-(methylamino)azepan-1-yl)phthalazin-1(2H)-on·Hydrochlorid;
157) (S)-4-(3-Aminoazepan-1-yl)-2-(2,4-difluorphenyl)phthalazin-1(2H)-on-Hydrochlorid;
158) (S)-4-(3-Aminoazepan-1-yl)-2-(4-fluorphenyl)phthalazin-1(2H)-on·Hydrochlorid;
159) (R)-4-(3-Aminoazepan-1-yl)-2-cyclohexyl-phthalazin-1(2H)-onHydrochlorid;
160) 2-(3-(3-Cyclohexyl-4-oxo-3,4-dihydrophthalazin-1-yl)phenyl)-2-methylpropansäure;
161) 3-(3-(3-(4-Fluorphenyl)-4-oxo-3,4-dihydrophthalazin-1-yl)phenyl)oxazolidin-2-on;
162) (R)-4-(3-Aminoazepan-1-yl)-2-(4-fluorphenyl)phthalazin-1(2H)-on·Hydrochlorid;
163) 2-(3-(3-Cyclohexyl-4-oxo-3,4-dihydrophthalazin-1-yl)phenyl)-2-methylpropanamid;
164) 2-(3-(3-Cyclohexyl-4-oxo-3,4-dihydrophthalazin-1-yl)phenyl)-N,2-dimethylpropanamid;
165) (R)-4-(3-Aminoazepan-1-yl)-2-(2,4-difluorphenyl)phthalazin-1(2H)-on-Hydrochlorid;
166) 2-(3-(3-(2,4-Difluorphenyl)-4-oxo-3,4-dihydrophthalazin-1-yl)phenyl)-2-methylpropansäure;
167) 4-(3-Aminoazepan-1-yl)-2-(2,4-difluorphenyl)phthalazin-1(2H)-on·Hydrochlorid;
168) 2-(3-(3-(2,4-Difluorphenyl)-4-oxo-3,4-dihydrophthalazin-1-yl)phenyl)-2-methylpropannitril;
169) 4-(3-(2-(1H-Tetrazol-5-yl)propan-2-yl)phenyl)-2-(2,4-difluorphenyl)phthalazin-1(2H)-on;
170) (E)-2-(3-(3-(2,4-Difluorphenyl)-4-oxo-3,4-dihydrophthalazin-1-yl)phenyl)-N'-hydroxy-2-methylpropanimidamid;
171) 2-(3-(3-(2-Fluorphenyl)-4-oxo-3,4-dihydrophthalazin-1-yl)phenyl)-2-methylpropansäure;
172) 4-(3-Aminoazepan-1-yl)-2-cyclobutylphthalazin-1(2H)-on·Hydrochlorid;
173) 4-(3-Aminoazepan-1-yl)-2-cyclopentylphthalazin-1(2H)-on·Hydrochlorid;
174) 2-(2,4-Difluorphenyl)-4-(3-(2-(5-methyl-1,2,4-oxadiazol-3-yl)propan-2-yl)phenyl)phthalazin-1(2H)-on;
175) (R)-4-(3-Aminoazepan-1-yl)-2-(2-fluorphenyl)phthalazin-1(2H)-on·Hydrochlorid;
176) 2-(3-(3-(2,4-Difluorphenyl)-4-oxo-3,4-dihydrophthalazin-1-yl)phenyl)-N-isopropyl-2-methylpropanamid;
177) 2-(2,4-Difluorphenyl)-4-(3-(2-methyl-1-oxo-1-(pyrrolidin-1-yl)propan-2-yl)phenyl)phthalazin-1(2H)-on;
178) N-Cyclopropyl-2-(3-(3-(2,4-difluorphenyl)-4-oxo-3,4-dihydrophthalazin-1-yl)phenyl)-2-methylpropanamid;
179) 2-(2,4-Difluorphenyl)-4-(3-(2-methyl-1-morpholino-1-oxopropan-2-yl)phenyl)phthalazin-1(2H)-on;
180) N-Cyclohexyl-2-(3-(3-(2,4-difluorphenyl)-4-oxo-3,4-dihydrophthalazin-1-yl)phenyl)-2-methylpropanamid;
181) 2-(3-(3-(2,4-Difluorphenyl)-4-oxo-3,4-dihydrophthalazin-1-yl)phenyl)-2-methyl-N-phenylpropanamid;
182) 2-(3-(3-(2,4-Difluorphenyl)-4-oxo-3,4-dihydrophthalazin-1-yl)phenyl)-2-methyl-N-(1-methyl-1H-pyrazol-4-yl)propanamid;
183) 1-(3-(3-(2,4-Difluorphenyl)-4-oxo-3,4-dihydrophthalazin-1-yl)phenyl)cyclopropan-1-carbonsäure;
184) 4-(1,4-Diazepan-1-yl)-2-(2,4-difluorphenyl)phthalazin-1(2H)-on·Hydrochlorid;
185) (R)-4-(3-Aminoazepan-1-yl)-2-(4-chlorphenyl)phthalazin-1(2H)-on·Hydrochlorid;
186) 2-(3-(3-(2,4-Difluorphenyl)-4-oxo-3,4-dihydrophthalazin-1-yl)phenyl)-N-(2-hydroxyethyl)-2-methylpropanamid;
187) (R)-N-(1-(3-(2,4-Difluorphenyl)-4-oxo-3,4-dihydrophthalazin-1-yl)azepan-3-yl)ethansulfonamid;
188) 2-(3-(3-(2,4-Difluorphenyl)-4-oxo-3,4-dihydrophthalazin-1-yl)phenyl)-2-methylpropanamid;
189) (R)-2-(2,4-Difluorphenyl)-4-(3-(methylamino)azepan-1-yl)phthalazin-1(2H)-on·Hydrochlorid;
190) 2-(2,4-Difluorphenyl)-4-(3-hydroxyazepan-1-yl)phthalazin-1(2H)-on;
191) (R)-2-(2,4-Difluorphenyl)-4-(3-((2-hydroxyethyl)amino)azepan-1-yl)phthalazin-1(2H)-on·Hydrochlorid;
192) (R)-N-(1-(3-(2,4-Difluorphenyl)-4-oxo-3,4-dihydrophthalazin-1-yl)azepan-3-yl)acetamid;
193) 2-(2,4-Difluorphenyl)-4-(3-oxoazepan-1-yl)phthalazin-1(2H)-on;
194) 2-(3-(3-(4-Chlorphenyl)-4-oxo-3,4-dihydrophthalazin-1-yl)phenyl)-2-methylpropansäure;
195) 1-(3-(3-(4-Chlorphenyl)-4-oxo-3,4-dihydrophthalazin-1-yl)phenyl)cyclopropan-1-carbonsäure;
196) 2-(4-Chlorphenyl)-4-(3-hydroxyazepan-1-yl)phthalazin-1(2H)-on;
197) 2-(3-(3-(4-Chlorphenyl)-4-oxo-3,4-dihydrophthalazin-1-yl)phenyl)-2-methylpropanamid;
198) 1-(3-(3-(4-Chlorphenyl)-4-oxo-3,4-dihydrophthalazin-1-yl)phenyl)cyclopropan-1-carboxamid;
199) 2-(3-(3-(2,4-Difluorphenyl)-4-oxo-3,4-dihydrophthalazin-1-yl)phenyl)essigsäure;
200) 4-(3-(2-Aminopropan-2-yl)phenyl)-2-(2,4-difluorphenyl)phthalazin-1(2H)-on-Hydrochlorid;
201) 2-(3-(3-(4-Chlor-2-fluorphenyl)-4-oxo-3,4-dihydrophthalazin-1-yl)phenyl)-2-methylpropansäure;
202) (R)-4-(3-Aminoazepan-1-yl)-2-(4-chlor-2-fluorphenyl)phthalazin-1-(2H)-on·Hydrochlorid;
203) 1-(3-(4-Chlor-2-fluorphenyl)-4-oxo-3,4-dihydrophthalazin-1-yl)azepan-3-carbonitril;
204) 4-(3-(1-Amino-2-methylpropan-2-yl)phenyl)-2-(2,4-difluorphenyl)phthalazin-1(2H)-on·Hydrochlorid;
205) 2-(1-(3-(2,4-Difluorphenyl)-4-oxo-3,4-dihydrophthalazin-1-yl)piperidin-3-yl)-2-methylpropansäure;
206) 2-(3-(3-(4-Cyclopropylphenyl)-4-oxo-3,4-dihydrophthalazin-1-yl)phenyl)-2-methylpropansäure;
207) 1-(3-(3-(2,4-Difluorphenyl)-4-oxo-3,4-dihydrophthalazin-1-yl)phenyl)cyclobutan-1-carbonsäure;
208) 1-(3-(3-(2,4-Difluorphenyl)-4-oxo-3,4-dihydrophthalazin-1-yl)phenyl)cyclopentan-1-carbonsäure;
209) 2-(1-(3-(4-Chlorphenyl)-4-oxo-3,4-dihydrophthalazin-1-yl)piperidin-3-yl)-2-methylpropansäure;
210-1 und 210-2) 2-(1-(3-(2,4-Difluorphenyl)-4-oxo-3,4-dihydrophthalazin-1-yl)piperidin-3-yl)-2-methylpropansäure;
211) Methyl-1-(1-(3-(2,4-difluorphenyl)-4-oxo-3,4-dihydrophthalazin-1-yl)piperidin-3-yl)cyclopropan-1-carboxylat;
212) 1-(1-(3-(2,4-Difluorphenyl)-4-oxo-3,4-dihydrophthalazin-1-yl)piperidin-3-yl)cyclopropan-1-carbonsäure;
213-1 und 213-2) 1-(1-(3-(2,4-Difluorphenyl)-4-oxo-3,4-dihydrophthalazin-1-yl)piperidin-3-yl)cyclopropan-1-carbonsäure;
214) 2-(2,4-Difluorphenyl)-4-(3-(pyrrolidin-1-ylsulfonyl)phenyl)phthalazin-1(2H)-on;
215) 2-(2,4-Difluorphenyl)-4-(1,2,3,6-tetrahydropyridin-4-yl)phthalazin-1(2H)-on·Hydrochlorid;
216) 3-(3-(2,4-Difluorphenyl)-4-oxo-3,4-dihydrophthalazin-1-yl)-N-ethyl-N-methylbenzolsulfonamid;
217) 3-(3-(2,4-Difluorphenyl)-4-oxo-3,4-dihydrophthalazin-1-yl)-N,N-diethylbenzolsulfonamid;
218) 2-(2,4-Difluorphenyl)-4-(1-(ethylsulfonyl)-1,2,3,6-tetrahydropyridin-4-yl)phthalazin-1(2H)-on;
219) 2-(4-(3-(2,4-Difluorphenyl)-4-oxo-3,4-dihydrophthalazin-1-yl)piperazin-2-yl)-2-methylpropansäure;
220) 2-(4-(3-(2,4-Difluorphenyl)-4-oxo-3,4-dihydrophthalazin-1-yl)-1-methylpiperazin-2-yl)-2-methylpropansäure; und
221) 2-(4-(3-(2,4-Difluorphenyl)-4-oxo-3,4-dihydrophthalazin-1-yl)morpholin-2-yl)-2-methylpropansäure.

2. Pharmazeutische Zusammensetzung zur Vorbeugung oder Behandlung einer Sirt6-assoziierten Erkrankung, umfassend eine Verbindung oder ein Racemat, ein Stereoisomer oder ein pharmazeutisch verträgliches Salz davon nach Anspruch 1, wobei die Sirt6-assoziierte Erkrankung aus der Gruppe ausgewählt ist, bestehend aus Krebs, Fettleber, Leberzirrhose/Entzündung, verminderter Insulinsekretion und durch Altern verursachten Erkrankungen.

## Revendications

1. Composé ou racémate, stéréoisomère ou sel pharmaceutiquement acceptable de celui-ci, dans lequel le composé est choisi dans le groupe constitué par :
1) N-(3-(4-oxo-3-phényl-3,4-dihydrophtalazin-1-yl)phényl)éthanesulfonamide ;
2) 4-(3-aminophényl)-2-(4-(trifluorométhyl)phényl)phtalazin-1(2H)-one ;
3) N-(3-(4-oxo-3-(4-(trifluorométhyl)phényl)-3,4-dihydrophtalazin-1-yl)phényl)éthanesulfonamide ;
4) 4-(3-(éthylamino)phényl)-2-(4-(trifluorométhyl)phényl)phtalazin-1(2H)-one ;
5) N-méthyl-N-(3-(4-oxo-3-(4-(trifluorométhyl)phényl)-3,4-dihydrophtalazin-1-yl)phényl)éthanesulfonamide ;
6) N-(3-(4-oxo-3-phényl-3,4-dihydrophtalazin-1-yl)phényl)méthanesulfonamide ;
7) N-méthyl-N-(3-(4-oxo-3-phényl-3,4-dihydrophtalazin-1-yl)phényl)méthanesulfonamide ;
8) N-(4-(4-oxo-3-phényl-3,4-dihydrophtalazin-1-yl)phényl)éthanesulfonamide ;
9) N-(3-(3-phényl)-4-oxo-3,4-dihydrophtalazin-1-yl)phényl)diméthylsulfamoylamide ;
10) N-(3-(3-(4-fluorophényl)-4-oxo-3,4-dihydrophtalazin-1-yl)phényl)méthanesulfonamide ;
11) N-(3-(3-(4-fluorophényl)-4-oxo-3,4-dihydrophtalazin-1-yl)phényl)éthanesulfonamide ;
12) N-(3-(3-(4-fluorophényl)-4-oxo-3,4-dihydrophtalazin-1-yl)phényl)diméthylsulfamoylamide ;
13) (Z)-N-méthyl-1-(3-(4-oxo-3-phényl-3,4-dihydrophtalazin-1-yl)phényl)méthanimine oxyde ;
14) (Z)-N-isopropyl-1-(3-(4-oxo-3-phényl-3,4-dihydrophtalazin-1-yl)phényl)méthanimine oxyde ;
15) (Z)-1-(3-(3-(4-fluorophényl)-4-oxo-3,4-dihydrophtalazin-1-yl)phényl)-N-méthylméthanimine oxyde ;
16) (Z)-N-éthyl-1-(3-(3-(4-fluorophényl)-4-oxo-3,4-dihydrophtalazin-1-yl)phényl)méthanimine oxyde ;
17) (Z)-1-(3-(3-(4-fluorophényl)-4-oxo-3,4-dihydrophtalazin-1-yl)phényl)-N-isopropylméthanimine oxyde ;
18) N-(2-chloro-5-(4-oxo-3-phényl-3,4-dihydrophtalazin-1-yl)phényl)-N-(méthylsulfonyl)méthane-sulfonamide ;
19) N-(3-(3-(phényl)-4-oxo-3,4-dihydrophtalazin-1-yl)-6-chlorophényl)diméthylsulfamoylamide ;
20) méthyl (N-(3-(3-(4-fluorophényl)-4-oxo-3,4-dihydrophtalazin-1-yl)phényl)sulfamoyl)carbamate ;
21) (Z)-1-(3-(3-(2,4-difluorophényl)-4-oxo-3,4-dihydrophtalazin-1-yl)phényl)-N-méthylméthanimine oxyde ;
22) (Z)-1-(3-(3-(2,4-difluorophényl)-4-oxo-3,4-dihydrophtalazin-1-yl)phényl)-N-isopropylméthanimine oxyde ;
23) (Z)-N-*tert*-butyl-1-(3-(3-(2,4-difluorophényl)-4-oxo-3,4-dihydrophtalazin-1-yl)phényl)méthanimine oxyde ;
24) 4-(3-aminophényl)-2-(2,4-difluorophényl)phtalazin-1(2H)-one ;
25) N-(3-(3-(2,4-difluorophényl)-4-oxo-3,4-dihydrophtalazin-1-yl)phényl)éthanesulfonamide ;
26) méthyl (N-(3-(3-(2,4-difluorophényl)-4-oxo-3,4-dihydrophtalazin-1-yl)phényl)sulfamoyl)carbamate ;
27) (Z)-1-(3-(3-(3-fluorophényl)-4-oxo-3,4-dihydrophtalazin-1-yl)phényl)-N-méthylméthanimine oxyde ;
28) (Z)-1-(3-(3-(3-fluorophényl)-4-oxo-3,4-dihydrophtalazin-1-yl)phényl)-N-isopropylméthanimine oxyde ;
29) (Z)-N-*tert*-butyl-1-(3-(3-(3-fluorophényl)-4-oxo-3,4-dihydrophtalazin-1-yl)phényl)méthanimine oxyde ;
30) N-(3-(3-(3-fluorophényl)-4-oxo-3,4-dihydrophtalazin-1-yl)phényl)éthanesulfonamide ;
31) (Z)-1-(3-(3-(3,5-difluorophényl)-4-oxo-3,4-dihydrophtalazin-1-yl)phényl)-N-isopropylméthanimine oxyde ;
32) (Z)-N-isopropyl-1-(3-(3-(4-méthoxyphényl)-4-oxo-3,4-dihydrophtalazin-1-yl)phényl)méthanimine oxyde ;
33) (Z)-1-(3-(3-(3-chloro-4-fluorophényl)-4-oxo-3,4-dihydrophtalazin-1-yl)phényl)-N-isopropylméthanimine oxyde ;
34) (Z)-N-tert-butyl-1-(3-(3-(3-chloro-4-fluorophényl)-4-oxo-3,4-dihydrophtalazin-1-yl)phényl)méthanimine oxyde ;
35) (Z)-N-benzyl-1-(3-(4-oxo-3-phényl-3,4-dihydrophtalazin-1-yl)phényl)méthanimine oxyde ;
36) (E)-1-(4-(3-(4-fluorophényl)-4-oxo-3,4-dihydrophtalazin-1-yl)phényl)-N-méthylméthanimine oxyde ;
37) (Z)-1-(3-(3-(4-fluorophényl)-4-oxo-3,4-dihydrophtalazin-1-yl)phényl)-N-isopropylméthanimine oxyde ;
38) (E)-1-(3-fluoro-4-(3-(4-fluorophényl)-4-oxo-3,4-dihydrophtalazin-1-yl)phényl)-N-méthylméthanimine oxyde ;
39) (E)-1-(3-fluoro-4-(3-(4-fluorophényl)-4-oxo-3,4-dihydrophtalazin-1-yl)phényl)-N-isopropylméthanimine oxyde ;
40) (Z)-1-(2-(3-(4-fluorophényl)-4-oxo-3,4-dihydrophtalazin-1-yl)phényl)-N-méthylméthanimine oxyde ;
41) (Z)-1-(2-(3-(4-fluorophényl)-4-oxo-3,4-dihydrophtalazin-1-yl)phényl)-N-isopropylméthanimine oxyde ;
42) (Z)-N-*tert*-butyl-1-(2-(3-(4-fluorophényl)-4-oxo-3,4-dihydrophtalazin-1-yl)phényl)méthanimine oxyde ;
43) (Z)-N-*tert*-butyl-1-(3-(3-(4-fluorophényl)-4-oxo-3,4-dihydrophtalazin-1-yl)phényl)méthanimine oxyde ;
44) (Z)-N-benzyl-1-(3-(3-(4-fluorophényl)-4-oxo-3,4-dihydrophtalazin-1-yl)phényl)méthanimine oxyde ;
45) (Z)-1-(5-(3-(4-fluorophényl)-4-oxo-3,4-dihydrophtalazin-1-yl)thiophén-2-yl)-N-méthylméthanimine oxyde ;
46) (Z)-1-(5-(3-(4-fluorophényl)-4-oxo-3,4-dihydrophtalazin-1-yl)thiophén-2-yl)-N-isopropylméthanimine oxyde ;
47) (Z)-N-*tert*-butyl-1-(5-(3-(4-fluorophényl)-4-oxo-3,4-dihydrophtalazin-1-yl)thiophène-2-yl)méthanimine oxyde ;
48) (Z)-1-(5-(3-(4-fluorophényl)-4-oxo-3,4-dihydrophtalazin-1-yl)furan-2-yl)-N-isopropylméthanimine oxyde ;
49) (Z)-N-*tert*-butyl-1-(5-(3-(4-fluorophényl)-4-oxo-3,4-dihydrophtalazin-1-yl)-2-méthoxyphényl)méthanimine oxyde ;
50) (Z)-N-benzyl-1-(5-(3-(4-fluorophényl)-4-oxo-3,4-dihydrophtalazin-1-yl)-2-méthoxyphényl)méthanimine oxyde ;
51) (Z)-1-(2-fluoro-5-(3-(4-fluorophényl)-4-oxo-3,4-dihydrophtalazin-1-yl)phényl)-N-méthylméthanimine oxyde ;
52) (Z)-1-(2-fluoro-5-(3-(4-fluorophényl)-4-oxo-3,4-dihydrophtalazin-1-yl)phényl)-N-isopropylméthanimine oxyde ;
53) (Z)-N-*tert*-butyl-1-(2-fluoro-5-(3-(4-fluorophényl)-4-oxo-3,4-dihydrophtalazin-1-yl)phényl)méthanimine oxyde ;
54) (Z)-N-benzyl-1-(2-fluoro-5-(3-(4-fluorophényl)-4-oxo-3,4-dihydrophtalazin-1-yl)phényl)méthanimine oxyde ;
55) (Z)-1-(4-fluoro-3-(3-(4-fluorophényl)-4-oxo-3,4-dihydrophtalazin-1-yl)phényl)-N-méthylméthanimine oxyde ;
56) (Z)-1-(4-fluoro-3-(3-(4-fluorophényl)-4-oxo-3,4-dihydrophtalazin-1-yl)phényl)-N-isopropylméthanimine oxyde ;
57) (Z)-N-*tert*-butyl-1-(4-fluoro-3-(3-(4-fluorophényl)-4-oxo-3,4-dihydrophtalazin-1-yl)phényl)méthanimine oxyde ;
58) (Z)-N-benzyl-1-(4-fluoro-3-(3-(4-fluorophényl)-4-oxo-3,4-dihydrophtalazin-1-yl)phényl)méthanimine oxyde ;
59) 4-(4-chloro-3-nitrophényl)-2-(4-fluorophényl)phtalazin-1(2H)-one ;
60) 4-(3-aminophényl)-2-(4-fluorophényl)phtalazin-1(2H)-one ;
61) N-(3-(3-benzyl-4-oxo-3,4-dihydrophtalazin-1-yl)phényl)éthanesulfonamide ;
62) N-(3-(4-oxo-3-phénéthyl-3,4-dihydrophtalazin-1-yl)phényl)éthanesulfonamide ;
63) N-(3-(3-(4-fluorobenzyl)-4-oxo-3,4-dihydrophtalazin-1-yl)phényl)éthanesulfonamide ;
64) N-(3-(3-([1,1'-biphényl]-4-yl)-4-oxo-3,4-dihydrophtalazin-1-yl)phényl)éthanesulfonamide ;
65) N-(3-(3-([1,1'-biphényl]-4-yl)-4-oxo-3,4-dihydrophtalazin-1-yl)phényl)-N-méthyléthanesulfonamide ;
66) 3-(3-(4-fluorophényl)-4-oxo-3,4-dihydrophtalazin-1-yl)benzoate de méthyle ;
67) N-(3-(3-(4-fluorophényl)-4-oxo-3,4-dihydrophtalazin-1-yl)phényl)propan-2-sulfonamide ;
68) N-(3-(3-(4-fluorophényl)-4-oxo-3,4-dihydrophtalazin-1-yl)phényl)propan-1-sulfonamide ;
69) N-(3-(3-(4-fluorophényl)-4-oxo-3,4-dihydrophtalazin-1-yl)phényl)-N-méthylméthanesulfonamide ;
70) N-(3-(3-(4-fluorophényl)-4-oxo-3,4-dihydrophtalazin-1-yl)phényl)-N-méthyléthanesulfonamide ;
71) N-(3-(3-(4-fluorophényl)-4-oxo-3,4-dihydrophtalazin-1-yl)phényl)cyclopropanesulfonamide ;
72) N-(3-(3-(4-fluorophényl)-4-oxo-3,4-dihydrophtalazin-1-yl)phényl)-N-méthylpropane-1-sulfonamide ;
73) N-(3-(3-(4-fluorophényl)-4-oxo-3,4-dihydrophtalazin-1-yl)phényl)-N-méthylpropan-2-sulfonamide ;
74) N-(3-(3-(4-fluorophényl)-4-oxo-3,4-dihydrophtalazin-1-yl)phényl)-N-méthylcyclopropanesulfonamide ;
75) N-(3-(3-(4-fluorophényl)-4-oxo-3,4-dihydrophtalazin-1-yl)phényl)-N-méthyl-diméthylsulfamoylamide ;
76) méthyl (N-(3-(3-(4-fluorophényl)-4-oxo-3,4-dihydrophtalazin-1-yl)phényl)-N-méthylsulfamoyl) (méthyl)carbamate ;
77) 1,1,1-trifluoro-N-(3-(3-(4-fluorophényl)-4-oxo-3,4-dihydrophtalazin-1-yl)phényl)méthanesulfonamide ;
78) 1,1,1-trifluoro-N-(3-(3-(4-fluorophényl)-4-oxo-3,4-dihydrophtalazin-1-yl)phényl)-N-méthylméthanesulfonamide ;
79) 3-(3-(4-fluorophényl)-4-oxo-3,4-dihydrophtalazin-1-yl)-N-((tétrahydro-2H-pyran-2-yl)oxy)benzamide ;
80) N-(3-(3-(4-fluorophényl)-4-oxo-3,4-dihydrophtalazin-1-yl)phényl)-N-méthyl-méthylsulfamoylamide ;
81) 4-(3-(1H-tétrazol-5-yl)phényl)-2-(4-fluorophényl)phtalazin-1(2H)-one ;
82) N-(5-(3-(4-fluorophényl)-4-oxo-3,4-dihydrophtalazin-1-yl)pyridin-3-yl)éthanesulfonamide ;
83) 3-(3-(4-fluorophényl)-4-oxo-3,4-dihydrophtalazin-1-yl)-N-hydroxybenzamide ;
84) (Z)-1-(3-(3-([1,1'-biphényl]-4-yl)-4-oxo-3,4-dihydrophtalazin-1-yl)phényl)-N-méthylméthanimine oxyde ;
85) (Z)-1-(3-(3-([1,1'-biphényl]-4-yl)-4-oxo-3,4-dihydrophtalazin-1-yl)phényl)-N-isopropylméthanimine oxyde ;
86) 3-(3-(4-fluorophényl)-4-oxo-3,4-dihydrophtalazin-1-yl)-N-méthylbenzènesulfonamide ;
87) N-éthyl-3-(3-(4-fluorophényl)-4-oxo-3,4-dihydrophtalazin-1-yl)benzènesulfonamide ;
88) 3-(3-(4-fluorophényl)-4-oxo-3,4-dihydrophtalazin-1-yl)-N,N-diméthylbenzènesulfonamide ;
89) 2-(2,4-difluorophényl)-4-(3-(méthylsulfonyl)phényl)phtalazin-1(2H)-one ;
90) 2-(2,4-difluorophényl)-4-(3-(éthylsulfonyl)phényl)phtalazin-1(2H)-one ;
91) 3-(3-(2,4-difluorophényl)-4-oxo-3,4-dihydrophtalazin-1-yl)-N-méthylbenzènesulfonamide ;
92) 3-(3-(2,4-difluorophényl)-4-oxo-3,4-dihydrophtalazin-1-yl)-N-éthylbenzènesulfonamide ;
93) 3-(3-(2,4-difluorophényl)-4-oxo-3,4-dihydrophtalazin-1-yl)-N,N-diméthylbenzènesulfonamide ;
94) (Z)-1-(3-(3-(2-fluorophényl)-4-oxo-3,4-dihydrophtalazin-1-yl)phényl)-N-méthylméthanimine oxyde ;
95) (Z)-1-(3-(3-(2-fluorophényl)-4-oxo-3,4-dihydrophtalazin-1-yl)phényl)-N-isopropylméthanimine oxyde ;
96) (Z)-N-*tert*-butyl-1-(3-(3-(2-fluorophényl)-4-oxo-3,4-dihydrophtalazin-1-yl)phényl)méthanimine oxyde ;
97) 2-(2-fluorophényl)-4-(3-(méthylsulfonyl)phényl)phtalazin-1(2H)-one ;
98) 4-(3-(éthylsulfonyl)phényl)-2-(2-fluorophényl)phtalazin-1(2H)-one ;
99) 3-(3-(2-fluorophényl)-4-oxo-3,4-dihydrophtalazin-1-yl)-N-méthylbenzènesulfonamide ;
100) N-éthyl-3-(3-(2-fluorophényl)-4-oxo-3,4-dihydrophtalazin-1-yl)benzènesulfonamide ;
101) 3-(3-(2-fluorophényl)-4-oxo-3,4-dihydrophtalazin-1-yl)-N,N-diméthylbenzènesulfonamide ;
102) (Z)-1-(3-(3-(2,6-difluorophényl)-4-oxo-3,4-dihydrophtalazin-1-yl)phényl)-N-méthylméthanimine oxyde ;
103) (*Z*)-1-(3-(3-(2,6-difluorophényl)-4-oxo-3,4-dihydrophtalazin-1-yl)phényl)-N-isopropylméthanimine oxyde ;
104) (Z)-N-tert-butyl-1-(3-(3-(2,6-difluorophényl)-4-oxo-3,4-dihydrophtalazin-1-yl)phényl)méthanimine oxyde ;
105) 2-(2,6-difluorophényl)-4-(3-(méthylsulfonyl)phényl)phtalazin-1(2H)-one ;
106) 2-(2,6-difluorophényl)-4-(3-(éthylsulfonyl)phényl)phtalazin-1(2H)-one ;
107) 3-(3-(2,6-difluorophényl)-4-oxo-3,4-dihydrophtalazin-1-yl)-N-méthylbenzènesulfonamide ;
108) 3-(3-(2,6-difluorophényl)-4-oxo-3,4-dihydrophtalazin-1-yl)-N-éthylbenzènesulfonamide ;
109) 3-(3-(2,6-difluorophényl)-4-oxo-3,4-dihydrophtalazin-1-yl)-N,N-diméthylbenzènesulfonamide ;
110) 6-fluoro-4-(3-(méthylsulfonyl)phényl)-2-phénylphtalazin-1(2H)-one ;
111) 3-(7-fluoro-4-oxo-3-phényl-3,4-dihydrophtalazin-1-yl)-N-méthylbenzènesulfonamide ;
112) 7-fluoro-4-(3-(méthylsulfonyl)phényl)-2-phénylphtalazin-1(2H)-one ;
113) 3-(6-fluoro-4-oxo-3-phényl-3,4-dihydrophtalazin-1-yl)-N-méthylbenzènesulfonamide ;
114) 3-(3-(4-fluorophényl)-4-oxo-3,4-dihydrophtalazin-1-yl)pyridine-1-oxyde ;
115) 2-(2,4-difluorophényl)-4-(pyridin-3-yl)phtalazin-1(2H)-one ;
116) 2-(2,6-difluorophényl)-4-(pyridin-3-yl)phtalazin-1(2H)-one ;
117) 3-(3-(2,4-difluorophényl)-4-oxo-3,4-dihydrophtalazin-1-yl)pyridine-1-oxyde ;
118) 3-(3-(2,6-difluorophényl)-4-oxo-3,4-dihydrophtalazin-1-yl)pyridine-1-oxyde ;
119) 2-(2,4-difluorophényl)-6-fluoro-4-(3-(méthylsulfonyl)phényl)phtalazin-1(2H)-one ;
120) 2-(2,4-difluorophényl)-7-fluoro-4-(3-(méthylsulfonyl)phényl)phtalazin-1(2H)-one ;
121) 3-(3-(2,4-difluorophényl)-7-fluoro-4-oxo-3,4-dihydrophtalazin-1-yl)-N-méthylbenzènesulfonamide ;
122) 3-(3-(2,4-difluorophényl)-6-fluoro-4-oxo-3,4-dihydrophtalazin-1-yl)-N-méthylbenzènesulfonamide ;
123) 2-(2,4-difluorophényl)-4-(3-(éthylsulfonyl)phényl)-6-fluorophtalazin-1(2H)-one ;
124) 3-(3-(2,4-difluorophényl)-7-fluoro-4-oxo-3,4-dihydrophtalazin-1-yl)-N-éthylbenzènesulfonamide ;
125) 3-(3-(2,4-difluorophényl)-7-fluoro-4-oxo-3,4-dihydrophtalazin-1-yl)-N,N-diméthylbenzènesulfonamide ;
126) 3-(3-(2,4-difluorophényl)-4-oxo-3,4-dihydrophtalazin-1-yl)benzènesulfonamide ;
127) 3-(3-(2,4-difluorophényl)-4-oxo-3,4-dihydrophtalazin-1-yl)-N-(2-hydroxyéthyl)benzènesulfonamide ;
128) N-(3-(3-cyclohexyl-4-oxo-3,4-dihydrophtalazin-1-yl)phényl)éthanesulfonamide ;
129) N-(3-(3-(1-méthylpipéridin-4-yl)-4-oxo-3,4-dihydrophtalazin-1-yl)phényl)éthanesulfonamide ;
130) 3-(3-(4-fluorophényl)-4-oxo-3,4-dihydrophtalazin-1-yl)benzonitrile ;
131) (E)-3-(3-(4-fluorophényl)-4-oxo-3,4-dihydrophtalazin-1-yl)-N'-hydroxybenzimidamide ;
132) N-(3-(3-isopropyl-4-oxo-3,4-dihydrophtalazin-1-yl)phényl)éthanesulfonamide ;
133) N-(3-(3-(1-méthyl-1H-pyrazol-4-yl)-4-oxo-3,4-dihydrophtalazin-1-yl)phényl)éthanesulfonamide ;
134) chlorhydrate de 4-(3-aminopipéridin-1-yl)-2-(4-fluorophényl)phtalazin-1(2H)-one ;
135) N-(1-(3-(4-fluorophényl)-4-oxo-3,4-dihydrophtalazin-1-yl)pipéridin-3-yl)éthanesulfonamide ;
136) N-(3-(3-(oxétan-3-yl)-4-oxo-3,4-dihydrophtalazin-1-yl)phényl)éthanesulfonamide ;
137) N-(1-(3-(4-fluorophényl)-4-oxo-3,4-dihydrophtalazin-1-yl)pyrrolidin-3-yl)éthanesulfonamide ;
138) (S)-N-(1-(3-(4-fluorophényl)-4-oxo-3,4-dihydrophtalazin-1-yl)pipéridin-3-yl)éthanesulfonamide ;
139) (R)-N-(1-(3-(4-fluorophényl)-4-oxo-3,4-dihydrophtalazin-1-yl)pipéridin-3-yl)éthanesulfonamide ;
140) N-(3-(3-(4,4-difluorocyclohexyl)-4-oxo-3,4-dihydrophtalazin-1-yl)phényl)éthanesulfonamide ;
141) N-(1-(3-(4-fluorophényl)-4-oxo-3,4-dihydrophtalazin-1-yl)azépan-3-yl)éthanesulfonamide ;
142) 3-(3-cyclohexyl-4-oxo-3,4-dihydrophtalazin-1-yl)benzènesulfonamide ;
143) chlorhydrate de 4-(3-aminoazépan-1-yl)-2-cyclohexylphtalazin-1(2H)-one ;
144) N-(1-(3-cyclohexyl-4-oxo-3,4-dihydrophtalazin-1-yl)azépan-3-yl)éthanesulfonamide ;
145) N-(3-(3-(4-méthylcyclohexyl)-4-oxo-3,4-dihydrophtalazin-1-yl)phényl)éthanesulfonamide ;
146) (S)-4-(3-aminopipéridin-1-yl)-2-cyclohexyl phtalazin-1(2H)-one-chlorhydrate ;
147) (S)-N-(1-(3-cyclohexyl-4-oxo-3,4-dihydrophtalazin-1-yl)pipéridin-3-yl)éthanesulfonamide ;
148) chlorhydrate de 4-(3-aminoazépan-1-yl)-2-(4-fluorophényl)phtalazin-1(2H)-one ;
149) N-(3-(2-(4-fluorophényl)-1-oxo-1,2-dihydro pyrido[3,4-d]pyridin-4-yl)phényl)éthanesulfonamide ;
150) N-(3-(3-(4-fluorophényl)-8-méthyl-4-oxo-3,4-dihydrophtalazin-1-yl)phényl)éthanesulfonamide ;
151) N-(3-(4-oxo-3-(tétrahydro-2H-pyran-4-yl)-3,4-dihydrophtalazin-1-yl)phényl)éthanesulfonamide ;
152) acide 2-(3-(3-(4-fluorophényl)-4-oxo-3,4-dihydrophtalazin-1-yl)phényl)-2-méthylpropanoïque ;
153) 2-(3-(3-(4-fluorophényl)-4-oxo-3,4-dihydrophtalazin-1-yl)phényl)-N,2-diméthylpropanamide ;
154) 2-cyclohexyl-4-(3-(méthylsulfonyl)phényl)phtalazin-1(2H)-one ;
155) (S)-4-(3-aminoazépan-1-yl)-2-cyclohexyl phtalazin-1(2H)-one·chlorhydrate ;
156) (S)-2-cyclohexyl-4-(3-(méthylamino)azépan-1-yl)phtalazin-1(2H)-one-chlorhydrate ;
157) (S)-4-(3-aminoazépan-1-yl)-2-(2,4-difluorophényl)phtalazin-1(2H)-one·chlorhydrate ;
158) chlorhydrate de (S)-4-(3-aminoazépan-1-yl)-2-(4-fluorophényl)phtalazin-1(2H)-one ;
159) chlorhydrate de (R)-4-(3-aminoazépan-1-yl)-2-cyclohexylphtalazin-1(2H)-one ;
160) acide 2-(3-(3-cyclohexyl-4-oxo-3,4-dihydrophtalazin-1-yl)phényl)-2-méthylpropanoïque ;
161) 3-(3-(3-(4-fluorophényl)-4-oxo-3,4-dihydrophtalazin-1-yl)phényl)oxazolidin-2-one ;
162) chlorhydrate de (R)-4-(3-aminoazépan-1-yl)-2-(4-fluorophényl)phtalazin-1(2H)-one ;
163) 2-(3-(3-cyclohexyl-4-oxo-3,4-dihydrophtalazin-1-yl)phényl)-2-méthylpropanamide ;
164) 2-(3-(3-cyclohexyl-4-oxo-3,4-dihydrophtalazin-1-yl)phényl)-N,2-diméthylpropanamide ;
165) chlorhydrate de (R)-4-(3-aminoazépan-1-yl)-2-(2,4-difluorophényl)phtalazin-1(2H)-one ;
166) acide 2-(3-(3-(2,4-difluorophényl)-4-oxo-3,4-dihydrophtalazin-1-yl)phényl)-2-méthylpropanoïque ;
167) 4-(3-aminoazépan-1-yl)-2-(2,4-difluorophényl)phtalazin-1(2H)-one-chlorhydrate ;
168) 2-(3-(3-(2,4-difluorophényl)-4-oxo-3,4-dihydrophtalazin-1-yl)phényl)-2-méthylpropanenitrile ;
169) 4-(3-(2-(1H-tétrazol-5-yl)propan-2-yl)phényl)-2-(2,4-difluorophényl)phtalazin-1(2H)-one ;
170) (E)-2-(3-(3-(2,4-difluorophényl)-4-oxo-3,4-dihydrophtalazin-1-yl)phényl)-N'-hydroxy-2-méthylpropane-imide-amide ;
171) acide 2-(3-(3-(2-fluorophényl)-4-oxo-3,4-dihydrophtalazin-1-yl)phényl)-2-méthylpropanoïque ;
172) 4-(3-aminoazépan-1-yl)-2-cyclobutylphtalazin-1(2H)-one ·chlorhydrate ;
173) chlorhydrate de 4-(3-aminoazépan-1-yl)-2-cyclopentylphtalazin-1(2H)-one ;
174) 2-(2,4-difluorophényl)-4-(3-(2-(5-méthyl-1,2,4-oxadiazol-3-yl)propan-2-yl)phényl)phtalazin-1(2H)-one ;
175) (R)-4-(3-aminoazépan-1-yl)-2-(2-fluorophényl)phtalazin-1(2H)-one·chlorhydrate ;
176) 2-(3-(3-(2,4-difluorophényl)-4-oxo-3,4-dihydrophtalazin-1-yl)phényl)-N-isopropyl-2-méthylpropanamide ;
177) 2-(2,4-difluorophényl)-4-(3-(2-méthyl-1-oxo-1-(pyrrolidin-1-yl)propan-2-yl)phényl)phtalazin-1(2H)-one ;
178) N-cyclopropyl-2-(3-(3-(2,4-difluorophényl)-4-oxo-3,4-dihydrophtalazin-1-yl)phényl)-2-méthylpropanamide ;
179) 2-(2,4-difluorophényl)-4-(3-(2-méthyl-1-morpholino-1-oxopropan-2-yl)phényl)phtalazin-1(2H)-one ;
180) N-cyclohexyl-2-(3-(3-(2,4-difluorophényl)-4-oxo-3,4-dihydrophtalazin-1-yl)phényl)-2-méthylpropanamide ;
181) 2-(3-(3-(2,4-difluorophényl)-4-oxo-3,4-dihydrophtalazin-1-yl)phényl)-2-méthyl-N-phénylpropanamide ;
182) 2-(3-(3-(2,4-difluorophényl)-4-oxo-3,4-dihydrophtalazin-1-yl)phényl)-2-méthyl-N-(1-méthyl-1H-pyrazol-4-yl)propanamide ;
183) acide 1-(3-(3-(2,4-difluorophényl)-4-oxo-3,4-dihydrophtalazin-1-yl)phényl)cyclopropan-1-carboxylique ;
184) 4-(1,4-diazépan-1-yl)-2-(2,4-difluorophényl)phtalazin-1(2H)-one-chlorhydrate ;
185) (R)-4-(3-aminoazépan-1-yl)-2-(4-chlorophényl)phtalazin-1(2H)-one-chlorhydrate ;
186) 2-(3-(3-(2,4-difluorophényl)-4-oxo-3,4-dihydrophtalazin-1-yl)phényl)-N-(2-hydroxyéthyl)-2-méthylpropanamide ;
187) (R)-N-(1-(3-(2,4-difluorophényl)-4-oxo-3,4-dihydrophtalazin-1-yl)azépan-3-yl)éthanesulfonamide ;
188) 2-(3-(3-(2,4-difluorophényl)-4-oxo-3,4-dihydrophtalazin-1-yl)phényl)-2-méthylpropanamide ;
189) (R)-2-(2,4-difluorophényl)-4-(3-(méthylamino)azépan-1-yl)phtalazin-1(2H)-one·chlorhydrate ;
190) 2-(2,4-difluorophényl)-4-(3-hydroxyazépan-1-yl)phtalazin-1(2H)-one ;
191) (R)-2-(2,4-difluorophényl)-4-(3-((2-hydroxyéthyl)amino)azépan-1-yl)phtalazin-1(2H)-one·chlorhydrate ;
192) (R)-N-(1-(3-(2,4-difluorophényl)-4-oxo-3,4-dihydrophtalazin-1-yl)azépan-3-yl)acétamide ;
193) 2-(2,4-difluorophényl)-4-(3-oxoazépan-1-yl)phtalazin-1(2H)-one ;
194) acide 2-(3-(3-(4-chlorophényl)-4-oxo-3,4-dihydrophtalazin-1-yl)phényl)-2-méthylpropanoïque ;
195) acide 1-(3-(3-(4-chlorophényl)-4-oxo-3,4-dihydrophtalazin-1-yl)phényl)cyclopropan-1-carboxylique ;
196) 2-(4-chlorophényl)-4-(3-hydroxyazépan-1-yl)phtalazin-1(2H)-one ;
197) 2-(3-(3-(4-chlorophényl)-4-oxo-3,4-dihydrophtalazin-1-yl)phényl)-2-méthylpropanamide ;
198) 1-(3-(3-(4-chlorophényl)-4-oxo-3,4-dihydrophtalazin-1-yl)phényl)cyclopropan-1-carboxamide ;
199) acide 2-(3-(3-(2,4-difluorophényl)-4-oxo-3,4-dihydrophtalazin-1-yl)phényl)acétique ;
200) chlorhydrate de 4-(3-(2-aminopropan-2-yl)phényl)-2-(2,4-difluorophényl)phtalazin-1(2H)-one ;
201) acide 2-(3-(3-(4-chloro-2-fluorophényl)-4-oxo-3,4-dihydrophtalazin-1-yl)phényl)-2-méthylpropanoïque ;
202) (R)-4-(3-aminoazépan-1-yl)-2-(4-chloro-2-fluorophényl)phtalazin-1-(2H)-one·chlorhydrate ;
203) 1-(3-(4-chloro-2-fluorophényl)-4-oxo-3,4-dihydrophtalazin-1-yl)azépane-3-carbonitrile ;
204) 4-(3-(1-amino-2-méthylpropan-2-yl)phényl)-2-(2,4-difluorophényl)phtalazin-1(2H)-one·chlorhydrate ;
205) acide 2-(1-(3-(2,4-difluorophényl)-4-oxo-3,4-dihydrophtalazin-1-yl)pipéridin-3-yl)-2-méthylpropanoïque ;
206) acide 2-(3-(3-(4-cyclopropylphényl)-4-oxo-3,4-dihydrophtalazin-1-yl)phényl)-2-méthylpropanoïque ;
207) acide 1-(3-(3-(2,4-difluorophényl)-4-oxo-3,4-dihydrophtalazin-1-yl)phényl)cyclobutane-1-carboxylique ;
208) acide 1-(3-(3-(2,4-difluorophényl)-4-oxo-3,4-dihydrophtalazin-1-yl)phényl)cyclopentane-1-carboxylique ;
209) acide 2-(1-(3-(4-chlorophényl)-4-oxo-3,4-dihydrophtalazin-1-yl)pipéridin-3-yl)-2-méthylpropanoïque ;
210-1 et 210-2) acide 2-(1-(3-(2,4-difluorophényl)-4-oxo-3,4-dihydrophtalazin-1-yl)pipéridin-3-yl)-2-méthylpropanoïque ;
211) 1-(1-(3-(2,4-difluorophényl)-4-oxo-3,4-dihydrophtalazin-1-yl)pipéridin-3-yl)cyclopropan-1-carboxylate de méthyle ;
212) acide 1-(1-(3-(2,4-difluorophényl)-4-oxo-3,4-dihydrophtalazin-1-yl)pipéridin-3-yl)cyclopropan-1-carboxylique ;
213-1 et 213-2) acide 1-(1-(3-(2,4-difluorophényl)-4-oxo-3,4-dihydrophtalazin-1-yl)pipéridin-3-yl)cyclopropan-1-carboxylique ;
214) 2-(2,4-difluorophényl)-4-(3-(pyrrolidin-1-ylsulfonyl)phényl)phtalazin-1(2H)-one ;
215) chlorhydrate de 2-(2,4-difluorophényl)-4-(1,2,3,6-tétrahydropyridin-4-yl)phtalazin-1(2H)-one ;
216) 3-(3-(2,4-difluorophényl)-4-oxo-3,4-dihydrophtalazin-1-yl)-N-éthyl-N-méthylbenzènesulfonamide ;
217) 3-(3-(2,4-difluorophényl)-4-oxo-3,4-dihydrophtalazin-1-yl)-N,N-diéthylbenzènesulfonamide ;
218) 2-(2,4-difluorophényl)-4-(1-(éthylsulfonyl)-1,2,3,6-tétrahydropyridin-4-yl)phtalazin-1(2H)-one ;
219) acide 2-(4-(3-(2,4-difluorophényl)-4-oxo-3,4-dihydrophtalazin-1-yl)pipérazin-2-yl)-2-méthylpropanoïque ;
220) acide 2-(4-(3-(2,4-difluorophényl)-4-oxo-3,4-dihydrophtalazin-1-yl)-1-méthylpipérazin-2-yl)-2-méthylpropanoïque ; et
221) acide 2-(4-(3-(2,4-difluorophényl)-4-oxo-3,4-dihydrophtalazin-1-yl)morpholin-2-yl)-2-méthylpropanoïque.

2. Composition pharmaceutique destinée à prévenir ou à traiter une maladie liée à Sirt6, comprenant un composé, ou un racémate, un stéréoisomère ou un sel pharmaceutiquement acceptable de celui-ci selon la revendication 1, dans laquelle la maladie liée à Sirt6 est choisie dans le groupe constitué par un cancer, une stéatose hépatique, une cirrhose hépatique/inflammation, une diminution de la sécrétion d'insuline et des maladies causées par le vieillissement.
